(19) Europäisches Patentamt
European Patent Office
Office européen des brevets

(11) EP 4 130 267 A1

(12) EUROPEAN PATENT APPLICATION
published in accordance with Art. 153(4) EPC

(43) Date of publication:
**08.02.2023 Bulletin 2023/06**

(21) Application number: **21776483.6**

(22) Date of filing: **29.03.2021**

(51) International Patent Classification (IPC):
***C12N 15/115*** *(2010.01)* ***C12Q 1/04*** *(1980.01)*
***C12Q 1/68*** *(1980.01)* ***C12N 15/10*** *(1990.01)*

(52) Cooperative Patent Classification (CPC):
**C12N 15/10; C12N 15/115; C12N 15/65; C12N 15/70; C12N 15/81; C12N 15/85; C12Q 1/04; C12Q 1/68; G01N 21/64; G01N 33/53; G01N 33/58**

(86) International application number:
**PCT/CN2021/083579**

(87) International publication number:
**WO 2021/190654 (30.09.2021 Gazette 2021/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.03.2020 CN 202010206404**

(71) Applicant: **East China University of Science and Technology**
**Shanghai 200237 (CN)**

(72) Inventors:
- **YANG, Yi**
  **Shanghai 200237 (CN)**
- **CHEN, Xianjun**
  **Shanghai 200237 (CN)**
- **XIE, Xin**
  **Shanghai 200237 (CN)**
- **SU, Ni**
  **Shanghai 200237 (CN)**

(74) Representative: **Bandpay & Greuter**
**30, rue Notre-Dame des Victoires**
**75002 Paris (FR)**

Remarks:
The complete document including Reference Table(s) and the Sequence Listing(s) can be downloaded from the EPO website

# (54) NEW METHOD FOR DETECTING AND QUANTIFYING RNA

(57) The present disclosure relates to an aptamer nucleic acid molecule, a complex containing the aptamer and fluorophore small molecules, a method for detecting intrecellular or extracellular RNA, DNA or other target molecules, as well as a kit containing the aptamer. The aptamer of the present disclosure can specifically bind to fluorophore small molecules, and can significantly improve their fluorescence intensity under light excitation at suitable wavelength.

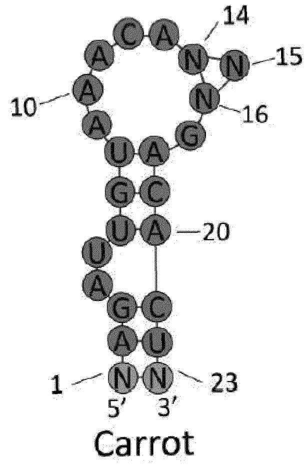


Carrot

Fig.1

EP 4 130 267 A1

## Description

### Technical Field

The present disclosure relates to an aptamer nucleic acid molecule, which is used in a method for detecting intracellular or extracellular RNA, DNA or other target molecules, and a kit containing the aptamer. The aptamer of the present disclosure can specifically bind to a fluorophore small molecule and significantly improve fluorescence intensity under light excitation at suitable wavelength.

### Background Art

RNA is one of the most important biomolecules, and it comes in many varieties, including messenger RNA (mRNA), transfer RNA (tRNA), ribosomal RNA (rRNA), small interfering RNA (siRNA), long non-coding RNA (IncRNA) and so on. In recent years, scientists have gradually discovered the crucial functions of RNA in a variety of vital activities, including many RNA-protein complexes, such as telomerase, splicing enzyme, ribozyme, and riboswitch or the like. Besides, the abundance of non-coding RNAs, such as short interfering RNA (siRNA), microRNA (microRNA), and long non-coding RNA (IncRNA), have played an irreplaceable role in the regulation of gene expression at the post-transcriptional level. Therefore, real-time monitoring of RNA transport and metabolic processes in cells is critical for studying the relationship between RNA localization and gene expression, as well as cell regulation.

Current methods for labeling RNA include in situ hybridization technology, RNA-binding protein-fluorescent protein technology, and RNA aptamer-fluorescent dye technology. Fluorescence in situ hybridization is a technique for directly labeling RNA based on fluorophore-modified oligonucleotide, and its basic principle is that fluorophore-modified oligonucleotide has a sequence complementary to the target RNA and the target RNA can be labeled by base pairing (Raj et al. Nature Methods 2018. 5: 877-879). The advantage thereof is that it does not require modification or genetic recombination of the target RNA itself, and labeling can be directly performed. The disadvantages thereof are as follows: the oligonucleotides do not enter cells easily, and auxiliary operation steps are required to introduce them into cells, which will easily damage the cells; the background fluorescence is high, and complex elution operation steps are required, so it can only be used for the study of fixed cells, not in real-time monitoring of dynamic changes of RNA in living cells. Molecular beacon technology relates to an oligonucleotide probe based on a hairpin structure, wherein: when it binds to the target RNA, the quenching effect of the quenching group labeled at one end on the fluorophore labeled at the other end will be eliminated, and the fluorophore will generate fluorescence, or the FRET of the fluorophore at both ends will disappear (Chen et al. Nucleic Acids Res 2010. 38: e148). The advantage thereof is that molecular beacons have a higher signal-to-noise ratio than other oligonucleotide probes because they have no background fluorescence. Oligonucleotide probes are difficult to enter cells; besides, the disadvantages thereof also lie in that they tend to accumulate in nucleus to cause non-specific fluorescence, are susceptible to the influence of RNA secondary structure, and need to customize oligonucleotide probes for each RNA (You et al. Ann. Rev. Biophys 2015. 44: 187-206), and these disadvantages limit the application of this technology.

RNA-binding protein-fluorescent protein technology is a commonly used method for labeling RNA in living cells at present, and commonly used RNA-binding protein systems include MCP/MS2 phage system (Bertrand et al. Molecular Cell 1998. 2: 437-445), $\lambda_N$/boxB phage system (Daigle et al. Nature Methods 2007. 4: 633-636) and dCas9-sgRNA system (Nelles et sl. Cell 2016. 165: 488-496). The basic principle thereof is to fuse a fluorescent protein with an RNA-binding protein that can bind to a specific RNA motif so as to obtain an RNA-binding protein-fluorescent protein fusion protein, meanwhile, the specific RNA sequence is fused with the target RNA, and thus the goal of labeling the target RNA with fluorescent protein can be achieved by binding the RNA-binding protein to the specific RNA sequence. This method is widely used for RNA labeling in live cells, but fusion proteins of unbound RNA-binding protein-fluorescent protein will result in comparatively high imaging background fluorescence. Although scientists have improved the imaging signal-to-noise ratio by concatenating multiple RNA sequences recognized by RNA-binding proteins and by binding multiple fluorescent proteins to one target RNA, the complexes with larger loads may affect the normal physiological function of RNA.

Fluorescent RNA technology based on RNA aptamer-fluorescent dye is an ideal RNA labeling technology. The basic principle thereof is that fluorescence is low before binding of fluorescent dye to RNA aptamer, and the fluorescence intensity is enhanced after binding of fluorescent dye to RNA aptamer. The advantages of this technology are as follows: the system is simple, and only requires to fuse RNA aptamer with target RNA; moreover, it also has the genetic coding characteristics and modularity, which can be universally applied to different RNAs. Early fluorescent RNAs were developed based on thiazole orange and its analogs or fluorophore-quencher groups (Dolgosheina et al. ACS chemical biology 2014. 9: 2412-2420; Sunbul et al. Angewandte Chemie 2013. 52: 13401-13404), and these fluorescent dyes have disadvantages such as high background fluorescence, high cytotoxicity or difficulty in entering cells, making them unsuitable for RNA labeling of living cells, especially mammalian cells. In 2011, S. Jaffrey's research group obtainedan

RNA aptamer, called Spinach, by screening based on fluorescent protein chromophore analogs (DFHBI) (Paige et al. Science 2011. 333: 642-646). Relative to previous fluorescent RNAs, DFHBI has the advantages of low background fluorescence, easy entry into cells and no cytotoxicity. Subsequently, the same research group developed Spinach2, Broccoli and Corn fluorescent RNAs (Song et al. Nature Chemical Biology 2017. 13: 1187-1194; Filonov et al. Journal of the American Chemical Society 2014. 136: 16299-16308; Strack et al. al. Nature methods 2013. 10: 1219-1224). Although these fluorescent RNAs can be used for labeling and imaging of a variety of high-abundance RNAs in mammalian cells, they still have disadvantages of unstable fluorescence, easy quenching, low brightness, dependence on $Mg^{2+}$ and aggregate properties, etc., and these disadvantages limit their widespread use. In 2019, Chen et al. developed a fluorescent RNA called Pepper, which has the advantages of high brightness and high stability, and can be used for labeling and imaging of various RNAs in living cells (Chen et al. Nature Biotechnology 2019. 37: 1287-1293). However, the execution of many cellular functions in living cells requires simultaneous participation of multiple RNAs, so it is necessary to develop multiple fluorescent RNAs with excellent properties that can be bioorthogonal, so as to label and image multiple RNAs in cells and explore their biological functions.

## Summary of the Invention

The present disclosure provides a nucleic acid aptamer molecule, a DNA molecule encoding the nucleic acid aptamer molecule, a complex of nucleic acid aptamer molecules and fluorophore molecules, and uses of the complex.
The technical solution provided by the present disclosure is as follows:

1. The present disclosure provides a nucleic acid aptamer molecule comprising the following nucleotide sequences (a), (b) or (c):

   (a): a nucleotide sequence $N_1$AGAUUGUAAACA$N_{14}$-$N_{15}$-$N_{16}$GACACU$N_{23}$ (called General Formula Carrot structure), wherein $N_1$, $N_{14}$, $N_{15}$, $N_{16}$ and $N_{23}$ represent nucleotide fragments greater than or equal to 1 in length, at least one base pair in $N_1$ and $N_{23}$ nucleotide sequences forms a complementary pair, and at least one base pair in $N_{14}$ and $N_{16}$ nucleotide sequences forms a complementary pair;

   (b): a nucleotide sequence with an identity of at least 70% to the nucleotide sequence (a); and

   (c): a nucleic acid aptamer molecule derived from the nucleotide sequence (a) at a position other than $N_1$, $N_{14}$, $N_{15}$, $N_{16}$ and $N_{23}$ in the nucleotide sequence (a), with substitution, missing and/or addition of one or several nucleotides, and having an aptamer function.

2. In some embodiments of the present disclosure, the nucleic acid aptamer molecule has a sequence with an identity of at least 72%, 77%, 83%, 88%, 94% or 100% to the Carrot structure nucleotide sequence of the nucleotide sequence (a).

3. In some embodiments of the present disclosure, the nucleotide sequence (c) is nucleic acid aptamer molecules obtained with substitution, missing and/or addition of 5, 4, 3, 2 or 1 nucleotide at a position other than $N_1$, $N_{14}$, $N_{15}$, $N_{16}$ and $N_{23}$ in the Carrot structure nucleotide sequence of the nucleotide sequence (a).

4. In some embodiments of the present disclosure, the nucleotide sequence (c) is nucleic acid aptamer molecules obtained with substitution, missing and/or addition of 4, 3, 2 or 1 nucleotide at a position other than $N_1$, $N_{14}$, $N_{15}$, $N_{16}$ and $N_{23}$ in the nucleotide sequence defined by the nucleotide sequence (a).

5. In some embodiments of the present disclosure, when $N_1$ and $N_{23}$ in the nucleotide sequence (a) form a complementary pair(s), the direction of $N_1$ nucleotide sequence is 5'-3', and the direction of $N_{23}$ nucleotide sequence is 3'-5'; and when $N_{14}$ and $N_{16}$ form a complementary pair(s), the direction of $N_{14}$ nucleotide sequence is 5'-3', and the direction of $N_{16}$ nucleotide sequence is 3'-5'.

6. In some embodiments of the present disclosure, when at least one fragment of $N_1$ and $N_{23}$ is greater than or equal to 5 nucleotide bases in length, at least two pairs of bases in $N_1$ and $N_{23}$ nucleotide sequences form complementary pairs; when at least one fragment of $N_{14}$ and $N_{16}$ is greater than or equal to 5 nucleotide bases in length, at least two pairs of bases in $N_{14}$ and $N_{16}$ nucleotide sequences form complementary pairs.

7. As for the afore-mentioned nucleic acid aptamer molecules, the nucleotide substitution in the General Formula Carrot structure is one selected from the following groups:

A4U, A4G, A4C, U5A, U5G, U5C, G7C, G7A, G7U, U8C, A10U, A10G, A10C, A11U, A11G, A11C, C12G, C12A, C12U, A13U, A13G, A13C, G17A, C19A, C19U, A20C, A4C/U5A, A4C/U5C, A4C/A11G, A4C/C12A, A4C/A13C, USA/A11G, U5A/C12A, USA/A13C, U5G/A13C, USC/G7U, USC/A11G, U5C/C12G, U5C/C12A, U5C/C12U, U5C/A13C, G7U/A13C, A11G/A13C, C12G/A13C, C12A/A13C, C12U/A13C, A4C/U5C/A13C, U5C/G7U/A13C, U5C/C12G/A13C, U5C/C12U/A13C, U5C/A11G/A13C, U5C/C12A/A13C, A4C/U5C/A11G/A13C, A4C/U5C/C12A/A13C, A4C/USC/G7U/A11G/A13C, A4C/U5C/G7U/C12A/A13C, A4C/U5A/A11G/C12A/A13C, and A4C/U5C/A11G/C12A/A13C.

8. In some embodiments of the present disclosure, the nucleotide substitution in the General Formula Carrot structure is one selected from the following groups: A4C, U5A, U5G, U5C, G7U, A11G, C12G, C12A, C12U, A13C, A4C/U5A, A4C/U5C, A4C/A11G, A4C/C12A, A4C/A13C, USA/A11G, U5A/C12A, USA/A13C, U5G/A13C, USC/G7U, USC/A11G, U5C/C12G, U5C/C12A, U5C/C12U, U5C/A13C, G7U/A13C, A11G/A13C, C12G/A13C, C12A/A13C, C12U/A13C, A4C/U5C/A13C, U5C/G7U/A13C, U5C/C12G/A13C, U5C/C12U/A13C, U5C/A11G/A13C, U5C/C12A/A13C, A4C/USC/A11G/A13C, and A4C/USC/C12A/A13C.

9. In some embodiments of the present disclosure, the nucleotide substitution in the General Formula Carrot structure is one selected from the following groups: A4C, U5A, U5G, U5C, G7U, A11G, C12G, C12A, C12U, A13C, A4C/U5C, A4C/A13C, U5A/A13C, USC/A11G, U5C/C12A, U5C/A13C, A11G/A13C, C12A/A13C, A4C/U5C/A13C, U5C/A11G/A13C, and U5C/C12A/A13C.

10. In the afore-mentioned nucleic acid aptamer molecules, the nucleotide sequences at $N_1$ and $N_{23}$ in the nucleotide sequence (a) are F30 or tRNA scaffold RNA sequences.

11. In some embodiments of the present disclosure, the aptamer molecules are RNA molecules or base-modified RNA molecules.

12. In some embodiments of the present disclosure, the aptamer molecules are DNA-RNA hybrid molecules or base-modified DNA-RNA molecules.

13. As for the afore-mentioned nucleic acid aptamer molecules, $N_{14}$-$N_{15}$-$N_{16}$ therein contains a nucleotide sequence capable of identifying target molecules.

14. In some embodiments of the present disclosure, the target molecules include but are not limited to: proteins, nucleic acid, lipid molecules, carbohydrates, hormones, cytokines, chemokines, metabolite and metal ions.

15. In some embodiments of the present disclosure, the $N_{14}$-$N_{15}$-$N_{16}$ is a nucleotide sequence capable of identifying S-ademetionine and adenosine molecules.

16. In some embodiments of the present disclosure, the aptamer function refers to that the nucleic acid aptamer can enhance fluorescence intensity of fluorophore molecules under light excitation at suitable wavelength by at least two times, by at least 5 to 10 times, by at least 20 to 50 times, by at least 100 to 200 times or by at least 200 times.

17. In some embodiments of the present disclosure, the nucleic acid aptamer molecule may further include concatemers that can bind to multiple fluorophore molecules, wherein the concatemers are connected by a spacer sequence(s) of suitable length, and the number may be 2, 3, 4, 5, 6, 7, 8 or more. The nucleotide of the concatemer(s) is selected from but are not limited to a sequence SEQ ID No: 7, 8, 9, 10, 11, 12, 13, 14, 15, 16, 17, 18, 19 and 20.

18. As for the afore-mentioned nucleic acid aptamer molecules, the nucleic acid aptamer molecule has a sequence selected from SEQ ID No: 1, 2, 3, 4, 5, 6, 7, 8, 9, 10, 11, 12, 13, 14, 15, 16, 17, 18, 19, 20, 22, 24, 25, 26, 27, 28, 29, 30 or 31.

19. A complex of nucleic acid aptamer molecules and fluorophore molecules, wherein the nucleic acid aptamer molecule(s) is the afore-mentioned nucleic acid aptamer molecule(s), and the fluorophore molecule(s) has a structure shown in Formula (I) below:

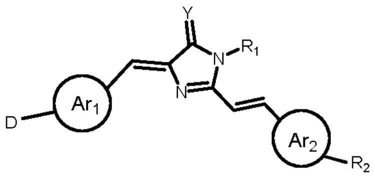 (I)

wherein: $Ar_1$, and $Ar_2$ are independently hexahydric aryl group or hexahydric heteroaryl group; D- is HO- or $N(X_1)(X_2)$-, and $X_1$ and $X_2$ are independently selected from hydrogen, alkyl and modified alkyl; $X_1$ and $X_2$ are optionally interconnected and form an alicyclic heterocycle with N atom; when D- is $N(X_1)(X_2)$- and $Ar_1$ is phenyl group, $X_1$ and $X_2$ independently form a saturated or unsaturated alicyclic heterocycle with the benzene ring; when D- is HO- and $Ar_1$ is phenyl group, at least one hydrogen atom adjacent to HO- is substituted by halogen; Y is O or S; $R_1$ is hydrogen, alkyl or modified alkyl; and $R_2$ is a hydrogen atom, a halogen atom, -OH, or - CN;
wherein: the "alkyl" is independently $C_1$-$C_{10}$ straight or branched alkyl; optionally, the "alkyl" is $C_1$-$C_7$ straight or branched alkyl; optionally, the "alkyl" is $C_1$-$C_5$ straight or branched alkyl; optionally, the "alkyl" is selected from methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl or 2,2,3-trimethylbutyl.

20. In some embodiments of the present disclosure, the "modified alkyl" is a group obtained by replacing any carbon atom of the alkyl with one or more groups selected from halogen atoms, -OH, -CO-, -O-, -CN, $-SO_3H$, primary amino group, secondary amino group, and tertiary amino group, and the modified alkyl has 1 to 10 carbon atoms, wherein the carbon-carbon single bond is optionally and independently replaced by a carbon-carbon double bond or a carbon-carbon triple bond;

the replacement of carbon atoms refers to that carbon atoms are replaced with corresponding group or that carbon atoms, together with hydrogen atoms thereon, are replaced with corresponding group;

the "modified alkylene" is a group obtained by replacing any carbon atom of $C_1$-$C_{10}$ (preferably $C_1$-$C_6$) alkylene with groups selected from -O-, -OH, -CO-, -CS-, -(S=O)-;

optionally, the "modified alkyl" contains one or more groups selected from -OH, -O-, ethylene glycol unit ($-(CH_2CH_2O)_n-$), monosaccharide unit, -O-CO-, -NH-CO-, $-SO_2$-O-, -SO-, $Me_2N$-, $Et_2N$-, -S-S-, -CH=CH-, F, Cl, Br, I, and cyano group;

optionally, $Ar_1$ is selected from structures represented by the following formulae ( II -1) to ( II -15):

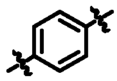 ( II -1), 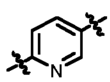 ( II -2), 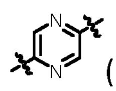 ( II -3), 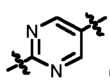 ( II -4), 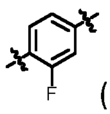 ( II -5), 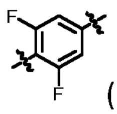 ( II -6),

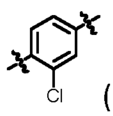 ( II -7), 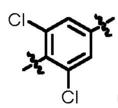 ( II -8), 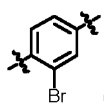 ( II -9), 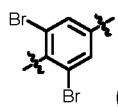 ( II -10), 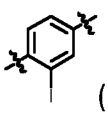 ( II -11), 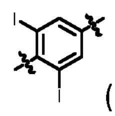 ( II -12).

$Ar_2$ is selected from structures represented by the following formulae (III-1) to (III-25):

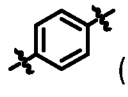 (III-1), 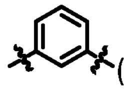 (III-2), 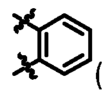 (III-3), 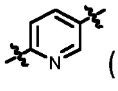 (III-4), 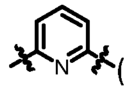 (III-5), 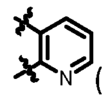 (III-6),

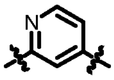 (Ⅲ-7), 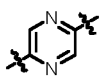 (Ⅲ-8), 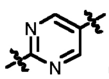 (Ⅲ-9).

Optionally, the compound represented by Formula (I) is selected from compounds represented by the following formulae:

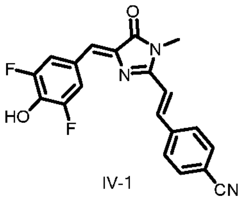
IV-1,

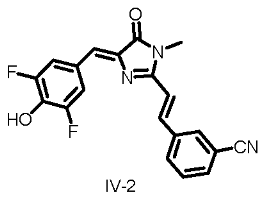
IV-2,

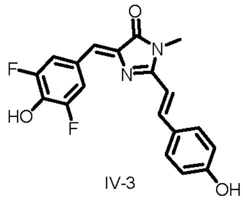
IV-3,

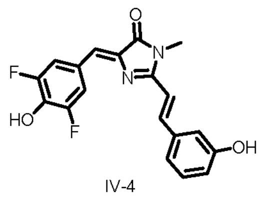
IV-4,

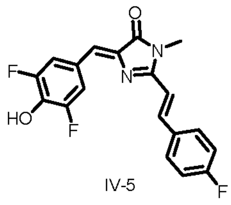
IV-5,

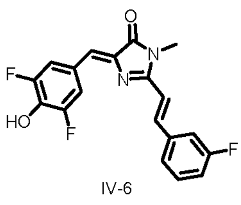
IV-6,

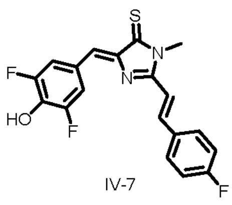
IV-7,

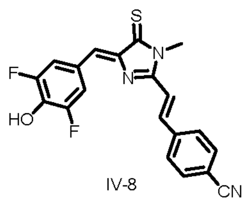
IV-8,

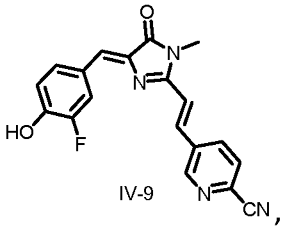
IV-9,

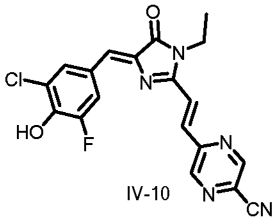
IV-10,

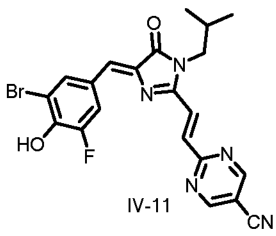
IV-11,

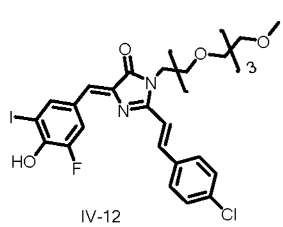
IV-12,

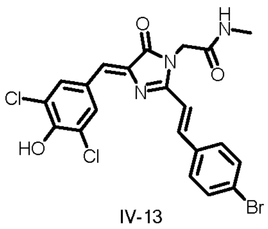
IV-13,

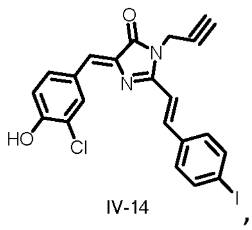
IV-14,

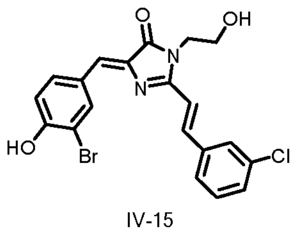
IV-15,

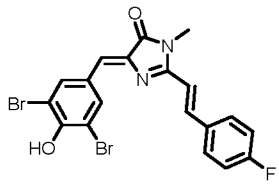
IV-16,

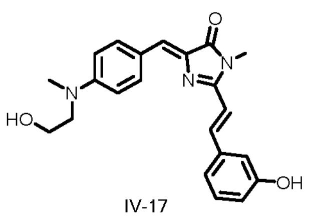
IV-17,

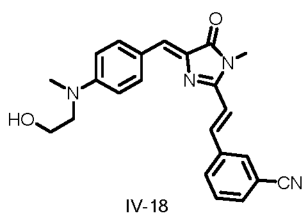
IV-18,

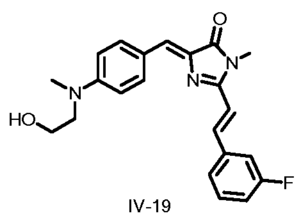
IV-19,

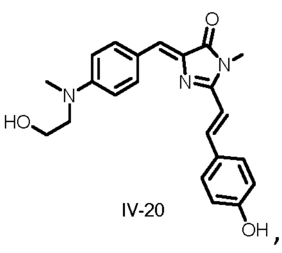
IV-20,

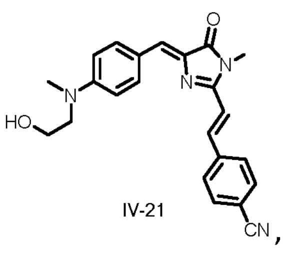
IV-21,

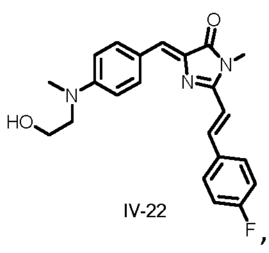
IV-22,

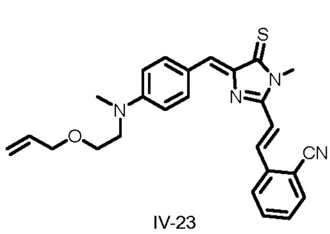
IV-23,

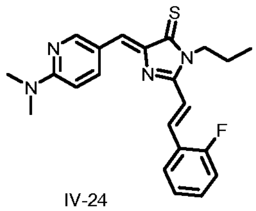
IV-24,
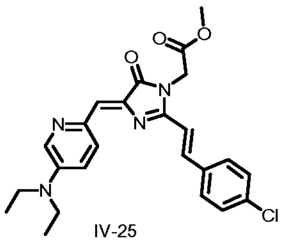
IV-25,
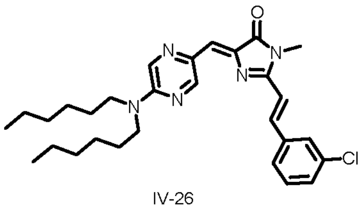
IV-26,
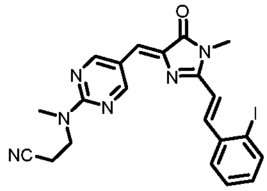
IV-27

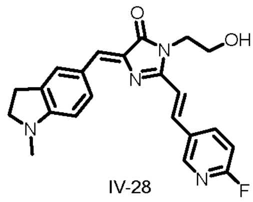
IV-28,
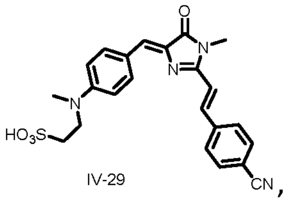
IV-29,
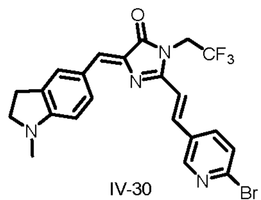
IV-30,

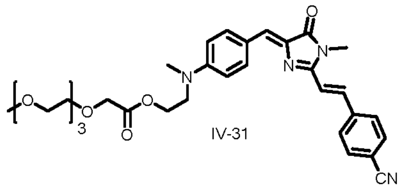
IV-31,
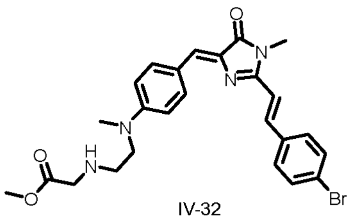
IV-32,
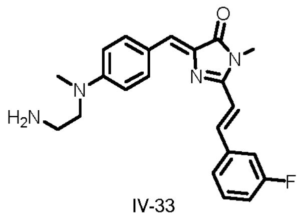
IV-33,

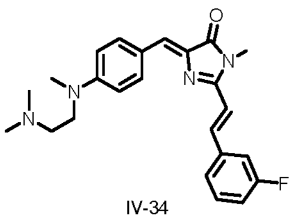
IV-34,
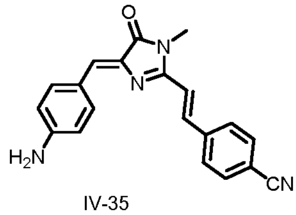
IV-35,
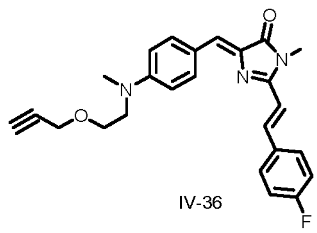
IV-36,
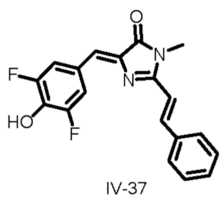
IV-37,

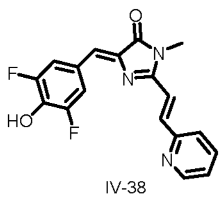
IV-38,
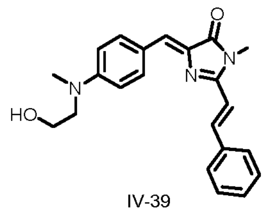
IV-39,
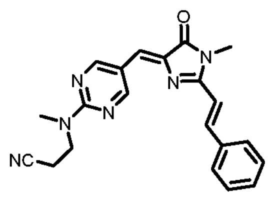
IV-40.

21. In some embodiments of the present disclosure, the aptamer molecules in the complex contain nucleotide sequence SEQ ID No: 1, 2, 3, 4, 5, 6, 7, 8, 9, 10, 11, 12, 13, 14, 15, 16, 17, 18, 19, 20, 22, 24, 25, 26, 27, 28, 29, 30 or 31.

22. A complex used for detecting or labeling target nucleic acid molecules in vitro or in vivo.

23. A complex used for detecting or labeling extracellular or intracellular target molecules.

24. A complex used for imaging of genomic DNA.

25. A complex used for detecting interaction between RNA and protein.

26. A DNA molecule, which transcribes the afore-mentioned nucleic acid aptamer molecules.

27. An expression vector containing the DNA molecule.

28. A host cell containing the expression vector.

29. A kit containing the nucleic acid aptamer molecules and/or the expression vectors and/or the host cells and/or the complexes.

30. A method of detecting a target molecule, including:

a) adding the complex to a solution containing the target molecule;

b) exciting the complex with light of suitable wavelength; and

c) detecting the fluorescence of the complex.

31. A method of detecting genomic DNA, including imaging genomic DNA with the complex.

32. A method of extracting and purifying RNA, including extracting and purifying RNA with the complex.

The inventor designed brand-new nucleic acid aptamer molecules, and synthesized brand-new fluorophore molecules, so as to form a brand-new fluorophore-nucleic acid aptamer complex. After binding of the aptamer molecules to the fluorophore molecules, the fluorescence intensity of the fluorophore molecules can be significantly increased under light excitation at suitable wavelength. They overcome the shortcomings of previous fluorophore-nucleic acid aptamer complexes, and can be used for effective real-time RNA/DNA labeling in living cells. The nucleic acid aptamer of the present disclosure has a strong affinity for fluorophore molecules, and shows different fluorescence spectra. These nucleic acid aptamer-fluorophore molecule complexes can be used for real-time labeling and imaging of RNA/DNA in prokaryotic and eukaryotic cells, detecting RNA-protein interactions, or as labels for RNA extraction and purification.

**Description of Drawings**

Fig. 1 Secondary structure prediction for the general structure of Carrot nucleic acid aptamer molecules.

Fig. 2 Secondary structure prediction of Carrot-1 and Carrot-2 nucleic acid aptamer molecules.

Fig. 3 Secondary structure prediction of F30-Carrot-2.

Fig. 4 Secondary structure prediction of tRNA-Carrot-2.

Fig. 5 Nature identification of Carrot-IV-39 complex. (a) is fluorescence excitation spectrum and emission spectrum of Carrot-1-IV-39 complex; (b) is dissociation constant determination of the binding of Carrot-1 to IV-39; and (c) is dependence determination of Carrot-1-IV-39 complex on $Mg^{2+}$.

Fig. 6 Activation effect of Carrot modified with different bases on IV-39. (a) is a diagram of Carrot modified with different bases: as for Carrot-3, the dark bases in the structure diagram are deoxyribonucleotide bases; as for Carrot-4, the dark bases in the structure diagram are bases modified with 2'-F; (b) is the activation effects of Carrot-3 and Carrot-4 on the fluorescence of IV-39 fluorophore molecules, and "Control" is to replace Carrot-3 or Carrot-4 aptamer RNA with buffer.

Fig. 7 Activation effects of different Carrot concatemers on IV-39. (a) is a diagram of Tandem 1; (b) is a diagram of Tandem 2; (c) is a diagram of Tandem 3; (d) is the activation effect of Carrot concatemers of Tandem 1 on the fluorescence of IV-39 fluorophore molecules; (e) is the activation effect of Carrot concatemers of Tandem 2 on the fluorescence of IV-39 fluorophore molecules; and (f) is the activation effect of Carrot concatemers of Tandem 3 on the fluorescence of IV-39 fluorophore molecules.

Fig. 8 Labeling effect of F30-Carrot-2-IV-4 complex on RNA in bacteria.

Fig. 9 Labeling effect of F30-Carrot-2-IV-4 complex on RNA in yeast cells.

Fig. 10 Labeling effects of Carrot, IV-39 and analogue thereof on RNA in mammalian cells. (a) is the labeling effect of tRNA-Carrot-2-IV-39 complex on RNA in mammalian cells; (b) is statistical results of tRNA-Carrot-2-IV-39 complex for RNA labeling in mammalian cells; and (c) is the effects of F30-4Carrot-2 and IV-39 analogs on RNA labeling in mammalian cells.

Fig. 11 Probe construction based on Carrot-1, (a) is a diagram of probe construction; and (b) is activation effects of probe on IV-39 fluorophore molecule fluorescence in the presence or absence of target.

Fig. 12 RNA localization in tracer cells by using Carrot, (a) is the detection of GAPDH mRNA localization by using 4Carrot-2; and (b) is the detection of ACTB mRNA localization by using 4Carrot-2.

Fig. 13 Imaging results of Carrot for detecting genomic DNA. (a) is a diagram of different chimeric sgRNA; and (b) is the imaging results of different chimeric sgRNA for genetic locus in living cells.

Fig. 14 Result of Carrot for detecting RNA-protein interaction.

Fig. 15 Result of Carrot for RNA extraction and purification.

**Details of the Disclosure**

The following definitions and embodiments cited in the present disclosure will be described in details here. The contents of all patents and published literature referred to herein, including all sequences disclosed in these patents and published literature, are expressly incorporated herein by reference. Hereinafter, "nucleotides" and "nucleotide bases" are used interchangeably and stand for the same meaning.

Nucleic acid aptamer molecules

The "nucleic acid aptamer molecules" of the present disclosure are also referred to as "aptamer molecules". The nucleic acid aptamer molecule contains (a) a nucleotide sequence $N_1AGAUUGUAAACAN_{14}$-$N_{15}$-$N_{16}GACACUN_{23}$ (corresponding to the General Formula Carrot structure in Fig. 1); or (b) which is a nucleotide sequence with an identity of at least 70% to the nucleotide sequence of (a); wherein at least one base pair in $N_1$ and $N_{23}$ nucleotide sequences forms a reversely complementary pair, namely, the direction of $N_1$ nucleotide sequence is 5'-3', and the direction of $N_{23}$ nucleotide sequence is 3'-5'. When the length of at least one nucleotide base of $N_1$ and $N_{23}$ is smaller than or equal to 4, at least one base pair is needed for forming the complementary pair; and when the length of at least one nucleotide base of $N_1$ and $N_{23}$ is greater than or equal to 5, at least two base pairs are needed for forming the complementary pair. Wherein, at least one base pair in $N_{14}$ and $N_{16}$ nucleotide sequences forms a reversely complementary pair, namely, the direction of $N_{14}$ nucleotide sequence is 5'-3', and the direction of $N_{16}$ nucleotide sequence is 3'-5'. When the length of at least one nucleotide base of $N_{14}$ and $N_{16}$ is smaller than or equal to 4, at least one base pair is needed for forming the complementary pair; and when the length of at least one nucleotide base of $N_{14}$ and $N_{16}$ is greater than or equal to 5, at least two base pairs are needed for forming the complementary pair. $N_{15}$ therein is a nucleotide base of any length or composition; or (c) which is at any position in the nucleotide sequence (a) with the substitution, missing and/or addition of 1 to 5 nucleotides.

The nucleic acid aptamer molecules contain substitution of the nucleotides in General Formula Carrot structure, the substitution being selected from one of the following groups: A4U, A4G, A4C, U5A, U5G, U5C, G7C, G7A, G7U, U8C, A10U, A10G, A10C, A11U, A11G, A11C, C12G, C12A, C12U, A13U, A13G, A13C, G17A, C19A, C19U, A20C, A4C/USA, A4C/USC, A4C/A11G, A4C/C12A, A4C/A13C, U5A/A11G, U5A/C12A, U5A/A13C, U5G/A13C, U5C/G7U, U5C/A11G, U5C/C12G, U5C/C12A, U5C/C12U, U5C/A13C, G7U/A13C, A11G/A13C, C12G/A13C, C12A/A13C, C12U/A13C, A4C/U5C/A13C, U5C/G7U/A13C, U5C/C12G/A13C, U5C/C12U/A13C, U5C/A11G/A13C, U5C/C12A/A13C, A4C/U5C/A11G/A13C, A4C/U5C/C12A/A13C, A4C/U5C/G7U/A11G/A13C, A4C/U5C/G7U/C12A/A13C, A4C/U5A/A11G/C12A/A13C, and A4C/U5C/A11G/C12A/A13C (which are the aptamer molecule structures in Table 1). These mutants can specifically bind to fluorophore molecules, and can significantly increase fluorescence intensity of fluorophore molecules under light excitation at suitable wavelength after binding. The nucleotide position sequence corresponds to the position shown in Fig. 1.

The afore-mentioned mutants indicate that nucleotide substitution occurs at the corresponding sites of the aptamer nucleotide sequence of the General Formula Carrot structure. For example, A4U indicates that adenine nucleotide A at the fourth position of Carrot is substituted by uridine monophosphate U, i.e. Carrot (A4U) in Table 1; U5C/A13C

indicates that U at the fifth position of Carrot is substituted by C, and A at the thirteenth position is substituted by C, i.e. Carrot (U5C/A13C) in Table 1.

Table 1: Aptamer structure of Carrot general formula structure after substitution of one nucleotide

| Substitutions of Carrot general formula structure | Aptamer structure general formula after substitution (bolded are bases after substitution) |
|---|---|
| Carrot (A4U) | $N_1$AG**U**UUGUAAACA$N_{14}$-$N_{15}$-$N_{16}$GACACU$N_{23}$ |
| Carrot (A4G) | $N_1$AG**G**UUGUAAACA$N_{14}$-$N_{15}$-$N_{16}$GACACU$N_{23}$ |
| Carrot (A4C) | $N_1$AG**C**UUGUAAACA$N_{14}$-$N_{15}$-$N_{16}$GACACU$N_{23}$ |
| Carrot (USA) | $N_1$AGA**A**UGUAAACA$N_{14}$-$N_{15}$-$N_{16}$GACACU$N_{23}$ |
| Carrot (USG) | $N_1$AGA**G**UGUAAACA$N_{14}$-$N_{15}$-$N_{16}$GACACU$N_{23}$ |
| Carrot (U5C) | $N_1$AGA**C**UGUAAACA$N_{14}$-$N_{15}$-$N_{16}$GACACU$N_{23}$ |
| Carrot (G7C) | $N_1$AGAUU**C**UAAACA$N_{14}$-$N_{15}$-$N_{16}$GACACU$N_{23}$ |
| Carrot (G7A) | $N_1$AGAUU**A**UAAACA$N_{14}$-$N_{15}$-$N_{16}$GACACU$N_{23}$ |
| Carrot (G7U) | $N_1$AGAUU**U**UAAACA$N_{14}$-$N_{15}$-$N_{16}$GACACU$N_{23}$ |
| Carrot (U8C) | $N_1$AGAUUG**C**AAACA$N_{14}$-$N_{15}$-$N_{16}$GACACU$N_{23}$ |
| Carrot (A10U) | $N_1$AGAUUGUA**U**ACA$N_{14}$-$N_{15}$-$N_{16}$GACACU$N_{23}$ |
| Carrot (A10G) | $N_1$AGAUUGUA**G**ACA$N_{14}$-$N_{15}$-$N_{16}$GACACU$N_{23}$ |
| Carrot (A10C) | $N_1$AGAUUGUA**C**ACA$N_{14}$-$N_{15}$-$N_{16}$GACACU$N_{23}$ |
| Carrot (A11U) | $N_1$AGAUUGUAA**U**CA$N_{14}$-$N_{15}$-$N_{16}$GACACU$N_{23}$ |
| Carrot (A11G) | $N_1$AGAUUGUAA**G**CA$N_{14}$-$N_{15}$-$N_{16}$GACACU$N_{23}$ |
| Carrot (A11C) | $N_1$AGAUUGUAA**C**CA$N_{14}$-$N_{15}$-$N_{16}$GACACU$N_{23}$ |
| Carrot (C12G) | $N_1$AGAUUGUAAA**G**A$N_{14}$-$N_{15}$-$N_{16}$GACACU$N_{23}$ |
| Carrot (C12A) | $N_1$AGAUUGUAAA**A**A$N_{14}$-$N_{15}$-$N_{16}$GACACU$N_{23}$ |
| Carrot (C12U) | $N_1$AGAUUGUAAA**U**A$N_{14}$-$N_{15}$-$N_{16}$GACACU$N_{23}$ |
| Carrot (A13U) | $N_1$AGAUUGUAAAC**U**$N_{14}$-$N_{15}$-$N_{16}$GACACU$N_{23}$ |
| Carrot (A13G) | $N_1$AGAUUGUAAAC**G**$N_{14}$-$N_{15}$-$N_{16}$GACACU$N_{23}$ |
| Carrot (A13C) | $N_1$AGAUUGUAAAC**C**$N_{14}$-$N_{15}$-$N_{16}$GACACU$N_{23}$ |
| Carrot (G17A) | $N_1$AGAUUGUAAACA$N_{14}$-$N_{15}$-$N_{16}$**A**ACACU$N_{23}$ |
| Carrot (C19A) | $N_1$AGAUUGUAAACA$N_{14}$-$N_{15}$-$N_{16}$GA**A**ACU$N_{23}$ |
| Carrot (C19U) | $N_1$AGAUUGUAAACA$N_{14}$-$N_{15}$-$N_{16}$GA**U**ACU$N_{23}$ |
| Carrot (A20C) | $N_1$AGAUUGUAAACA$N_{14}$-$N_{15}$-$N_{16}$GAC**C**CU$N_{23}$ |
| Carrot (A4C/USA) | $N_1$AG**CA**UGUAAACA$N_{14}$-$N_{15}$-$N_{16}$GACACU$N_{23}$ |
| Carrot (A4C/USC) | $N_1$AG**CC**UGUAAACA$N_{14}$-$N_{15}$-$N_{16}$GACACU$N_{23}$ |
| Carrot (A4C/A11G) | $N_1$AG**C**UUGUAA**G**CA$N_{14}$-$N_{15}$-$N_{16}$GACACU$N_{23}$ |
| Carrot (A4C/C12A) | $N_1$AG**C**UUGUAAA**A**A$N_{14}$-$N_{15}$-$N_{16}$GACACU$N_{23}$ |
| Carrot (A4C/A13C) | $N_1$AG**C**UUGUAAAC**C**$N_{14}$-$N_{15}$-$N_{16}$GACACU$N_{23}$ |
| Carrot (U5A/A11G) | $N_1$AGA**A**UGUAA**G**CA$N_{14}$-$N_{15}$-$N_{16}$GACACU$N_{23}$ |
| Carrot (U5A/C12A) | $N_1$AGA**A**UGUAAA**A**A$N_{14}$-$N_{15}$-$N_{16}$GACACU$N_{23}$ |
| Carrot (USA/A13C) | $N_1$AGA**A**UGUAAAC**C**$N_{14}$-$N_{15}$-$N_{16}$GACACU$N_{23}$ |
| Carrot (U5G/A13C) | $N_1$AGA**G**UGUAAAC**C**$N_{14}$-$N_{15}$-$N_{16}$GACACU$N_{23}$ |

(continued)

| Substitutions of Carrot general formula structure | Aptamer structure general formula after substitution (bolded are bases after substitution) |
|---|---|
| Carrot (U5C/G7U) | $N_1$AGA**C**U**U**UAAACA$N_{14}$-$N_{15}$-$N_{16}$GACACU$N_{23}$ |
| Carrot (U5C/A11G) | $N_1$AGA**C**UGUAA**G**CA$N_{14}$-$N_{15}$-$N_{16}$GACACU$N_{23}$ |
| Carrot (USC/C12G) | $N_1$AGA**C**UGUAAA**G**A$N_{14}$-$N_{15}$-$N_{16}$GACACU$N_{23}$ |
| Carrot (U5C/C12A) | $N_1$AGA**C**UGUAAA**A**A$N_{14}$-$N_{15}$-$N_{16}$GACACU$N_{23}$ |
| Carrot (USC/C12U) | $N_1$AGA**C**UGUAAA**U**A$N_{14}$-$N_{15}$-$N_{16}$GACACU$N_{23}$ |
| Carrot (USC/A13C) | $N_1$AGA**C**UGUAAAC**C**$N_{14}$-$N_{15}$-$N_{16}$GACACU$N_{23}$ |
| Carrot (G7U/A13C) | $N_1$AGAUU**U**UAAAC**C**$N_{14}$-$N_{15}$-$N_{16}$GACACU$N_{23}$ |
| Carrot (A11G/A13C) | $N_1$AGAUUGUAA**G**C**C**$N_{14}$-$N_{15}$-$N_{16}$GACACU$N_{23}$ |
| Carrot (C12G/A13C) | $N_1$AGAUUGUAAA**GC**$N_{14}$-$N_{15}$-$N_{16}$GACACU$N_{23}$ |
| Carrot (C12A/A13C) | $N_1$AGAUUGUAAA**AC**$N_{14}$-$N_{15}$-$N_{16}$GACACU$N_{23}$ |
| Carrot (C12U/A13C) | $N_1$AGAUUGUAAA**UC**$N_{14}$-$N_{15}$-$N_{16}$GACACU$N_{23}$ |
| Carrot (A4C/U5C/A13C) | $N_1$AG**CC**UGUAAAC**C**$N_{14}$-$N_{15}$-$N_{16}$GACACU$N_{23}$ |
| Carrot (U5C/G7U/A13C) | $N_1$AGA**C**U**U**UAAAC**C**$N_{14}$-$N_{15}$-$N_{16}$GACACU$N_{23}$ |
| Carrot (U5C/C12G/A13C) | $N_1$AGA**C**UGUAAA**GC**$N_{14}$-$N_{15}$-$N_{16}$GACACU$N_{23}$ |
| Carrot (U5C/C12U/A13C) | $N_1$AGA**C**UGUAAA**UC**$N_{14}$-$N_{15}$-$N_{16}$GACACU$N_{23}$ |
| Carrot (U5C/A11G/A13C) | $N_1$AGA**C**UGUAA**G**C**C**$N_{14}$-$N_{15}$-$N_{16}$GACACU$N_{23}$ |
| Carrot (USC/C12A/A13C) | $N_1$AGA**C**UGUAAA**AC**$N_{14}$-$N_{15}$-$N_{16}$GACACU$N_{23}$ |
| Carrot (A4C/U5C/A11G/A13C) | $N_1$AG**CC**UGUAA**G**C**C**$N_{14}$-$N_{15}$-$N_{16}$GACACU$N_{23}$ |
| Carrot (A4C/U5C/C12A/A13C) | $N_1$AG**CC**UGUAAA**AC**$N_{14}$-$N_{15}$-$N_{16}$GACACU$N_{23}$ |
| Carrot (A4C/U5C/G7U/A11G/A13C) | $N_1$AG**CC**U**U**UAA**G**C**C**$N_{14}$-$N_{15}$-$N_{16}$GACACU$N_{23}$ |
| Carrot (A4C/U5C/G7U/C12A/A13C) | $N_1$AG**CC**U**U**UAAA**AC**$N_{14}$-$N_{15}$-$N_{16}$GACACU$N_{23}$ |
| Carrot (A4C/U5A/A11G/C12A/A13C) | $N_1$AG**CA**UGUAA**GAC**$N_{14}$-$N_{15}$-$N_{16}$GACACU$N_{23}$ |
| Carrot (A4C/U5C/A11G/C12A/A13C) | $N_1$AG**CC**UGUAA**GAC**$N_{14}$-$N_{15}$-$N_{16}$GACACU$N_{23}$ |

Aptamer molecules are single-stranded nucleic acid molecules that have a secondary structure (Fig. 1) consisting of one or more base pairing regions (stems) and one or more unpaired regions (loops). The nucleic acid molecules of the present disclosure contain a secondary structure as predicted in Fig. 1. The 5' end or 3' end of the structure can be fused with any objective RNA molecule for extracellular or intracellular detection of the target RNA molecules. In a preferable embodiment of the present disclosure, the 5' end of the nucleic acid aptamer molecule is fused with an ACTB RNA sequence (Genebank: BC016045); in another preferable embodiment of the present disclosure, the 5' end of the nucleic acid aptamer molecule is fused with a GAPDH RNA sequence (Genebank: BC009081).

The stem-loop structure ($N_{14}$-$N_{15}$-$N_{16}$) in Fig. 1 plays the role of stabilizing the entire nucleic acid aptamer molecule structure, and can be replaced with other nucleotide sequences of any length and any composition that can form stem-loop structures. The aptamer molecules of the present disclosure may also contain other nucleotide sequences that can be inserted into the position of $N_{14}$-$N_{15}$-$N_{16}$, wherein the inserted nucleotide sequence replaces the stem-loop structure ($N_{14}$-$N_{15}$-$N_{16}$) in Fig. 1. The nucleotide sequence can specifically identify/bind to target molecules. In the absence of target molecules, the binding of aptamer molecules to fluorophore molecules is weak, as a result of which the fluorophore molecules show weak fluorescence light; in the presence of target molecules, the binding of target molecules to the aptamer will promote the binding of the aptamer to fluorophore molecules, and thus can significantly enhance the fluorescence intensity of fluorophore molecules under light excitation at suitable wavelength. The target molecules can be small molecules, and signal molecules on the cell surface, etc. These nucleic acid aptamers bind to specific target molecules through non-covalent binding, which mainly depends on intermolecular ionic forces, dipole force, hydrogen bonds, Van der Waals forces, positron and negative electron interactions, stacking or the combination

of the above forces. The stem-loop structure ($N_{14}$-$N_{15}$-$N_{16}$) can be replaced with an RNA sequence that identifies the target molecules for extracellular or intracellular detection of the target molecules.

In a preferable embodiment of the present disclosure, the nucleic acid aptamer molecules are preferably SEQ ID NO: 1, 2, 3, 4, 5, 6, 7, 8, 9, 10, 11, 12, 13, 14, 15, 16, 17, 18, 19, 20, 22, 24, 25, 26, 27, 28, 29, 30 or 31, or their mutation sequence which can bind fluorophore molecules so as to significantly enhance the fluorescence of fluorophore molecules under light excitation at suitable wavelength.

The nucleic acid aptamer molecules of the present disclosure can also comprise a fragment of nucleotide sequence that increases its stability. In a preferable embodiment of the present disclosure, F30 scaffold RNA (Sequence 3) was adopted, and its connection mode with the nucleic acid aptamer molecules is shown in Fig. 3; in another preferable embodiment of the present disclosure, tRNA scaffold RNA (Sequence 4) was adopted, and its connection mode with the nucleic acid aptamer molecules is shown in Fig. 4.

Identity

"Identity" describes the correlation between two nucleotide sequences in the present disclosure. The calculation of identity of two aptamer nucleotide sequences in the present disclosure does not include $N_1$, $N_{14}$, $N_{15}$, $N_{16}$, $N_{23}$ in Sequence (a). As for the present disclosure, identity of two aptamer nucleotide sequences is determined by using, for instance, Needle program, preferably Needleman-Wunsch Algprithm (Needleman and Wunsch, 1970, J.Mol.Biol.48: 443-453) executed in 3.0.0 version or later, of EMBOSS software package (EMBOSS: The European Molecular Biology Open Software Suite, Rice etc., 2000, Trends in Genetics 16: 276-277). Optional parameters in use are gap penalty 10, gap extension penalty 0.5 and EBLOSUM62 substitution matrix (EMBOSS version of BLOSUM62). Output result marked by Needle as "longest identity" (obtained by using the "-nobrief" option) serves as the percentage identity, and is calculated in a way as follows:

$$(\text{Identical residue}\times 100)/(\text{Alignment length-Total number of gaps in alignment}).$$

For instance, the sequences of Carrot-1 and Carrot-1 (U5C) in Table 1 of the present disclosure are $N_1$AGAUUGUAAACA$N_{14}$-$N_{15}$-$N_{16}$GACACU$N_{23}$ and $N_1$AGA**C**UGUAAACA$N_{14}$-$N_{15}$-$N_{16}$GACACU$N_{23}$, and, according to the definition of the present disclosure, their identity alignment should not include the nucleotide bases of $N_1$, $N_{14}$-$N_{15}$-$N_{16}$ and $N_{23}$, so the alignment result of their sequence identity is 94.4% (the difference being one nucleotide).

Fluorophore molecules

The "fluorophore molecules" in the present disclosure are also called as "fluorophore" or "fluorescence molecules". "Fluorophore molecules" in the present disclosure are a kind of fluorophore molecules that can be conditionally activated, and show a relatively low quantum yield in the absence of nucleic acid aptamers. In specific embodiments, when a fluorophore is not bound to specific aptamers, its quantum yield is lower than 0.1, preferably lower than 0.01, and optimally lower than 0.001; when the fluorophore is bound to specific aptamers, its quantum yield will be enhanced by more than two times, preferably by more than 10 times, and optimally by more than 100 times. Fluorophore molecules are preferably water-soluble, non-toxic to cells and easy to penetrate membranes. Fluorophore of the present disclosure can preferably enter cytoplasm or pericytoplasm through membrane or cell wall by means of active transport or passive diffusion. In the embodiments of the present disclosure, the fluorophore can penetrate outer and inner membranes of Gram-negative bacteria, cell walls and membranes of plant cells, cell walls and membranes of fungi, membranes of animal cells, and GI and endothelial membranes of living animals.

The nucleic acid aptamer molecules in the present disclosure can specifically bind to a fluorophore and significantly increase its fluorescence value under light excitation at specific wavelength. The fluorophore molecules are selected from the structure (I) below:

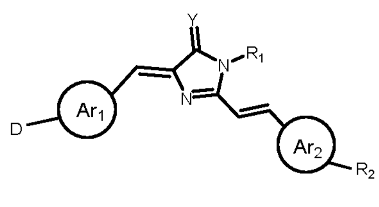

(I)

(I) wherein: $Ar_1$, and $Ar_2$ are independently hexahydric aryl group and hexahydric heteroaryl group; D- is HO- or $N(X_1)(X_2)$-, and $X_1$ and $X_2$ are respectively and independently selected from hydrogen, alkyl and modified alkyl; $X_1$ and $X_2$ are optionally interconnected and form an alicyclic heterocycle with N atoms; when D- is $N(X_1)(X_2)$- and $Ar_1$ is phenyl group, $X_1$ and $X_2$ independently form a satured or unsatured alicyclic heterocycle with a benzene ring; when D- is HO- and $Ar_1$ is phenyl group, at least one hydrogen atom adjacent to HO- is substituted by halogen; Y is O and S; $R_1$ is hydrogen, alkyl or modified alkyl; and $R_2$ is a hydrogen atom, a halogen atom, -OH, or -CN ;

wherein: the "alkyl" is respectively and independently $C_1$-$C_{10}$ straight or branched alkyl; optionally, the "alkyl" is $C_1$-$C_7$ straight or branched alkyl; optionally, the "alkyl" is $C_1$-$C_5$ straight or branched alkyl; optionally, the "alkyl" is selected from methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl or 2,2,3-trimethylbutyl;

wherein: the "modified alkyl" is a group obtained by replacing any carbon atom of the alkyl with one or more groups selected from halogen atoms, -OH, -CO-, -O-, -CN, - $SO_3H$, primary amino group, secondary amino group, and tertiary amino group, and the modified alkyl has 1 to 10 carbon atoms, wherein the carbon-carbon single bond is optionally and independently replaced by a carbon-carbon double bond or a carbon-carbon triple bond;

wherein: the replacement of carbon atoms refer to that carbon atoms are replaced with corresponding group or that carbon atoms, together with hydrogen atoms thereon, are replaced with corresponding group; the "modified alkylene" is a group obtained by replacing any carbon atom of $C_1$-$C_{10}$ (preferably $C_1$-$C_6$) alkylene with groups selected from -O-, -OH, - CO-, -CS-, -(S=O)-;

optionally, the "modified alkyl" contains one or more groups selected from -OH, -O-, - $(CH_2CH_2O)_n$-, monosaccharide unit, -O-CO-, -NH-CO-, $-SO_2-O-$, -SO-, $Me_2N$-, $Et_2N$-, -S-S-, - CH=CH-, F, Cl, Br, I, and cyano group;

optionally, $Ar_1$ is selected from structures represented by the following formulae ( II -1) to ( II -15):

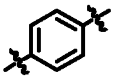 ( II -1), 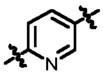 ( II -2), 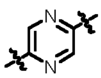 ( II -3), 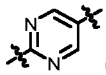 ( II -4), 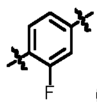 ( II -5), 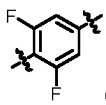 ( II -6),

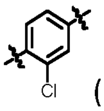 ( II -7), 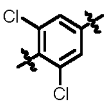 ( II -8), 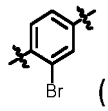 ( II -9), 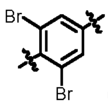 ( II -10), 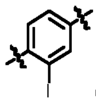 ( II -11), 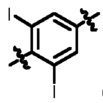 ( II -12).

$Ar_2$ is selected from structures represented by the following formulae (III-1) to (III-25):

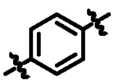 (III-1), 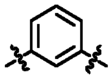 (III-2), 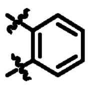 (III-3), 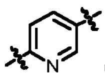 (III-4), 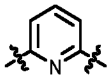 (III-5), 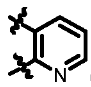 (III-6),

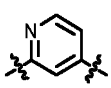 (III-7), 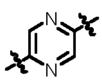 (III-8), 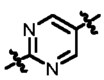 (III-9).

Optionally, the compound represented by Formula (I) is selected compounds represented by the following formulae:

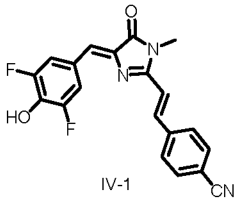
IV-1,
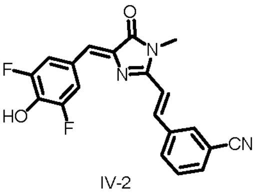
IV-2,
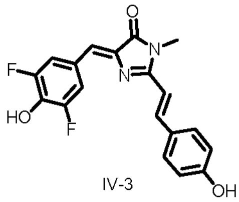
IV-3,
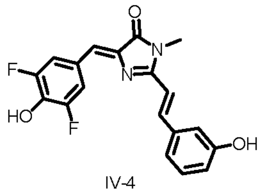
IV-4,

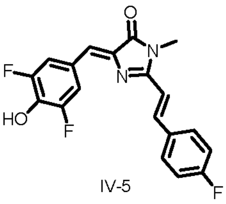
IV-5,
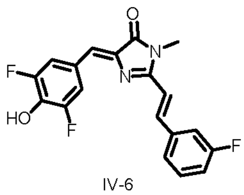
IV-6,
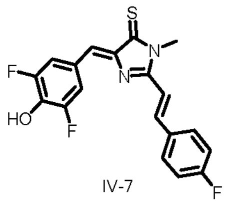
IV-7,
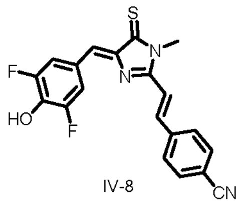
IV-8,

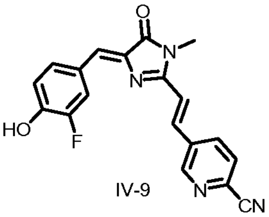
IV-9,
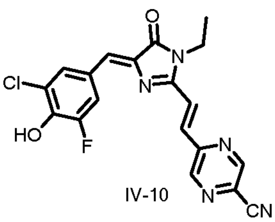
IV-10,
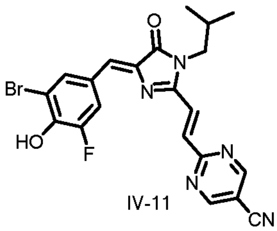
IV-11,
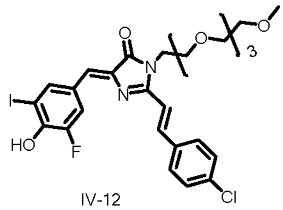
IV-12,

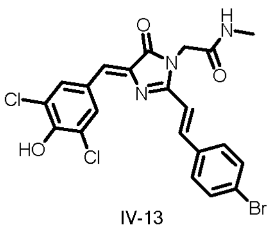
IV-13,
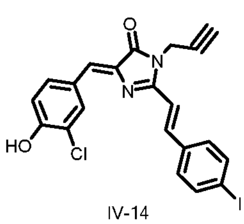
IV-14,
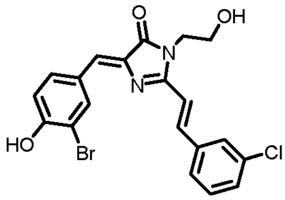
IV-15,
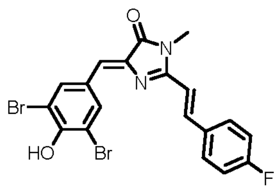
IV-16,

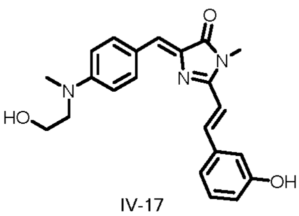
IV-17,
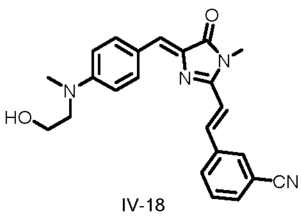
IV-18,
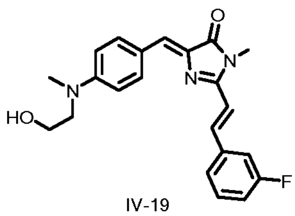
IV-19,

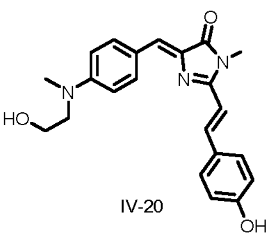
IV-20,
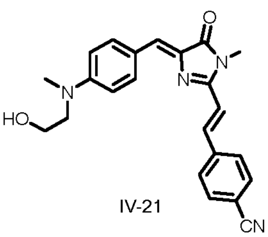
IV-21,
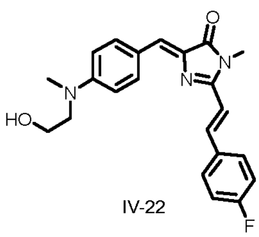
IV-22,
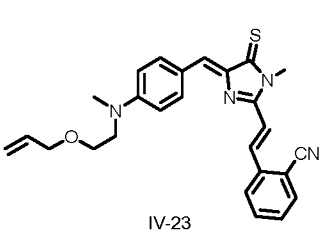
IV-23,

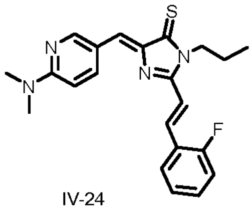
IV-24 ,
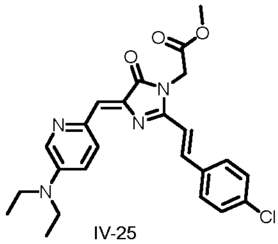
IV-25 ,
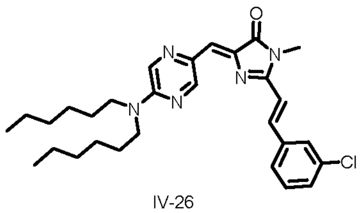
IV-26 ,
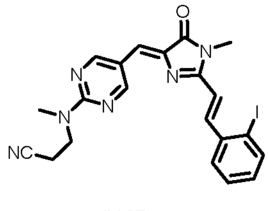
IV-27 ,

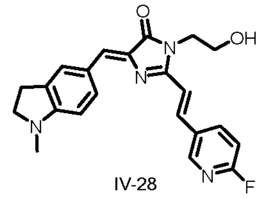
IV-28 ,
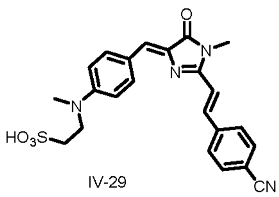
IV-29 ,
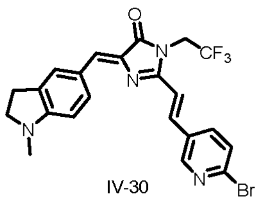
IV-30 ,

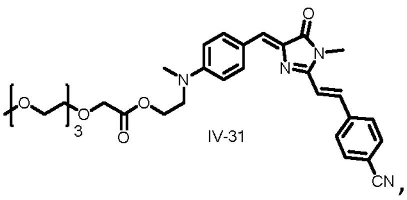
IV-31 ,
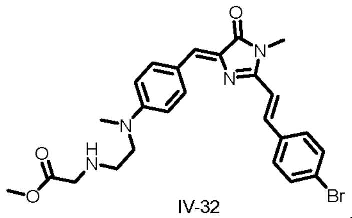
IV-32 ,
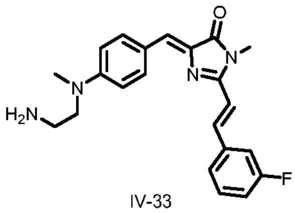
IV-33 ,

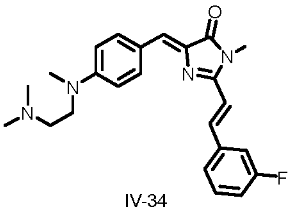
IV-34 ,
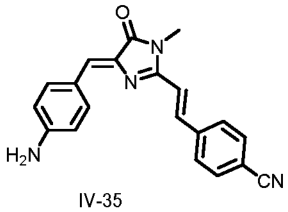
IV-35 ,
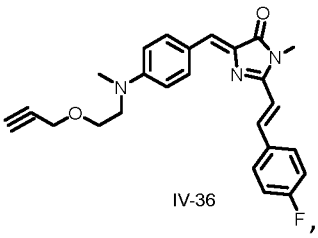
IV-36 ,
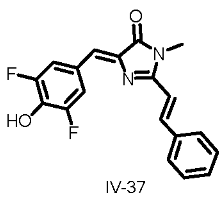
IV-37 ,

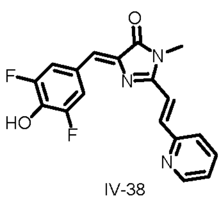
IV-38 ,
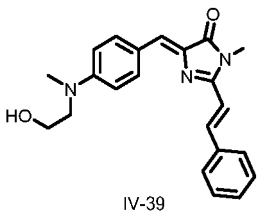
IV-39 ,
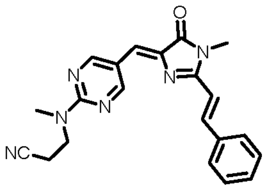
IV-40 .

In preferable embodiments of the present disclosure, the fluorophore molecules include: IV-1, IV-2, IV-3, IV-4, IV-5, IV-6, IV-7, IV-8, IV-9, IV-10, IV-11, IV-12, IV-13, IV-14, IV-15, IV-16, IV-17, IV-18, IV-19, IV-20, IV-21, IV-22, IV-23, IV-24, IV-25, IV-26, IV-27, IV-28, IV-29, IV-30, IV-31, IV-32, IV-33, IV-34, IV-35, IV-36, IV-37, IV-38, IV-39, IV-40. The expressions such as "improving fluorescence signals", "fluorescence increase", "enhancing fluorescence intensity", "improving fluorescence intensity" in the present disclosure refers to the increase of the quantum yield of the fluorophore or the migration (relative to emission peaks of fluorophore itself in ethanol or aqueous solution) of the maximum emission peak of fluorescence signals under light excitation at suitable wavelength, or an increase of molar extinction coefficient, or two or more of the above. In a preferable embodiment of the present disclosure, the quantum yield is increased by at least two times; in another preferable embodiment of the present disclosure, the quantum yield is increased by at least 5 to 10 times; in another more preferable embodiment of the present disclosure, the quantum yield is increased by at least 20 to 50 times; in another more preferable embodiment of the present disclosure, the quantum yield is increased by at least 100 to 200 times; in another more preferable embodiment of the present disclosure, the quantum yield is increased by at least 200 times. The light source used for exciting the fluorophore to produce fluorescence signals

can be any appropriate lighting device, such as LED lamp, incandescent lamp, fluorescent lamp and laser; excitation light can be either emitted directly from these devices or obtained indirectly by means of other fluorophores, such as donor fluorophores of fluorescence resonance energy transfer (FRET), or donor luminophors of bioluminescence resonance energy transfer (BRET).

## Target molecules

The target molecules of the present disclosure can be any biomaterial or small molecules, including but not limited to: proteins, nucleic acid (RNA or DNA), lipid molecules, carbohydrates, hormones, cytokines, chemokines, metabolite, metal ions and so on. Target molecules can be molecules associated with diseases or pathogen infection.

In the structure shown in Fig. 1, the inserted nucleotide sequence replaced the stem-loop structures of $N_{14}$-$N_{15}$-$N_{16}$ in Fig. 1 by means of the aptamer molecules in the present disclosure, wherein the nucleotide sequence can specifically indentify/bind to target molecules. In the absence of target molecules, aptamer molecules do not or weakly bind to fluorophore molecules, and thus cannot significantly improve the fluorescence of fluorophore molecules under light excitation at suitable wavelength; in the presence of target molecules, the binding of target molecules to the nucleotide sequence will promote binding of the aptamer molecules to fluorophore molecules, and thus can significantly improve the fluorescence of fluorophore molecules under light excitation at suitable wavelength, thereby realizing detection, imaging and quantitative analysis of target molecules.

Target molecules can also be whole cells or molecules expressed on the entire cell surface. Typical cells include but are not limited to cancer cells, bacterial cells, fungal cells and normal animal cells. The target molecules can also be virus particles. At present, many aptamers of the afore-mentioned target molecules have been identified, and can be integrated into the polyvalent nucleic acid aptamers of the present disclosure. RNA aptamers that have been reported to bind to target molecules include but are not limited to: T4 RNA polymerase aptamer, HIV reverse transcriptase aptamer, and phage R17 capsid protein aptamer.

## Objective nucleic acid molecules

"Objective nucleic acid molecules", also called "target nucleic acid molecules", refer to the nucleic acid molecules to be detected, which can be either intracellular or extracellular; objective nucleic acid molecules include objective RNA molecules and objective DNA molecules. In the present disclosure, objective nucleic acid molecules are connected with the nucleic acid aptamer molecules, and are bound to the nucleic acid aptamer molecules via fluorophore molecules so as to significantly improve the fluorescence value of fluorophore molecules under light excitation at suitable wavelength, thereby detecting the content and distribution of objective nucleic acid molecules.

"Objective RNA molecules" in the present disclosure include any RNA molecule, including but not limited to pre-mRNA, and mRNA, pre-rRNA, rRNA, tRNA, hnRNA, snRNA, miRNA, siRNA, shRNA, sgRNA, crRNA and lncRNA for coding cells per se or exogenous expression products thereof, wherein phage capsid protein MCP identifies the binding sequence MS2RNA, phage capsid protein PCP identifies the binding sequence PP7 RNA, λ phage transcription termination protein N identifies the binding sequence boxB RNA or the like. Target RNA can be fused at 5' end or 3' end or the position of $N_{14}$-$N_{15}$-$N_{16}$ of the RNA aptamer molecules of the present disclosure.

"sgRNA" in the present disclosure refers to single guide RNA (single guide RNA, sgRNA) formed by modifying tracrRNA and crRNA in the CRISPR/Cas9 system, wherein the sequence of about 20 nt at the 5' end of the system targets DNA site via base pair complementation, and promotes the Cas9 protein to induce DNA double-strand break at this site

## Concatemers of nucleic acid aptamer

The nucleic acid aptamer molecules of the present disclosure may further include concatemers that can bind multiple fluorophore molecules. The concatemers are connected by spacer sequences of appropriate length, and the number of Carrot structures in series may be 2, 3, 4, 5, 6, 7, 8, 9, 10 or greater. The concatermers may be in many forms. In a preferable embodiment of the present disclosure, the series form is "Series 1", as shown in Fig. 7a, and a preferable nucleotide sequence is SEQ ID NO: 7, 8, 9, 10, or 11, wherein 2Carrot-2 indicates Concatemer 1 with 2 Carrot-2 structures; in another preferable embodiment of the present disclosure, the series form is "Series 2", as shown in Fig. 7b, and a preferable nucleotide sequence is SEQ ID NO: 12, 13, 14, 15 or 16, wherein 2×Carrot-2 indicates Concatemer 2 with 2 Carrot-2 structures; in another preferable embodiment of the present disclosure, the series form is "Series 3", as shown in Fig. 7c, and a preferable nucleotide sequence is SEQ ID NO: 17, 18, 19 or 20, wherein 2×2Carrot-2 indicates Concatemer 3 with 4 Carrot-2 structures; in any form, the spacer sequences between the concatemers can be changed.

The monomer aptamers of the present disclosure refer to aptamer containing only one Carrot structure, that

is, only one aptamer as shown in Fig. 1 is contained.

The polymer aptamers refer to aptamers containing more than one Carrot structure, including but not limited to the aptamer composed of several series forms as shown in Fig. 7.

Aptamer-fluorophore complex

The aptamer-fluorophore complex of the present disclosure contains one nucleic acid aptamer molecule and one or more fluorophore molecules. In an embodiment of the present disclosure, molecule complexes containing one nucleic acid molecule and one fluorophore molecule are as follows: Carrot-1-IV-1, Carrot-1-IV-2, Carrot-1-IV-3, Carrot-1-IV-4, Carrot-1-IV-5, Carrot-1-IV-6, Carrot-1-IV-7, Carrot-1-IV-8, Carrot-1-IV-9, Carrot-1-IV-10, Carrot-1-IV-11, Carrot-1-IV-12, Carrot-1-IV-13, Carrot-1-IV-14, Carrot-1-IV-15, Carrot-1-IV-16, Carrot-1-IV-17, Carrot-1-IV-18, Carrot-1-IV-19, Carrot-1-IV-20, Carrot-1-IV-21, Carrot-1-IV-22, Carrot-1-IV-23, Carrot-1-IV-24, Carrot-1-IV-25, Carrot-1-IV-26, Carrot-1-IV-27, Carrot-1-IV-28, Carrot-1-IV-29, Carrot-1-IV-30, Carrot-1-IV-31, Carrot-1-IV-32, Carrot-1-IV-33, Carrot-1-IV-34, Carrot-1-IV-35, Carrot-1-IV-36, Carrot-1-IV-37, Carrot-1-IV-38, Carrot-1-IV-39, and Carrot-1-IV-40.

In another embodiment of the present disclosure, nucleic acid molecules of the concatemer and a plurality of fluorophore molecules form a complex, for instance, complexes formed in the way of "Series 1" by F30-4Carrot-2 containing 4 aptamer units and 4 fluorophore molecules, namely, F30-4Carrot-2-4×(IV-1), F30-4Carrot-2-4×(IV-2), F30-4Carrot-2-4×(IV-3), F30-4Carrot-2-4×(IV-4), F30-4Carrot-2-4×(IV-5), F30-4Carrot-2-4×(IV-6), F30-4Carrot-2-4×(IV-37), F30-4Carrot-2-4×(IV-17), F30-4Carrot-2-4×(IV-18), F30-4Carrot-2-4×(IV-19), F30-4Carrot-2-4×(IV-20), F30-4Carrot-2-4×(IV-21) and F30-4Carrot-2-4×(IV-22). The molecule complexes may exist in vitro in the form of two separate solutions, or in the same solution, or in cells.

Nucleic acid aptamer function

The aptamer function of the present disclosure means to significantly enhance fluorescence intensity of fluorophore molecules under light excitation at suitable wavelength, and aptamers can be detected by function detection of nucleic acid aptamer as shown in common Experimental Method (V) in the embodiments. In a preferable embodiment of the present disclosure, the fluorescence intensity is increased by at least two times (the fluorescence intensity is detected according to the Experimental Method (V)); in another preferable embodiment of the present disclosure, the fluorescence intensity is increased by at least 5 to 10 times; in another more preferable embodiment of the present disclosure, the fluorescence intensity is increased by at least 20 to 50 times; in another more preferable embodiment of the present disclosure, the fluorescence intensity is increased by at least 100 to 200 times; in another more preferable embodiment of the present disclosure, the fluorescence intensity is increased by at least 200 times.

Secondary structure of nucleic acid aptamers

In the present disclosure, the secondary structure of nucleic acid aptamers is obtained by simulation and prediction using mFold online analysis software (http://unafold.rna.albany.edu/?q=mfold). The stem structure in the secondary structure refers to a local double-strand structure formed by complementary pairing of hydrogen bonds in some regions of the single strand of nucleic acid aptamer molecules. In general, the formation of the double-strand structure does not require complementary pairing of all nucleotides in this region; in general, the stem structure will be formed when complementary pairing occurs between at least 50% of the nucleotides in a fragment of sequences $N_1$ and $N_{23}$, as well as $N_{14}$ and $N_{16}$ and the other fragment. If $N_1$ and $N_{23}$ are single nucleotides, the stem structure can be formed with complete complement of $N_1$ and $N_{23}$ (as shown in Fig. 1).

DNA molecules expressing nucleic acid aptamers

The DNA molecules contain a DNA sequence which can encode the nucleic acid aptamer molecules of the present disclosure. The DNA molecules contain a nucleotide sequence $R_1$AGATTGTAAACA$R_{14}$-$R_{15}$-$R_{16}$GACACT$R_{23}$, as well as a nucleotide sequence with identity of at least 70%, wherein $R_1$ encodes $N_1$ in the General Formula Carrot structure, $R_{14}$ encodes $N_{14}$ in the General Formula Carrot structure, $R_{15}$ encodes $N_{15}$ in the General Formula Carrot structure, $R_{16}$ encodes $N_{16}$ in the General Formula Carrot structure, and $R_{23}$ encodes $N_{23}$ in the General Formula Carrot structure. The DNA molecules may also contain a promoter which controls DNA transcription, wherein the promoter is in operable connection to the DNA sequence encoding the nucleic acid aptamer. In an embodiment of the present disclosure, the DNA molecule contains an U6 promoter; in another embodiment of the present disclosure, the DNA molecules contain a CMV promoter. Besides the afore-mentioned DNA molecules, DNA molecules may further contain a DNA sequence which encodes any objective nucleic acid molecule. In an embodiment of the present disclosure, the DNA molecules encoding the objective RNA contain a DNA sequence (sequences for embedding RNA are respectively

SEQ ID No: 26, 27) for encoding glyceraldehyde-3-phosphate dehydrogenase (GAPDH) or β-actin.

Promoters

"Promoters" in the present disclosure include promoters of eukaryotic and prokaryotic cells. Promoter sequences of eukaryotic cells are totally different from those of prokaryotic cells. Generally, eukaryotic promoters cannot be identified by RNA polymerases in prokaryotic cells or mediate RNA transcription. Similarly, prokaryotic promoters cannot be identified by RNA polymerases in eukaryotic cells or mediate RNA transcription either. The strength of different promoters varies greatly (strength refers to the ability to mediate transcription). According to actual application, strong promoters can be used for achieving high level transcription. For instance, high level expression is better for labeling, and, for evaluation of transcription behavior, lower-level transcription will allow cells to adjust transcription in a timely manner. One or more suitable promoters can be selected for different host cells. For instance, when used in Escherichia coli cells, T7 phage promoter, lac promoter, trp promoter, recA promoter, ribosome RNA promoter, PR and PL promoters in λ phage, and other promoters, but not limited to: lacUV5 promoter, ompF promoter, bla promoter, Ipp promoter etc. Moreover, a hybrid trp-lacUV5 promoter (tac promoter) or other Escherichia coli cells obtained through recombinant or synthetic DNA technology can all be used for transcribing the RNA aptamers of the present disclosure. Some of the operator sequences in bacteria per se can combine with promoter sequences to form inducible promoters, and specific inducers need to be added at this moment to induce transcription of DNA molecules. For instance, the expression of lac operator needs to be induced by the addition of lactose or lactose analogues (IPTG), and other operators include trp, pro or the like.

As mentioned above, the regulating sequence of 5' end of DNA molecule decoding sequence is promoters. Suitable promoters need to be selected according to the promoter intensity either to obtain RNA apatmers via in vitro transcription or to express aptamers in cultured cells or tissues. Since the expression of aptamers in vivo can be genetically manipulated, another type of promoters is inducible promoters that induce DNA transcription in response to a specific environment, such as in a specific tissue, at a specific time, and in a specific developmental stage. These different promoters can be identified by RNA polymerase I, II or III.

Promotion of transcription in eukaryotic cells also needs suitable promoters, including but not limited to β-globulin promoter, CAG promoter, GAPDH promoter, β-actin promoter, actin promoter, Cstf2t promoter, SV40 promoter, PGK promoter, MMTV promoter, adenovirus Ela promoter, CMV promoter and so on. Termination of transcription in eukaryotic cells depends on the specific cleavage site in RNA sequence. Similarly, since the transcription genes of RNA polymerase are different, transcriptional terminators also vary significantly. However, those skilled in the art can realize screening of suitable 3' transcriptional terminator subregions by means of basic experimental skills.

Expression system

The "expression system" of the present disclosure, also called "expression vector", contains and is integrated with DNA molecules expressing nucleic acid aptamers. The expression system of the present disclosure can be a plasmid or a virus particle.

Recombinant virus of "expression vector" can be obtained by transfection of plasmids into viral-infected cells. Suitable vectors include but are not limited to virus vectors such as λ vector system gt11, gt WES.tB and Charon 4; and plasmid vectors include pBR322, pBR325, pACYC177, pACYC184, pUC8, pUC9, pUC18, pUC19, pLG399, pR290, pKC37, pKC101, pBluescript II SK+/- or KS+/- (see Stratagene cloning system), pET28 series, pACYCDuet1, pCDFDuet1, pRSET series, pBAD series, pQE, pIH821, pGEX, pIIIEx426 RPR and so on.

A large number of host expression systems can be used for expressing the DNA molecules of the present disclosure. Mostly, the vector system has to be compatible to the host cells in use, wherein the host vector system includes but is not limited to: transformed phage DNA, or plasmid DNA, or bacteria with cosmid DNA; yeast containing yeast vector; mammalian cells infected with a virus (e.g. adenovirus, adeno-associated virus, retrovirus); mammalian cells transfected with plasmids; insect cells infected with a virus (e.g. baculovirus); and plant cells infected with bacteria or transformed by means of particle bombardment. Expression elements in the vectors are significantly different in strength and characteristics. Any one or more suitable transcription elements can be selected according to the host-vector system in use.

Once the constructed DNA molecules are cloned into the vector system, it will be easy to transfer them into host cells. Based on different vector or host cell systems, the method includes but is not limited to transformation, transduction, conjugation, fixation, electrical transfer or the like.

An embodiment of the present disclosure provides expression plasmids pET28a-T7-F30-Carrot-2 and pYES2.1-F30-Carrot-2 containing DNA molecules for encoding F30-Carrot-2 RNA. Another embodiment of the present disclosure provides an expression plasmid pU6-tRNA-Carrot-2 containing DNA molecules for encoding tRNA-Carrot-2 RNA. Another embodiment of the present disclosure provides an expression plasmid pU6-F30-4Carrot-2 containing DNA molecules for encoding F30-4Carrot-2 RNA. Another embodiment of the present disclosure provides expression plasmids pCDNA3.1 hygro(+)-GAPDH-4Carrot-2 and pCDNA3.1 hygro(+)-ACTB-4Carrot-2 containing DNA molecules for encoding GAPDH-4Carrot-2 and ACTB-4Carrot-2. Another embodiment of the present disclosure provides expression plasmids psgRNA-Carrot-2-1, psgRNA-Carrot-2-2 and psgRNA-Carrot-2-3 containing DNA molecules for encoding sgRNA-Carrot-2-1, sgRNA-Carrot-2-2 and sgRNA-Carrot-2-3. Another embodiment of the present disclosure provides an expression plasmid pU6-Carrot-1-MS2 containing DNA molecules for encoding Carrot-1-MS2.

The present disclosure further provides expression vectors integrated with DNA molecules for encoding nucleic acid aptamers, but with vacant encoding DNA sequences of objective RNA molecules, wherein the vacancy of encoding DNA sequences of objective RNA molecules allows the users to choose DNA sequences of objective RNA molecules to be detected, for instance, corresponding encoding DNA sequence of GAPDH mRNA inserts the DNA sequence into the expression vector of the present disclosure by means of standard recombination DNA technology, and guides the obtained expression vector into the host cells of (transfection, transform, infection and so on), thereby detecting the content and distribution of objective RNA.

Host cells

"Host cells" in the present disclosure include but are not limited to bacteria, yeast, mammalian cells, insect cells, plant cells, zebra fish cells, fruit fly cells, and nematode cells. Host cells preferably are cultured cells in vitro or whole in vivo living tissue. Mammalian cells contained in the host cells of the present disclosure include but are not limited to 297T, COS-7, BHK, CHO, HEK293, HeLa, H1299, stem cells of fertilized eggs, inducible totipotent stem cell, and primary cells isolated directly from mammalian tissues and so on; escherichia coli cells contained therein include but are not limited to BL21 (DE3), BL21 (DE3, Star), TOP10, Mach1, and DH5$\alpha$; and yeast cells contained therein include but are not limited to BY4741, BY4742, and AH109.

Detection array

The detection array of the present disclosure includes one or more nucleic acid aptamer molecules of the present disclosure, wherein the nucleic acid aptamer molecules are anchored at discrete locations on the array surface composed of solid supports, including but not limited to glass, metals, and ceramic and so on. The nucleic acid aptamer molecules of the present disclosure can be anchored to the array surface by, but not limited to, the following methods: (1) labeling the 5' end or 3' end of the nucleic acid aptamer molecule with biotin, coating the array surface with streptavidin, and anchoring the nucleic acid aptamer molecule by specific binding of biotin and streptavidin; (2) identifying the binding sequence MS2 by using the phage capsid protein MCP, identifying the biding sequence PP7 by using the phage capsid protein PCP or identifying the binding sequence boxB by using the $\lambda$ phase transcription terminating protein N, fusing the RNA sequence at the 5', 3' or stem-loop structure of the nucleic acid aptamer molecules, coating the array surface with protein MCP, PP7 or $\lambda_N$ protein identified and bound thereby, and anchoring the nucleic acid aptamer molecules through the specific effects of MS2 with MCP protein, PP7 with PCP protein or boxB RNA with $\lambda_N$ protein; (3) fusing a fragment of RNA or DNA sequence at the 5' end or 3' end of the nucleic acid aptamer molecules, anchoring an RNA sequence in complementary pairing with the RNA sequence segment or an DNA sequence in complementary pairing with the DNA sequence segment on the array surface, and anchoring the nucleic acid aptamer molecules on the array surface by means of the molecular hybridization principle. The detection array can be used for detecting the presence or absence of the target molecule as well as the concentration level, as a result, the nucleic acid aptamer molecules be bound with the fluorophore molecules and significantly improve the fluorescence intensity under light excitation at suitable wavelength only with the presence of target molecules; and, within a certain range, the higher the concentration of the target molecules, the higher the fluorescence intensity.

Kit

Kit of the present disclosure includes the nucleic acid aptamer molecules and/or the fluorophore molecules of the present disclosure, and corresponding instructions; or includes an expression system for expressing the nucleic acid aptamer molecules and/or the fluorophore molecules, and corresponding instructions; or includes host cells expressing the aptamer molecular expression system and/or the fluorophore molecules, and corresponding instructions. The nucleic acid aptamer molecules and the fluorophore molecules in the kits respectively exist in individual solutions, or exist in the same solution.

**Embodiments**

The present disclosure will be further elaborated in the following examples, which are merely used for giving

examples, rather than limiting the scope of the present disclosure. The examples mainly adopt conventional cloning methods of molecular biology in genetic engineering, which are well known to ordinary technicians in this field, for instance, relevant chapters from *Lab Ref: A Handbook of Recipes, Reagents, and Other Reference Tools for Use at the Bench* by Jane Roskams et al, and Molecular Cloning-A Laboratory Manual (Third Edition, August 2002, Science Press, Beijing) written by Sambrook.J, D. W. Russell and translated by Peitang HUANG et al. Based on the following examples, it is easy for one skilled in the art to successfully implement the present disclosure after slight amendment and change made according to actual situations.

In the examples, the pCDNA3.1 hygro (+) plasmid vector was purchased from Invitrogen Company, pLKO.1-puro plasmid vector was purchased from Sigma Company, pET28a plasmid vector was purchased from Novagen Company, and pYES2.1 TOPO TA plasmid vector was purchased from Invitrogen. All primers used for PCR were synthesized, purified and identified correct via mass spectrometry by Shanghai Generay Biotech Co., Ltd. Expression plasmids constructed in the examples all went through the sequence determination accomplished by JIE LI BIOLOGY. Taq DNA polymerase used in all examples was purchased from Yeasen Biotechnology (Shanghai) Co., Ltd., PrimeSTAR DNA polymerase was purchased from TaKaRa Company, and corresponding polymerase buffers and dNTP were included during purchasing of these three kinds of polymerases. EcoRI, BamHI, BglII, HindIII, NdeI, XhoI, SacI, XbaI, SpeI and other restriction endonuclease, T4 ligase, T4 phosphorylase (T4 PNK), and T7 RNA polymerase were purchased from Fermentas Company, and corresponding polymerase buffers and so on were included during purchasing. Hieff Clone™ One Step cloning kits used in the examples were purchased from Yeasen Biotechnology (Shanghai) Co., Ltd. Unless otherwise stated, chemical reagents such as inorganic salts were all purchased from Shanghai Chemical Reagent Company of Sinopharm. Kanamycin was purchased from Ameresco Company; Amp was purchased from Ameresco Company; and 384-well and 96-well fluorescence detection blackboards were purchased from Grenier Company. DFHBI-1T and DFHO were purchased form Lucerna Company. GTP and SAM were purchased from Sigma Company.

DNA purification kits used in the examples were purchased from BBI Company (Canada), ordinary plasmid micro extraction kits were purchased from Tiangen Biotech (Beijing) Co., Ltd. BL21 (DE3, Star) bacterial strains were purchased from Invitrogen Company. 293T/17 cells and COS-7 cells were purchased from Cell Bank of Committee of Typical Culture Collection, Chinese Academy of Sciences. BY4741 yeast strain was purchased from Shanghai Weidi Biotechnology Co., Ltd.

Main instruments used in the examples: Synergy Neo2 Multiscan Spectrum (Bio-Tek Company of America), X-15R high speed freezing centrifuge (Beckman Company of America), Microfuge22R tabletop high speed freezing centrifuge (Beckman Company of America), PCR amplifier (Biometra Company of German), in vivo imaging system (Kodak Company of America), photometer (Wako Company of Japan), nucleic acid electrophoresis apparatus (Shenneng Bocai Company).

Meanings of abbreviations are as follows: "h" refers to hours, "min" refers to minutes, "s" refers to seconds, "d" refers to days, "μL" refers to micro-liters, "mL" refers to milliliters, "L" refers to liters, "bp" refers to base pairs, "mM" refers to millimoles, and "μM" refers to micromoles.

**Common experimental methods and materials in examples**

**(1) Preparation of nucleic acid aptamer molecules:**

Corresponding cDNA of RNA to be detected was amplified by using primers containing T7 promoter, and RNA was obtained after transcription with double-stranded cDNA, which was obtained after being recovered, as a template by using T7 RNA polymerase (purchased from Fermentas Company). 10 μL of 3 M NaAc and 115 μL of DEPC water were added to 20 μL of transcription system, and 150 μL of phenol chloroform-isopropanol mixture (phenol:chloroform:isopropanol=2S:24:1) was added after blending, and, after oscillation and blending, as well as centrifuge at 10,000 rpm for 5 min, the supernatant was taken. An equal volume of chloroform solution was added, and, after oscillation and blending, as well as centrifuge at 10,000 rpm for 5 min, the supernatant was taken, and the whole process was repeated once. 2.5 times the volume of anhydrous alcohol was added to the supernatant, was left in a -20°C refrigerator for 30 min and was centrifuged at 12,000 rpm at 4°C for 5 min before the supernatant was removed, and the precipitate was washed twice with 75% precooled anhydrous alcohol. After alcohol volatilization, a suitable amount of screening buffer was added for resuspending the precipitate; then the product was treated at 75°C for 5 min, and was left at room temperature for more than 10 min so as to be used in subsequent experiments.

**(2) Cell culture and transfection:**

Cells in this example were all cultured in a $CO_2$ incubator with a high glucose dulbecco's modified eagle medium (DMEM) containing fetal bovine serum (FBS) of 10%, streptomycin and penicillin, and the cells were subcultured when growth reached 80% to 90% confluence. Transfection was carried out with FuGENE®HD (purchased from Promega)

according to the instructions.

**(3) Fluorescence imaging:**

In major imaging experiments of the examples, a Leica SP8 confocal laser microscope was used for photographing, HCXPL APO 63.0x1.47 oil lens and HyD detector were used, and various lasers, such as 405 nm, 457 nm, 476 nm, 488 nm, 497 nm, 514 nm nm, 561 nm and 633 nm, were used for imaging; complexes formed by each Carrot and dye molecules were imaged by laser close to the wavelength corresponding to the maximum excitation peak. For others, GFP used a 488 nm laser; Hoechst and DAPI fluorescence used a 405 nm laser; and Rhodamine used a 561 nm laser.

**(4) Construction of recombinant plasmid based on homologous recombination method**

1. Preparation of linearized vector: a suitable cloning site was selected and the vector was linearized, and thus the linearized vector could be prepared by enzyme digestion or reverse PCR amplification.

2. PCR amplification for preparing an inserted fragment: by means of introducing a 15-25 bp (excluding enzyme cleavage site) linearized vector terminal homologous sequence at 5' end of the PCR forward and reverse primers of the inserted fragment, 5' and 3' ends of PRC products of the inserted fragment respectively carried consistent sequences corresponding to two terminals of the linearized vector.

3. Concentration measurement of linearized vectors and insertedinserted fragments: several equivoluminal dilution gradients were made for the linearized vectors and the amplified products of inserted fragments, 1 μL of original product and 1 μL of diluted product were respectively taken for agarose gel electrophoresis, and band brightness was compared with DNA molecular weight standards (DNA Marker) so that approximate concentrations thereof could be determined.

4. Recombination reaction

The optimal amount of vector used in the recombination reaction system is 0.03 pmol; and the optimal molar ratio of vectors to inserted fragments is 1:2 to 1:3, namely, the optimal quantity of inserted fragments is 0.06-0.09 pmol.

| **Components** | **Recombination Reaction** |
|---|---|
| $ddH_2O$ | up to20 μL |
| 2×Hieff Clone Enzyme Premix (purchased from Yeasen Company) | 10 μL |
| Linearized Vector | X μL |
| Inserted Fragment | Y μL |

X and Y are usage amounts of linearized vector and inserted fragments respectively calculated according to formulae. After system preparation, the components were blended, and were left for reaction at 50°C for 20 min. When the inserted fragment is >5 kb, the incubation temperature could be extended to 25 min. After the reaction, it is recommended to cool the reaction tube on ice for 5 min. The reaction product can be directly converted, or can be stored at -20°C so as to be thawed and converted when necessary.

**(5) Function detection of nucleic acid aptamers**

Carrot or Carrot mutant nucleic acid aptamer molecules were prepared according to common experimental method (I), 5 μM of nucleic acid aptamer molecules and 1 μM of fluorophore molecules were incubated in detection buffer (40 mM HEPES, pH 7.4, 125 mM KCl, 5 mM $MgCl_2$, 5% DMSO), and Synergy Neo2 multifunctional microplate reader was used for detecting and obtaining the maximum excitation peak and the maximum emission peak of the fluorescence of the nucleic acid aptamer-fluorophore molecule complex. The Synergy Neo2 multifunctional microplate reader was used again for detecting the fluorescence intensity of the aptamer-fluorophore molecule complex under its maximum excitation and emission conditions. Control sample (1 μM of fluorophore molecules without nucleic acid

aptamer) was measured under the same condition, and the ratio of fluorescence intensity was calculated. For example, the complex composed of 5 μM of Carrot-1 nucleic acid aptamers and 1 μM of IV-39 fluorophore molecules had a maximum excitation peak of 524 nm and a maximum emission peak of 580nm. By using Synergy Neo2 multifunctional microplate reader, it was detected that the fluorescence intensity of the complex was 5720 when it was excited at 524±10 nm and emitted under the emission condition of 580 nm±10 nm, while fluorescence intensity of the control sample (1 μM of IV-39 fluorophore molecules) under the same detection condition was 20, so the activation multiple of Carrot-1 nuclear acid aptamer to IV-39 fluorophore molecule was 286.

### Example 1. The secondary structure of Carrot aptamer

The secondary structure of Pepper aptamer was analyzed using the mFold online RNA structure analysis software. Pepper contains two stems, two loops and one stem-loop structures (Figure 1). For the given Carrot-1 and Carrot-2 (SEQ ID NO: 1, 2) containing the specific $N_1$, $N_{14}$-$N_{15}$-$N_{16}$ and $N_{23}$,and their predicted secondary structures were shown in Figure 2.

### Example 2. Characterization of Carrot-IV-39 Complex

In order to detect the spectral properties of the Carrot-IV-39 complex, Carrot-1 (SEQ ID NO: 2) RNA was prepared according to the commonly used experimental method (1). 1 μM IV-39 with 5 μM) Carrot-1 was incubated. The results showed that the maximum excitation and emission of the Carrot-IV-39 complex were 524 nm, and 580 nm, respectively (Figure 5a).

In order to detect the binding constant of Carrot-1 and IV-39, 20 nM Carrot-1 was incubated with different concentrations of IV-39 and their fluorescence was determined. The results showed that the binding constant of Carrot-1 and IV-39 was 58 nM (Figure 5b).

In order to detect the dependence of Pepper-III-3 complex on $Mg^{2+}$, Carrot-1-IV-39 complex was incubated in buffer containing different concentrations of $Mg^{2+}$, and the fluorescence was detected. The results showed that Carrot-1-IV-39 complex has lower dependence on $Mg^{2+}$ when compared to Pepper599 and Corn-DFHO.

### Example 3. Fluorescence activation of IV-39 fluorophore by different Carrot mutants

In order to detect the fluorescence activation of IV-39 fluorophore by different Carrot mutant, the Carrot-1 sequence was mutated according to Table 1. The Carrot RNA containing different mutations were prepared according to the commonly used experimental method (1). 1 μM IV-39 was incubated with 5 μM Carrot-1 mutant RNA and their fluorescence activation folds were determined according to the commonly used experimental method (5). The results showed that some of the Carrot-1 mutants containing single mutation retained a strong fluorescence activation of IV-39 (>20-fold) (Table 2). Several Carrot-1 mutants containing 2-5 mutations still retained strong fluorescence activation of IV-39 (>100 times) (Table 3). In summary, many Carrot-1 mutants containing single and multiple mutants still retain the ability to activate the fluorescence of IV-39 fluorophore.

**Table 2 Activation of IV-39 by Carrot mutants with single mutation**

| Mutant | Activation fold | Mutant | Activation fold | Mutant | Activation fold |
|---|---|---|---|---|---|
| Carrot-1 | 286 | G7U | 196 | C12A | 234 |
| A4U | 86 | U8C | 87 | C12U | 156 |
| A4G | 95 | A10U | 65 | A13U | 105 |
| A4C | 205 | A10G | 78 | A13G | 63 |
| USA | 215 | A10C | 62 | A13C | 311 |
| USG | 249 | A11U | 43 | G17A | 78 |
| USC | 277 | A11G | 187 | C19A | 42 |
| G7C | 117 | A11C | 35 | C19U | 69 |
| G7A | 75 | C12G | 195 | A20C | 47 |
| Note: Carrot-1 in Table 2 is an aptamer with the sequence of SEQ ID NO: 1. Other aptamers are generated by mutating the corresponding nucleotide in Figure 1 within the Carrot-1 sequence. | | | | | |

**Table 3 Activation of IV-39 by Carrot-1 mutants with multiple mutations**

| Mutant | Activation fold | Mutant | Activation fold | Mutant | Activation fold |
|---|---|---|---|---|---|
| Carrot-1 | 286 | U5C/A11G | 186 | USC/G7U/A13C | 26 |
| A4C/USA | 47 | U5C/C12G | 125 | U5C/C12G/A13C | 57 |
| A4C/USC | 126 | U5C/C12A | 174 | U5C/C12U/A13C | 28 |
| A4C/A11G | 56 | U5C/C12U | 201 | U5C/A11G/A13C | 75 |
| A4C/C12A | 75 | U5C/A13C | 234 | U5C/C12A/A13C | 85 |
| A4C/A13C | 157 | G7U/A13C | 97 | A4C/U5C/A11G/A13C | 36 |
| U5A/A11G | 28 | A11G/A13C | 188 | A4C/U5C/C12A/A13C | 28 |
| USA/C12A | 87 | C12G/A13C | 83 | A4C/U5C/G7U/A11G/A13C | 27 |
| U5A/A13C | 187 | C12A/A13C | 215 | A4C/U5C/G7U/C12A/A13C | 38 |
| U5G/A13C | 139 | C12U/A13C | 76 | A4C/U5A/A11G/C12A/A13C | 19 |
| USC/G7U | 178 | A4C/USC/A13C | 77 | A4C/U5C/A11G/C12A/A13C | 17 |

**Example 4. Base-modified Carrot's activating effect on IV-39**

In order to detect the activation of IV-39 by modified Carrot, base-modified Carrot-3 was synthesized (SEQ ID NO: 5, the underlined bold nucleotides in GGGAAGATTGTAAACACG**CC**GAAAGGC**GG**ACACTTCCC contain deoxyribonucleotide bases) and Pepper-4 (SEQ ID NO: 6, the underlined bold nucleotides in GGGAAGATTGTAAACACGCC**GAAA**GGCGGACACTTCCC contain 2'-F modification) (synthesized by Shanghai GenePharma Co.,Ltd) (Figure 6a). Detection of the fluorescence activation of IV-39 fluorophore by these modified Carrot was carried out according to the commonly used experimental method (5). The results showed that the modified Carrot-3 and Carrot-4 could still significantly activate the fluorescence of IV-39 fluorophore (Figure 6b).

**Example 5. Carrot tandem arrays**

In order to detect the fluorescence activation of IV-39 by different Carrot arrays, Carrot-2 is connected to form tandem arrays in different forms, including the following three types:

(1) "tandem array 1" (Figure 7a), different copies of Carrot are connected via "head-to-tail" to generate nCarrot (where n represents any copy number of Carrot). In this example, the cDNA encoding F30-2Carrot-5, F30-4Carrot-5, F30-6Carrot-5, F30-8Carrot-5 and F30-10Carrot-2 (The sequences of the RNA aptamer are SEQ ID NO: 7, SEQ ID NO: 8, SEQ ID NO: 9, SEQ ID NO: 10, SEQ ID NO: 11, respectively). After PCR amplification, the RNA aptamers were prepared according to the commonly used experimental methods (1). 0.2 μM RNA aptamer was incubated with 5 μM IV-39 and fluorescence was measured according to the commonly used experimental method (5). The results showed that the fluorescence of F30-nCarrot-IV-39 increased along with the increasing copy number of Carrot (n) (Figure 7d), indicating that the " tandem array 1" can be used to increase the fluorescence intensity of Carrot-IV-39 complex.

(2) "tandem array 2" (Figure 7b), Carrot serves as a structural unit and is connected to generate n×Carrot (where n represents any copy number of Carrot). In this example, the cDNAs encoding 2xCarrot-6, 4xCarrot-6, 6xCarrot-6, 8xCarrot-6 and 10xCarrot-6 (The sequences of the RNA aptamer are SEQ ID NO: 12, SEQ ID NO: 13, SEQ ID NO:14, SEQ ID NO:15 and SEQ ID NO:16 respectively) were commercial synthesized. The RNA aptamers were prepared according to the commonly used experimental methods (1). 0.2 μM RNA aptamer was incubated with 5 μM IV-39 and fluorescence was measured according to the commonly used experimental method (5). The results showed that that the fluorescence of n×Carrot-IV-39 also increased along with the increasing copy number of Carrot (n) (Figure 7e), indicating that the fluorescence intensity of Carrot-IV-39 complex can be increased through the form of "tandem array 2".

(3) "tandem array 3" (Figure 7c), which is a combination of "tandem array 1" and " tandem array 2". nCarrot generated from "tandem array 1" serves as a structural unit and is connected to generate $n_1 \times n_2$Carrot according to the form

of "tandem array 2" (where n1 and n2 represent any copy number of Pepper). In this example, the cDNAs encoding 2x2Carrot-2, 2x4Carrot-2, 4x2Carrot-2 and 4x4Carrot-2 (The sequences of the RNA aptamer are SEQ ID NO: 17, SEQ ID NO: 18, SEQ ID NO: 19 and SEQ ID NO: 20, respectively) were commercial synthesized. The RNA aptamers were prepared according to the commonly used experimental methods (1). 0.2 μM RNA aptamer was incubated with 5 μM IV-39 and fluorescence was measured according to the commonly used experimental method (5). The results showed that the fluorescence intensity of Carrot tandem array-IV-39 complex obtained by the form of "tandem array 3" was significantly higher than that of Carrot-2-IV-39 (Figure 7f), indicating that the fluorescence of Carrot-IV-39 complex can be improved by the form of "tandem array 3".

**Example 6. Characterization of IV-39 analogues**

Carrot-1 RNA aptamer was prepared according to the commonly used experimental methods (1), and was used to detect the properties of IV-39 analogues upon Carrot binding, including the fluorescence spectrum, extinction coefficient, quantum yield and fluorescence activation fold. The results were shown in Table 4. From the data shown in the table, Carrot-1 still could activate the fluorescence of IV-39 analogues.

**Table 4 : The properties of Carrot-1 RNA aptamer with different fluorophores**

| | $Ex_{max}$ (nm) | $Em_{max}$ (nm) | Extinction coefficient ($M^{-1}$ $cm^{-1}$ | Quantum yield | Activation fold |
|---|---|---|---|---|---|
| Carrot-1-IV-1 | 510 | 618 | 21000 | 0.17 | 57 |
| Carrot-1-IV-2 | 492 | 595 | 25000 | 0.36 | 120 |
| Carrot-1-IV-3 | 490 | 565 | 26000 | 0.27 | 128 |
| Carrot-1-IV-4 | 490 | 574 | 23000 | 0.37 | 191 |
| Carrot-1-IV-5 | 492 | 570 | 23000 | 0.33 | 185 |
| Carrot-1-IV-6 | 490 | 578 | 24000 | 0.37 | 179 |
| Carrot-1-IV-7 | 514 | 598 | 24000 | 0.27 | 201 |
| Carrot-1-IV-8 | 534 | 638 | 22500 | 0.21 | 68 |
| Carrot-1-IV-9 | 527 | 615 | 21000 | 0.17 | 47 |
| Carrot-1-IV-10 | 504 | 612 | 22000 | 0.07 | 23 |
| Carrot-1-IV-11 | 507 | 614 | 21000 | 0.05 | 16 |
| Carrot-1-IV-12 | 482 | 577 | 19000 | 0.03 | 6 |
| Carrot-1-IV-13 | 478 | 579 | 20000 | 0.02 | 5 |
| Carrot-1-IV-14 | 482 | 585 | 21000 | 0.02 | 6 |
| Carrot-1-IV-15 | 480 | 578 | 21000 | 0.03 | 7 |
| Carrot-1-IV-16 | 485 | 587 | 22000 | 0.08 | 34 |
| Carrot-1-IV-17 | 524 | 582 | 32000 | 0.47 | 282 |
| Carrot-1-IV-18 | 536 | 600 | 25000 | 0.43 | 211 |
| Carrot-1-IV-19 | 522 | 590 | 30000 | 0.43 | 331 |
| Carrot-1-IV-20 | 512 | 577 | 26000 | 0.36 | 178 |
| Carrot-1-IV-21 | 534 | 624 | 26000 | 0.28 | 128 |
| Carrot-1-IV-22 | 522 | 580 | 32000 | 0.46 | 301 |
| Carrot-1-IV-23 | 547 | 648 | 19000 | 0.12 | 57 |
| Carrot-1-IV-24 | 498 | 615 | 21000 | 0.09 | 45 |
| Carrot-1-IV-25 | 475 | 574 | 18000 | 0.02 | 4 |
| Carrot-1-IV-26 | 479 | 581 | 20000 | 0.07 | 39 |
| Carrot-1-IV-27 | 472 | 576 | 19000 | 0.09 | 57 |
| Carrot-1-IV-28 | 476 | 579 | 19000 | 0.04 | 19 |
| Carrot-1-IV-29 | 531 | 619 | 27000 | 0.26 | 87 |
| Carrot-1-IV-30 | 485 | 588 | 19000 | 0.05 | 15 |
| Carrot-1-IV-31 | 508 | 621 | 24000 | 0.15 | 75 |
| Carrot-1-IV-32 | 489 | 591 | 22000 | 0.11 | 64 |
| Carrot-1-IV-33 | 519 | 592 | 28000 | 0.38 | 287 |
| Carrot-1-IV-34 | 515 | 581 | 29000 | 0.38 | 287 |
| Carrot-1-IV-35 | 527 | 619 | 21000 | 0.14 | 97 |

(continued)

| | $Ex_{max}$ (nm) | $Em_{max}$ (nm) | Extinction coefficient ($M^{-1}$ $cm^{-1}$ | Quantum yield | Activation fold |
|---|---|---|---|---|---|
| Carrot-1-IV-36 | 511 | 578 | 22000 | 0.28 | 262 |
| Carrot-1-IV-37 | 488 | 571 | 25000 | 0.35 | 162 |
| Carrot-1-IV-38 | 519 | 581 | 26000 | 0.31 | 257 |
| Carrot-1-IV-39 | 524 | 580 | 33000 | 0.48 | 286 |
| Carrot-1-IV-40 | 506 | 578 | 21000 | 0.25 | 236 |

**Example 7. Labeling of bacterial RNA using Carrot-IV-4 complex**

In order to test the effect of Carrot-IV-4 in bacteria, a bacterial expression plasmid expressing F30-Carrot-2 was constructed. cDNA encoding F30-Carrot-2 was synthesized and amplified using primer. Primers were used to amplify pET28a to remove the promoter and multiple cloning site regions. The obtained F30-Carrot-2 fragment and the linearized pET28a were ligated according to the commonly used experimental method (4). The obtained recombinant plasmid was named pET28a-T7-F30-Carrot-2.
The primers used to amplify the F30-Carrot-2 fragment are:

Forward primer (P1):

5'-TAATACGACTCACTATAGGGTTGCCATGTGTATGTGGGA-3'

Reverse primer (P2): 5'-CAAGGGGTTATGCTATTGCCATGAATGATCC-3'

The primers used to amplify and linearize the pET28a vector are:

Forward primer (P3): 5'-TAGCATAACCCCTTGGGGCCTC -3'

Reverse primer (P4): 5'-TAGTGAGTCGTATTAATTTCGCGGGATCGAGATCTCG -3'

The recombinant plasmid pET28a-T7-F30-Carrot-2 was transformed into BL21(DE3, Star) E. coli strain. A single clone was picked and cultured at 37°C to an $OD_{600}$ around 0.2 before addition of 1 mM IPTG to induce the expression of F30-F30-Carrot-2. 4 hours after induction, the bacteria were harvested and resuspended in PBS solution containing 2 μM !!!-3. BL21 (DE3, Star) E. coli transformed with pET28a empty vector was used as the control. The results showed that bacteria exhibited bright yellow-green fluorescence only when F30-F30-Carrot-2 was expressed and in the presence of III-3 (Figure 8), indicating that Carrot-IV-4 complex can be used for fluorescent labeling of RNA in bacteria.

**Example 8. Labeling of yeast RNA using Carrot-IV-4 complex**

In order to test the effect of Carrot-IV-4 in yeast, a yeast expression plasmid expressing F30-Carrot-2 was constructed. The F30-Carrot-2 DNA fragment in Example 2 was amplified using primers, and the amplified F30-Carrot-2 fragment was inserted into the pYES2.1TOPO TA vector according to the commonly used experimental method (4). The obtained recombinant plasmid was named pYES2.1-F30-Carrot-2.
The primers used to amplify the F30-Carrot-2 fragment are:

Forward primer (P5): 5'- GGAATATTAAGCTCGCCCTTTTGCCATGTGTATGTGGG -3'

Reverse primer (P6): 5'- TGACCTCGAAGCTCGCCCTTGTTGCCATGAATGATCC -3'

The recombinant plasmid pYES2.1-F30-Carrot-2was transformed into BY4741 strain, and a single clone was picked and cultured at 30°C to an $OD_{600}$=0.1 before addition of 1 mM galactose to induce the expression of F30-Carrot-2. 10 hours after induction, the yeast cells were harvested and resuspended in PBS containing 1 μM IV-4. The untreated BY4741 strain was used as the control. The results showed that yeast cells exhibited bright yellow-green fluorescence only when F30-Carrot-2 was expressed and in the presence of IV-4 (Figure 9), indicating that Carrot-IV-4 complex can be used for RNA labeling in yeast cells.

**Example 9. RNA labeling in mammalian cells using Carrot and IV-39 and** its **analogs.**

In order to use Carrot and IV-39 for RNA labeling in mammalian cells, the reported Pepper and Corn aptamer (binding to HBC599 and DFHO fluorophores, respectively) were used as the controls (Chen et al. Nature Biotechnology 2019. 37: 1287-1293; Song et al. Nature Chemical Biology 2017. 13: 1187-1194). The mammalian cell expression plasmids expressing the RNA aptamers were constructed. cDNAs encoding tRNA-Carrot-2 (SEQ ID No : 4) , tRNA-Pepper (SEQ ID No : 22) and tRNA-Corn (SEQ ID No : 21) were synthesized and amplified using the primers P7 and P8, and the primers P9 and P10 were used to amplify pEGFP-N1 to remove the promoter and multiple cloning site regions. The obtained fragments were ligated into the pEGFP-N1 vector according to the commonly used experimental method (4). The obtained plasmids were linearlized by amplification using primers P11 and P12, pLKO.1 puro vector was used as the template for amplification of U6 promoter (SEQ ID No : 23), and the U6 promoter was inserted into the linearized vector to obtain pU6-tRNA-Carrot-2, pU6-tRNA-Pepper and pU6-tRNA-Corn, which encode tRNA-Carrot-2, tRNA-Pepper and tRNA-Corn. pU6-tRNA-Carrot-2, pU6-tRNA-Pepper and pU6-tRNA-Corn plasmids were transfected into 293T/17 cells. The cells were incubated with 0.5 μM IV-39, 0.5 μM HBC599 and 10 μM DFHO to label tRNA-Carrot-2, tRNA-Pepper and tRNA-Corn 24 h after transfection, respectively. The cells expressing no RNA aptamer were used as the controls. Fluorescence imaging was performed according to the commonly used experimental method (3). The results showed that tRNA-Carrot-IV-39 complex showed bright orange fluorescence with or without the addition of 20 mM $Mg^{2+}$ into the assay buffer (Figure 10a). The fluorescence intensity was markedly higher than tRNA-Pepper-HBC599 and tRNA-Corn-DFHO complexes (Figure 10b), indicating that Carrot-IV-39 can be used to work in mammalian cells.

The primers used to amplify tRNA-Carrot-2, tRNA-Pepper and tRNA-Corn are:

Forward primer (P7): 5'-GCCGCCCCCTTCACCTCTAGAGCCCGGATAGCTCAGTCGG -3'

Reverse primer (P8): 5'- GAGAATTCAAAAAAATGGCGCCCGAACAGGGAC -3'

The primers used to amplify and linearize the pEGFP-N1 vector are:

Forward primer (P9):

5'- TTTTTTTGAATTCTCGACCTCGAGACAAATGGCAGTATTCA -3'

Reverse primer (P10): 5'- GGTGAAGGGGGCGGCCGCTCGAGG -3'

The primers used to amplify and linearize the vector to introduce U6 promoter are:

Forward primer (P11):

5'- TTTTTTTGAATTCTCGACCTCGAGACAAATGGCAGTATTCA -3'

Reverse primer (P12): 5'- GGTGAAGGGGGCGGCCGCTCGAGG -3'

The primers used to amplify U6 promoter are:

Forward primer (P13): 5'- GCCGCCCCCTTCACCGAGGGCCTATTTCCCATG -3'

Reverse primer (P14): 5'-TATCCGGGCTCTAGAGTTTCGTCCTTTCCACAAGATATAT -3'

In order to use Carrot and IV-39 analogues for RNA labeling in mammalian cells, a mammalian expression plasmid expressing F30-4Carrot-2 was constructed. The primers P15 and P16 in this example were used to amplify the F30-4Carrot-2 fragment in Example 5, and the fragments was inserted into the XbaI and EcoRI sites of pU6-tRNA-Carrot-2 vector using the commonly used experimental method (4). The obtained expression vector was named pU6-F30-4Carrot-2.

The primers used to amplify F30-4Carrot-2 fragment are:

Forward primer (P15): 5'- GGAAAGGACGAAACTCTAGATTGCCATGTGTATGTGGGA -3'

Reverse primer (P16):

5'- TGTCTCGAGGTCGAGAATTCAAAAAAATTGCCATGAATGATCCCGAAG -3'

The pU6-F30-4Carrot-2 plasmid was transfected into 293T/17 cells. Different IV-39 analogues were added into the culture for labeling 24 hours after transfection. Fluorescence imaging was performed according to the commonly used experimental method (3). The results showed that different IV-39 analogs could be used to specifically label cells expressing F30-4Carrot-2, but not the control cells without expression of F30-4Carrot-2 (Figure 10c), indicating that Carrot and IV-39 and its analogs can be used to label RNA in mammalian cells.

**Example 10. Construction of Carrot-based sensors**

In order to construct Carrot-based sensors for detecting analytes, the nucleotides in the $N_{14}$-$N_{15}$-$N_{16}$ region in the Carrot-1 (SEQ ID No: 1) structure were replaced with the aptamers that can specifically recognize and bind adenosine and S-Adenosyl methionine (SAM), respectively. The aptamers Carrot-1 were fused by linkage with different lengths and different nucleotides (Figure 11a). The RNA sensors were prepared according to the commonly used experimental method (1). The RNA sensors were incubated with IV-39 fluorophore, and the fluorescence was measured in the presence or absence of adenosine or SAM using a multifunctional microplate reader. The results showed that the fluorescence of these probes in the presence of the target is significantly higher than that in the absence of the target (Figure 11b), indicating that they can be used as probes for the detection of adenosine and SAM, respectively. The RNA sequences for the corresponding probes are SEQ ID No: 24 and SEQ ID No: 25.

**Example 11. Tracking of RNA localization in cells by Carrot**

In order to use Carrot to track RNA localization in cells, chimeric RNA expression plasmids in which Carrot was fused to different RNAs were constructed. cDNA encoding 4Carrot-2 was amplified using primers and inserted into HindIII and XhoI sites of pCDNA3.1 hygro(+) to obtain pCDNA3.1 hygro(+)-4Carrot-2. cDNAs encoding GAPDH and ACTB (the encoding sequences for GAPDH and ACTB were Genebank: BC009081, BC016045) were synthesized and amplified using primers. The obtained fragment was inserted into the HindIII and XhoI sites of pCDNA3.1 hygro(+)-4Carrot-2 vector to generate pCDNA3.1 hygro(+)-GAPDH-4Carrot-2 and pCDNA3.1 hygro(+)-ACTB-4Carrot-2 plasmids that express GAPDH-4Carrot-2 and ACTB-4Carrot-2 chimeric RNAs, respectively. The sequences of the chimeric RNAs are SEQ ID Nos: 26 and 27, respectively.

The primers used to amplify 4Carrot-2 are:
Forward primer (P17): 5'- TAGCGTTTAAACTTAAGCTTGGAAGATTGTAAACACGCC -3'

Reverse primer (P18):
5'- ACGGGCCCTCTAGACTCGAGGGAAGTGTCCGCCGGAAGT -3'

The primers used to amplify GAPDH are:
Forward primer (P19): 5'- GGAGACCCAAGCTGGCTAGCATGGGGAAGGTGAA GGTCGG -3'

Reverse primer (P20):
5'-CACGGACACATGGCAAGCTTAACCATGCTCTAGCGAGTGTTACTCCTTGGAGGCCATGT -3'

The primers used to amplify ACTB are:
Forward primer (P21): 5'- GGAGACCCAAGCTGGCTAGCATGGTGACGCTT GCTGAACT -3'

Reverse primer (P22):
5'-CACGGACACATGGCAAGCTTAACCATGCTCTAGCGAGTGCTAGAAGCATTTGCGGTGGA -3'

After construction of above plasmids, the inserted sequences were validated by sequencing to ensure correct insertion. The plasmids were extracted using a transfection-grade plasmid extraction kit for subsequent transfection experiments.

The pCDNA3.1 hygro(+)-GAPDH-4Carrot-2 and pCDNA3.1 hygro(+)-ACTB-4Carrot-2 plasmids constructed in this example were co-transfected into COS-7 cells, respectively. 24 hours after transfection, the cells were incubated with 1 $\mu$M IV-4 and imaged according to the fluorescence imaging method described in the commonly used experimental method (3). The imaging results showed that the fluorescence of GAPDH-4Carrot-2 and ACTB-4Carrot-2 was mainly localized in the cytoplasm, which was consistent with previous studies and the results obtained by fluorescent-labeled in situ hybridization (FISH) (Figure 12). These results indicate that Carrotcan be used to track RNA location.

**Example 12. Detection of genomic DNA by Carrot**

In order to use Carrot to detect genomic DNA, a recombinant plasmid expressing chimeric RNA of Carrot-2 and sgRNA was constructed. cDNAs encoding sgRNA-Carrot-2-1, sgRNA-Carrot-2-2 and sgRNA-Carrot-2-3 containing centromere targeting sequence were synthesized. The encoded RNA sequences are SEQ ID No: 28, 29 and 30, respectively. Primers P23 and P24 were used to amplify above cDNAs, primers P25 and P26 were used to amplify the psgRNA plasmid (Shao et al. Nucleic acids research 2016. 44: e86). The obtained cDNAs were inserted into the linearized psgRNA vector according to the commonly used experimental method (4) to generate psgRNA-Carrot-2-1, psgRNA-Carrot-2-2 and psgRNA-Carrot-2-3, respectively (Figure 13a). Primers P27 and P28 were used to amplify the dCas9-GFP gene fragment using pSLQ1645(dCas9-GFP) (Shao et al. Nucleic acids research 2016. 44: e86) as the template. The obtained gene fragment was inserted into the HindIII and XhoI sites of pCDNA3.1 hygro(+) vector to generate pCDNA3.1 hygro(+)-dCas9-GFP according to the commonly used experimental method (4).

The primers used to amplify the cDNA encoding Carrot and sgRNA chimeric RNA are:

Forward primer (P23):
5'- AAAGGACGAAACACCGAATCTGCAAGT GGATATTGTTTGAG -3'

Reverse primer (P24): 5'- TGATCTAGAAAAAAAGCACCGACTCGGTGCCAC -3'

The primers used to amplify the psgRNA plasmid to linearize it are:
Forward primer (P25): 5'- TTTTTTTCTAGATCATAATCAGCCATACC -3'

Reverse primer (P26): 5'-GGTGTTTCGTCCTTTCCACAAG-3'

The primers used to amplify SpdCas9-GFP are:

Forward primer (P27):
5' -TAG CGTTTAAACTTAAG CTTGTG CAG GCTG G CG CC ACCATGG CCCC-3'

Reverse primer (P28):
5'-ACGGGCCCTCTAGACTCGAGTTACTTGTACAGCTCGTCCATGC-3'

pCDNA3.1 hygro(+)-dCas9-GFP and psgRNA-Carrot-2-1, psgRNA-Carrot-2-2 and psgRNA-Carrot-2-3 were co-transfected into COS-7 cells, respectively. 24 hours after transfection, the cells were labeled with 1 μM IV-4 and Hoechst, and the fluorescence of Carrot-2-IV-4, GFP and Hoechst were imaged using a fluorescence microscope. The imaging results showed that the fluorescence of Carrot-2-IV-4 was mainly localized in the nucleus to exhibit aggregates in dots (centromeres), which was almost completely overlayed with the fluorescence of dCas9-GFP (Figure 13b), indicating that Carrot can be used to image genetic DNA.

**Example 13. Detection of RNA-protein interactions by Carrot**

In order to use Carrot to detect RNA-protein interactions, the interaction between phage capsid protein MCP and its binding sequence MS2 RNA was used as an example. The cDNA encoding Carrot-1-MS2 (SEQ ID No: 31) was synthesized and amplified using primers P29 and P30, the obtained fragment was inserted into the XbaI and EcoRI sites of pU6-tRNA-Carrot-2 to generate pU6-Carrot-1-MS2 according to the commonly used experimental method (4). The cDNAs encoding tdMCP protein (the dimeric form of MCP, SEQ ID No: 32) and NanoLuc protein (SEQ ID No: 33) were synthesized. Primers P29 and P30, P33 and P34 were used to amplify tdMCP gene fragment, respectively. Primers P35 and P36 were used to amplify NanoLuc gene fragment. The obtained fragments were inserted into the HindIII and XhoI sites of pCDNA3.1 hygro(+) to generate pCDNA3.1 hygro(+)-tdMCP-NanoLuc-tdMCP according to the commonly used experimental method (4), the sequence of the fusion protein encoded by which is SEQ ID No:34.

The primers used to amplify Carrot-1-MS2 fragment are:

Forward primer (P29):
5'- GGAAAGGACGAAACTCTAGAGGGAAGATTGTAAACAC -3'

Reverse primer (P30):
5'- TGTCTCGAGGTCGAGAATTCAAAAAAAGGGAAGTGTCCGATGGG -3'

The primers used to amplify tdMCP fragment are:

Forward primer (P31):
5'- TAGCGTTTAAACTTAAGCTTATGCTAGCCGTTAAAATGGC -3'

Reverse primer (P32):
5'- ACTCCCTCCGCCACCTCCAGAATCCGCGTAGATGC -3'

The other primers used to amplify tdMCP fragment are:

Forward primer (P33):
5'- ACTCCCTCCCGCCAGAATGCGTTCGCAC -3'

Reverse primer (P34):
5'- ACGGGCCCTCTAGACTCGAGTTATCCAGAATCCGCGTAG -3'

The primers used to amplify NanoLuc fragment are:

Forward primer (P35):
5'- GGTGGCGGAGGGAGTATGGTCTTCACACTCGAAGA -3'

Reverse primer (P36):
5'- ACTCCCTCCCGCCAGAATGCGTTCGCAC -3'

pCDNA3.1 hygro(+)-tdMCP-NanoLuc-tdMCP and pU6-Carrot-1-MS2 recombinant plasmids were co-transfected into 293T/17 cells. After 24 h of transfection, 1 μM IV-4 fluorophore was added to label Carrot. Imaging was taken immediately after addition of 20 μM Furimazine to the medium. The results showed that tdMCP motif in the tdMCP-NanoLuc-tdMCP fusion proteinc could recognize the MS2 RNA in the Carrot-1-MS2 chimeric RNA, the distance between the Carrot-IV-4 complex and the NanoLuc protein is proximity, the light emitted by the furimazine catalysed by NanoLuc could be transferred to the Carrot-IV-4 complex, which made the luminescence energy transfer (BRET) more efficient, and enabled the Carrot-IV-4 complex to produce obvious orange-red fluorescence (Figure 14). As a control, Carrot-1 RNA alone without MS2 could not interact with the tdMCP-NanoLuc-tdMCP fusion protein, the Carrot-IV-4 complex was farther away from the NanoLuc protein, and the light emitted by the furimazine catalysed by NanoLuc could not be transferred to the Carrot-IV-4 complex, making the luminescence energy transfer (BRET) less efficient, and the Carrot-IV-4 complex could not emit obvious orange-red fluorescence (Figure 14).

### Example 14. RNA extraction and purification by Carrot

In order to use Carrot for RNA extraction and purification, the pCDNA3.1 hygro(+)-GAPDH-4Carrot-2 plasmid in Example 11 were transfected into COS-7 cells, respectively. 24 hours after transfection, the cells were collected and the total RNA of the cells was extracted using the Easyp Super Total RNA Extraction Kit (Promega). The extracted total RNA was dissolved in buffer containing 40 mM HEPES, pH 7.4, 125 mM KCl, 5 mM $MgCl_2$. The RNA was incubated at 70°C for 10 min and placed at room temperature for more than 30 min. 500 uL activated Thiol Sepharose 4B (GE Healthcare) was washed twice with 500 μL PBS, and then was incubated with PBS containing 10 mM TCEP (Sigma) for 1 h at room temperature. After washing twice with 500 μL PBS, maleamide conjugated IV-39 fluorophore (Mal-IV-39) was added to react for 30 min at room temperature, and was washed three times with 500 μL PBS. The treated total RNA was incubated with the treated beads at room temperature. After 30 minutes, the mixture was centrifuged at 4000 rpm for 2 minutes, and the supernatant was discarded. The agarose beads were washed with buffer containing 40 mM HEPES, pH 7.4, 125 mM KCl, and 5 mM $MgCl_2$ for 6 times, and the supernatant was removed by centrifugation each time. The beads were resuspended with DEPC water, treated at 70°C for 10 min, and centrifuged at 4000 rpm for 2 min. Then the supernatant was collected. 1/10 volume of NaAc, 2.5 times volume of absolute ethanol was added into the collected supernatant and placed in a refrigerator at -80°C for 20 min. The mixture was centrifuged at 14000 rpm at 4°C for 10 min. The precipitate was collected and the supernatant was discarded. The pre-cooled 70% ethanol solution was used to wash the precipitate. The mixture was then centrifuged at 14000 rpm for 10 min at 4°C. The precipitate was collected and the supernatant was discarded. Such procedure was repeated once again. The precipitate was placed at room temperature for 5 minutes, and then a small volume of DEPC water was used to resuspend the precipitate after the alcohol was evaporated.

The fluorescence of the complex of IV-39 with supernatant and the eluate after high temperature elution was

measured. The supernatant of blank cells was used as the control. The detection results showed that the fluorescence of the eluate after incubation with IV-39 was significantly higher than that of the fragmented supernatant before loading (Figure 15), indicating that GAPDH-4Carrot-2 RNA was well enriched, indicating that Carrot can be used as a tag for RNA isolation and purification.

**Example 15. Synthesis of IV - 39 and its analogs**

Compound IV-1 :

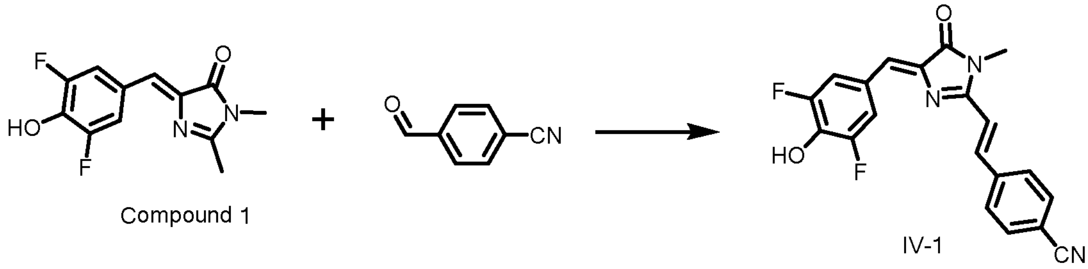

Compound 1

IV-1

To a stirred solution of Compound 1 (0.504 g, 2 mmol), anhydrous zinc chloride (0.545 g, 4 mmol) in 100 mLTHF, 4-Cyanobenzaldehyde (0.626 g, 5 mmol) was added. The complete solution were stirred at 80°C under Ar atmosphere. The progress of reaction was monitored on silica gel TLC. After completion of reaction, the solution allowing the reaction to cool to room temperature. The solvent is evaporated to dryness, to give a crude product, then purified by silica gel column chromatography to afford a target compound (0.292 g, 40%).$^1$H NMR (400 MHz, DMSO-$d_6$) δ 11.07 (s, 1H), 8.10 (d, J = 8.5 Hz, 2H), 8.06 (dd, J = 7.8, 2.1 Hz, 2H), 8.03 (s, 1H), 7.94 (d, J = 8.3 Hz, 2H), 7.44 (d, J = 15.9 Hz, 1H), 7.03 (s, 1H), 3.29 (s, 3H).MS(ESI): m/z Calcd. For $C_{20}H_{13}F_2N_3O_2$ 365.0976; found 364.0902, $[M-H]^-$.

Compound IV-2 :

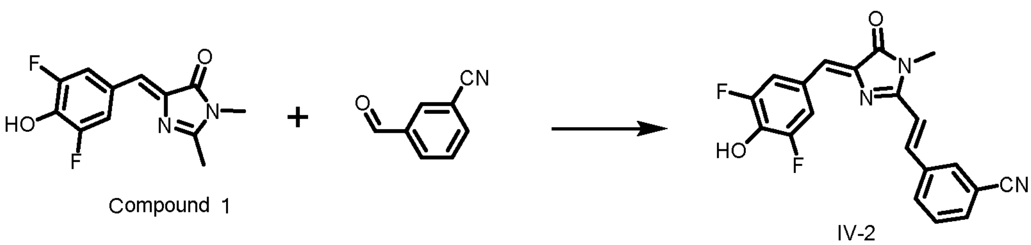

Compound 1

IV-2

This compound was obtained by following the general procedure for CompoundIV-1, ( 0.475 g, 65%)。$^1$H NMR (400 MHz, DMSO-$d_6$) δ 11.04 (s, 1H), 8.49 (d, *J* = 1.9 Hz, 1H), 8.21 (dt, *J* = 8.0, 1.4 Hz, 1H), 8.10-8.02 (m, 2H), 8.00 (s, 1H), 7.88 (dt, *J* = 7.7, 1.4 Hz, 1H), 7.67 (t, *J* = 7.8 Hz, 1H), 7.43 (d, *J* = 16.0 Hz, 1H), 7.01 (s, 1H), 3.29 (s, 3H).MS(ESI): m/z Calcd. For $C_{20}H_{13}F_2N_3O_2$ 365.0976; found 364.0903, $[M-H]^-$.

Compound IV-3 :

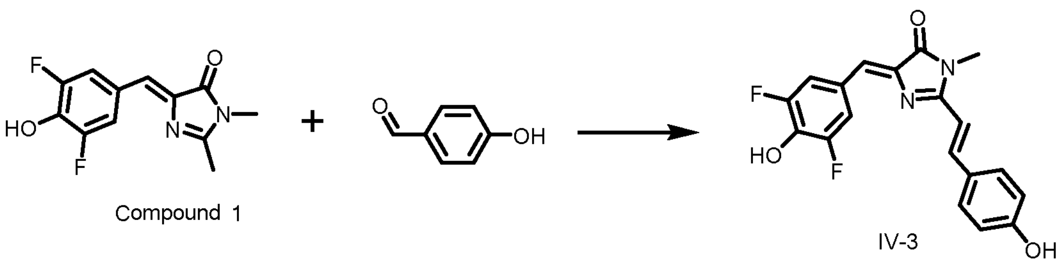

Compound 1

IV-3

This compound was obtained by following the general procedure for CompoundIV-1, ( 0.221 g, 31%) 。$^1$H NMR (400 MHz, DMSO-$d_6$) δ 10.94 (s, 1H), 10.11 (s, 1H), 8.07 - 8.01 (m, 2H), 7.95 (d, J = 15.7 Hz, 1H), 7.74 (d, J = 8.7 Hz, 2H), 7.01 (d, J = 15.7 Hz, 1H), 6.90 (s, 1H), 6.87 - 6.83 (m, 2H), 3.26 (s, 3H).MS(ESI): m/z Calcd. For $C_{19}H_{14}F_2N_2O_3$ 356.0972; found 355.0901, [M-H]$^-$.

Compound Ⅳ-4 :

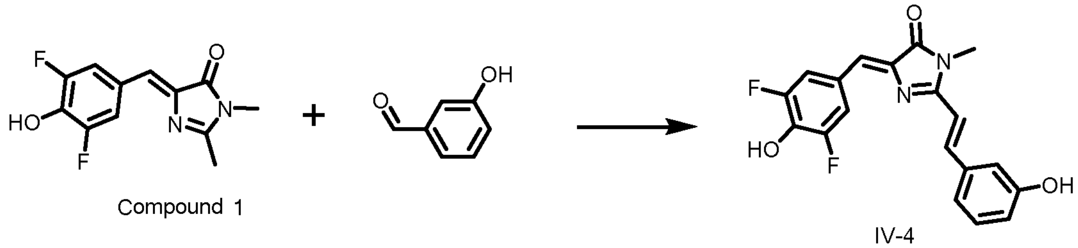

Compound 1

IV-4

This compound was obtained by following the general procedure for CompoundIV-1, ( 0.207 g, 29%) 。$^1$H NMR (400 MHz, DMSO-$d_6$) δ 9.70 (s, 1H), 8.05 (d, *J* = 8.9 Hz, 2H), 7.98 - 7.89 (m, 1H), 7.30 (t, *J* = 6.2 Hz, 1H), 7.26 (d, *J* = 8.9 Hz, 1H), 7.15 (d, *J* = 15.8 Hz, 1H), 6.97 (s, 1H), 6.87 (d, *J* = 7.5 Hz, 1H), 3.27 (s, 3H).MS(ESI): m/z Calcd. For $C_{19}H_{14}F_2N_2O_3$ 356.0972; found 355.0900, [M-H]$^-$.

Compound Ⅳ-5 :

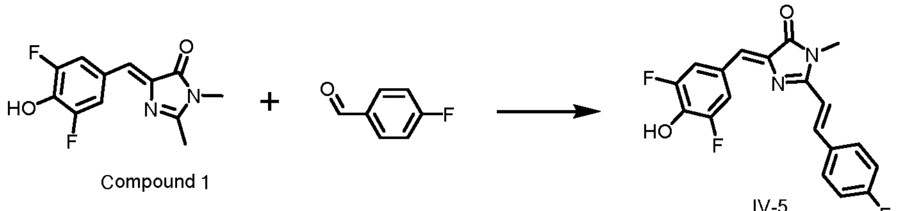

Compound 1

IV-5

This compound was obtained by following the general procedure for CompoundIV-1, ( 0.186 g, 26%) 。$^1$H NMR (400 MHz, DMSO-$d_6$) δ 8.16 (d, *J* = 8.5 Hz, 2H), 7.99-7.86 (m, 3H), 7.31 (t, *J* = 8.9 Hz, 2H), 7.18 (d, *J* = 15.9 Hz, 1H), 6.95 (s, 1H), 6.83-6.72 (m, 2H), 3.59 (t, *J* = 5.9 Hz, 2H), 3.51 (t, *J* = 5.9 Hz, 2H), 3.27 (s, 3H), 3.05 (s, 3H).MS(ESI): m/z Calcd. For $C_{19}H_{13}F_3N_2O_2$ 358.0929; found 357.0856, [M-H]$^-$.

Compound Ⅳ-6 :

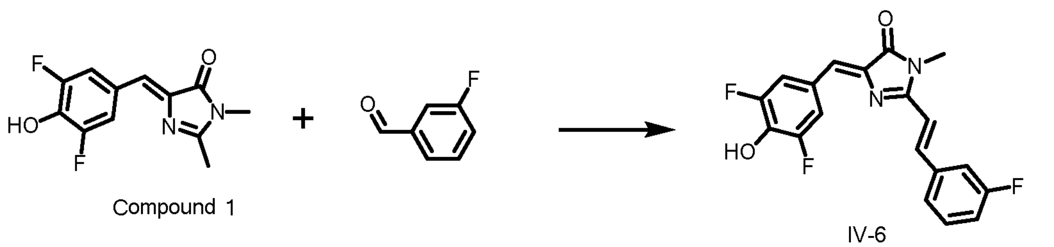

Compound 1

IV-6

This compound was obtained by following the general procedure for CompoundIV-1, ( 0.222 g, 31%) .$^1$H NMR (400 MHz, DMSO-$d_6$) δ 11.02 (s, 1H), 8.09-8.03 (m, 2H), 8.01 (d, *J* = 15.8 Hz, 1H), 7.85 (dt, *J* = 10.6, 2.1 Hz, 1H), 7.72 (d, *J* = 7.8 Hz, 1H), 7.51 (td, *J* = 8.0, 6.1 Hz, 1H), 7.34 (d, *J* = 15.9 Hz, 1H), 7.28 (td, *J* = 8.7, 2.7 Hz, 1H), 7.00 (s, 1H), 3.28 (s, 3H).MS(ESI): m/z Calcd. For $C_{19}H_{13}F_3N_2O_2$ 358.0929; found 357.0857, [M-H]$^-$.

Compound Ⅳ-7：

Compound 2：

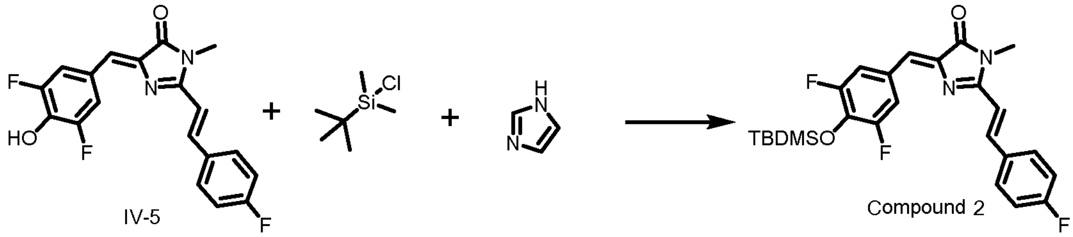

IV-5 Compound 2

To a stirred solution of compound IV-5 (0.716 g, 2.0 mmol) , TBSCl (0.450 g, 3.0 mmol), in 50 mL dry DMF, and imidazole (0.204 g, 3.0 mmol) was added. The complete solution was stirred for 3 hours at room temperature under Ar atmosphere. The mixture was poured into 150 mL water and extracted with DCM. The organic layer was dried over anhydrous $MgSO_4$, filtered, and concentrated under reduced pressure to give the crude product, then purified by silica gel column chromatography to afford the compound 2 (0.927 g, 98%).$^1$H NMR (400 MHz, DMSO-$d_6$) $\delta$ 8.16 (d, $J$ = 8.5 Hz, 2H), 7.99-7.86 (m, 3H), 7.31 (t, $J$ = 8.9 Hz, 2H), 7.18 (d, $J$ = 15.9 Hz, 1H), 6.95 (s, 1H), 6.83-6.72 (m, 2H), 3.59 (t, $J$ = 5.9 Hz, 2H), 3.51 (t, $J$ = 5.9 Hz, 2H), 3.27 (s, 3H), 3.05 (s, 3H), 1.50(s, 9 H), 0.2(s, 6 H).MS(ESI): m/z Calcd. For $C_{25}H_{28}F_3N_2O_2Si$ 473.2; found 473.2, $[M+H]^+$.

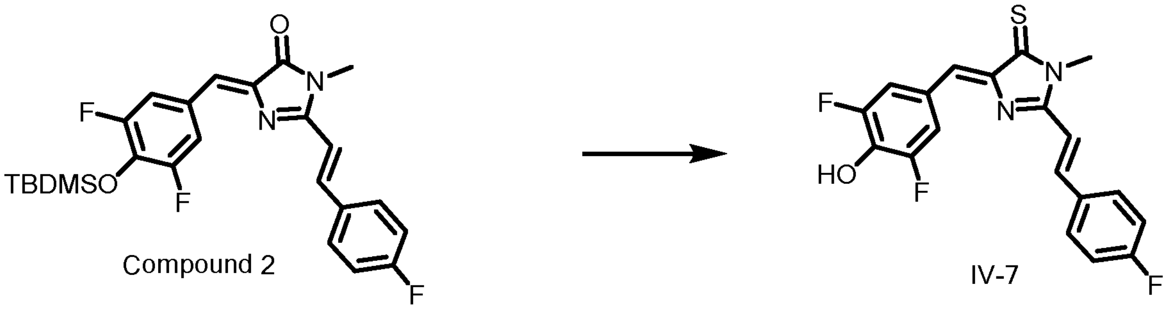

Compound 2 IV-7

Compound 2 (0.473 g, 1 mmol), Lawesson's reagent (0.808 g, 2 mmol) was in 250 mL three neck bottles and dissolved in 100 ml toluene. Two drops of aniline was added. The reaction mixture was reflexed until the TLC showed the complete the reaction. The solvent was removed under reduce pressure to give the crude product which was redissolved in 50 mL DCM, then TBAF (0.313 g, 1.2 mmol) was added. The mixture was stirred at rt under Ar atmosphereatmosphere. After complete the reaction, the solvent was removed under reduce pressure to give the crude product, then purified by silica gel column chromatography to afford a target compound IV-7 (0.209 g, 56%). $^1$H NMR (400 MHz, DMSO-$d_6$) $\delta$ 8.17 (d, $J$ = 8.5 Hz, 2H), 7.98-7.86 (m, 3H), 7.31 (t, $J$ = 8.9 Hz, 2H), 7.18 (d, $J$ = 15.9 Hz, 1H), 6.95 (s, 1H), 6.83-6.72 (m, 2H), 3.59 (t, $J$ = 5.9 Hz, 2H), 3.51 (t, $J$ = 5.9 Hz, 2H), 3.27 (s, 3H), 3.05 (s, 3H).MS(ESI): m/z Calcd. For $C_{19}H_{13}F_3N_2NaOS$ 397.0598; found 397.0597, $[M+Na]^+$.

Compound Ⅳ-8：

Compound 3：

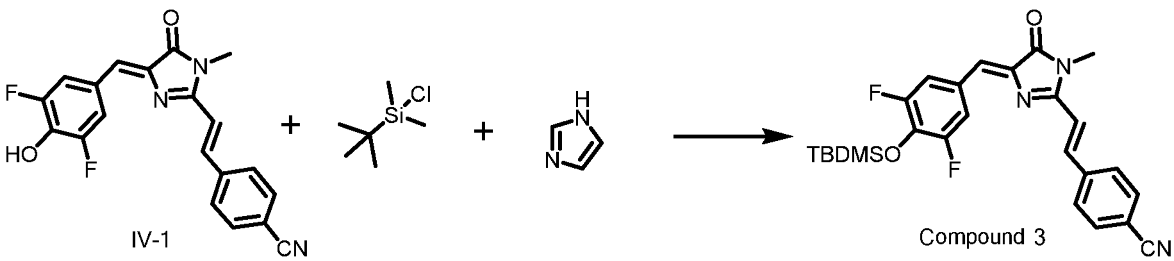

IV-1 Compound 3

This compound was obtained by following the general procedure for CompoundIV-1Compound 2, (0.932 g, 99%)。$^1$H NMR (400 MHz, DMSO-$d_6$) δ 8.10 (d, J = 8.5 Hz, 2H), 8.06 (dd, J = 7.8, 2.1 Hz, 2H), 8.03 (s, 1H), 7.94 (d, J = 8.3 Hz, 2H), 7.44 (d, J = 15.9 Hz, 1H), 7.03 (s, 1H) , 3.29 (s, 3H), 1.51 (s, 9H), 0.29 (s, 9H).MS(ESI): m/z Calcd. For $C_{26}H_{28}F_2N_3O_2Si$ 480.2; found 480.2, $[M+H]^+$.

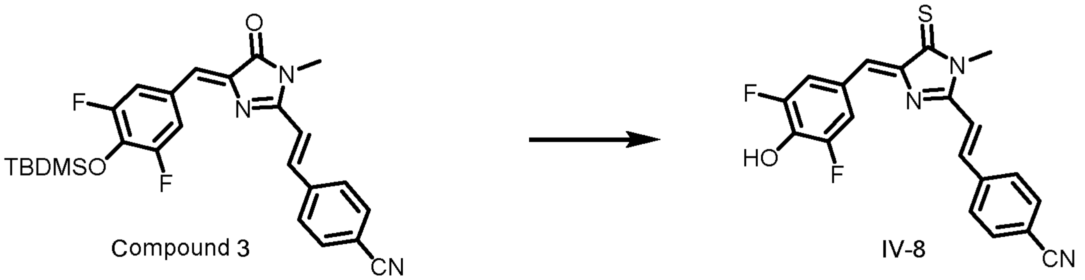
Compound 3 IV-8

This compound was obtained by following the general procedure for Compound IV-7, ( 0.332 g, 49%)。$^1$H NMR (400 MHz, DMSO-$d_6$) δ 11.00 (s, 1H), 8.11 (d, J = 8.5 Hz, 2H), 8.07 (dd, J = 7.8, 2.1 Hz, 2H), 8.04 (s, 1H), 7.94 (d, J = 8.3 Hz, 2H), 7.44 (d, J = 15.9 Hz, 1H), 7.03 (s, 1H), 3.29 (s, 3H).HMS(ESI): m/z Calcd. For $C_{20}H_{13}F_2N_3NaOS$ 404.0645; found 404.0646, $[M+Na]^+$.

Compound Ⅳ-9：

Compound 5：

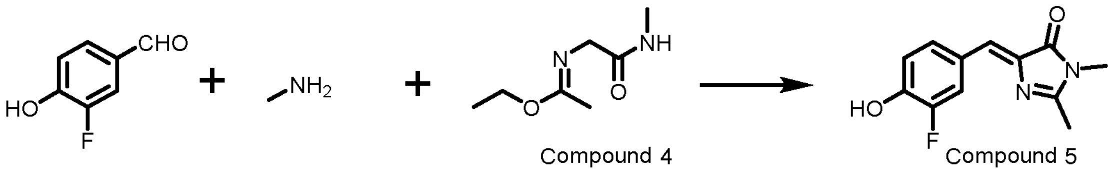
Compound 4 Compound 5

To a stirred solution of 3-Fluor-4-hydroxybenzaldehyde (0.560 g, 4.0 mmol) and 5 mL 33% methylamine aqueous solution in 40 mL anhydrous ethanol, 10 g $Na_2SO_4$ was added in one portion. The obtained mixture was stirred and kept at room temperature for 24 hr, then filtered and dried with additional $Na_2SO_4$. The solvent was removed under reduce pressure to give the intermediate which was used directly without any further purification. After re-dissolved in 10 mL anhydrous methanol, compound 4 (0.790 g, 5.0 mmol) was added. The complex was stirred and kept at room temperature for 12 hr, the precipitated product was filtered and washed with cooled methanol for three times to give the yellow compound 5. (0.796 g, 85%).$^1$H NMR (400 MHz, DMSO-$d_6$) δ10.52 (s, 1H), 8.19 (m, 1H), 7.76 (m, 1H), 6.99 (t, J=8.8 Hz, 1H), 6.89 (s, 1H), 3.09 (s, 3H), 2.34(s, 3H). MS(ESI): m/z Calcd. For $C_{12}H_{10}FN_2O_2$ 234.2; found 234.2, $[M-H]^-$.

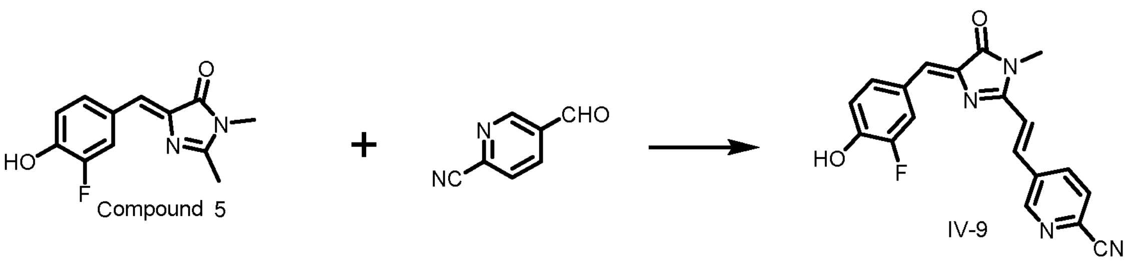
Compound 5 IV-9

This compound was obtained by following the general procedure for CompoundIV-1, ( 0.239 g, 21%)。$^1$H NMR (400 MHz, DMSO-$d_6$) δ10.52 (s, 1H), 8.71 (s, 1H), 8.19 (m, 1H), 7.84 (d, J=8.0 Hz, 1 H), 7.76(m, 1H), 7.67 (d, J=8.4 Hz, 1 H),7.32 (m, 1 H), 6.99 (t, J=8.8 Hz, 1H), 6.89 (s, 1H), 6.78 (m, 1H), 2.34(s, 3H). HR-MS(ESI): m/z Calcd. For $C_{19}H_{12}FN_4O_2$ 347.0950; found 347.0951, $[M-H]^-$.

Compound IV-10 :

Compound 6 :

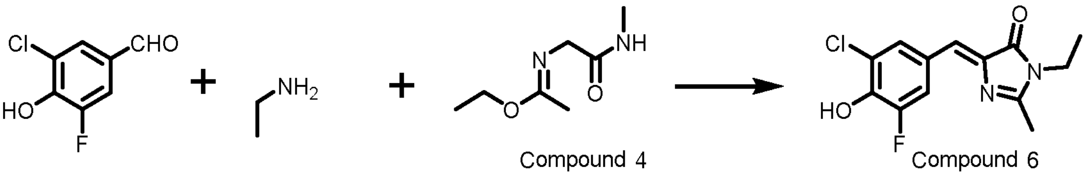


This compound was obtained by following the general procedure for Compound 5, ( 0.812 g, 91%)。$^1$H NMR (400 MHz, $CD_3OD$) δ 7.28 (s, 1H), 7.19 (d, J = 8.0 Hz, 2H), 3.59 (t, J=5.6Hz, 3H), 3.12 (s, 3H), 1.23(q, J=5.6Hz, 3H). MS(ESI): m/z Calcd. For $C_{13}H_{11}ClFN_2O_2$ 281.0; found 281.0, $[M-H]^-$.

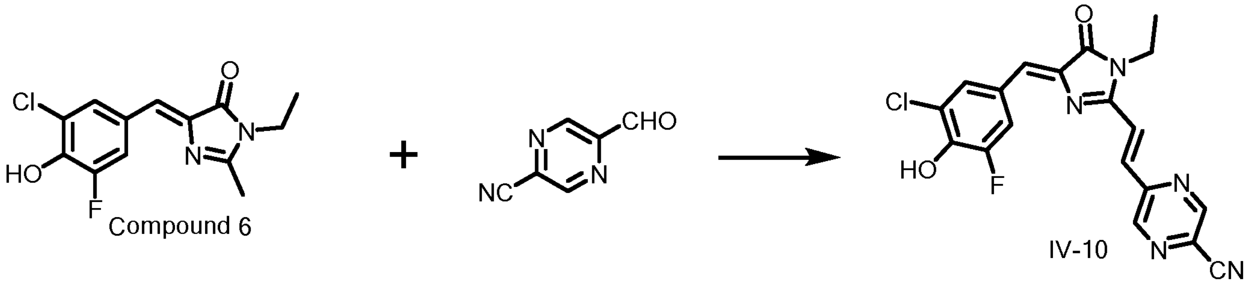


This compound was obtained by following the general procedure for CompoundIV-1, ( 0.239 g, 21%)。$^1$H NMR (400 MHz, $CD_3OD$) δ 7.84 (s, 2H), 7.28 (s, 1H), 7.19 (d, J = 8.0 Hz, 2H), 3.12 (s, 3H), 1.23(q, J=5.6Hz, 3H). HR-MS(ESI): m/z Calcd. For $C_{21}H_{14}ClFN_3O_2$ $C_{19}H_{12}ClFN_5O_2$ 396.0669; found 396.0668, $[M-H]^-$.

Compound IV-11 :

Compound 7 :

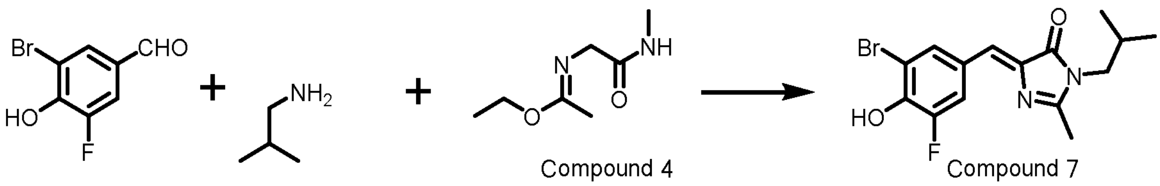


This compound was obtained by following the general procedure for Compound 5, ( 0.812 g, 91%)。$^1$H NMR (400 MHz, $CD_3OD$) δ 7.28 (s, 1H), 7.19 (d, J = 8.0 Hz, 2H), 3.81 (s, 3H) , 3.12 (s, 3H), 3.12 (s, 3H), 1.58(m, 1H), 1.11 (d, 6H). MS(ESI): m/z Calcd. For $C_{15}H_{15}BrFN_2O_2$ 353.0; found 353.0, $[M-H]^-$.

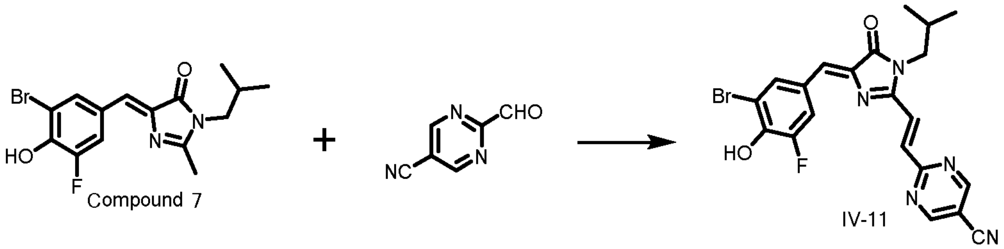

This compound was obtained by following the general procedure for Compound IV-1, (0.209 g, 22%)。$^1$H NMR (400 MHz, $CD_3OD$) δ 7.95 (d, J = 15.7 Hz, 1H), 7.74 (d, J = 8.7 Hz, 2H), 7.28 (s, 1H), 7.19 (d, J = 8.0 Hz, 2H),7.01 (d, J = 15.7 Hz, 1H), 6.87 - 6.83 (m, 2H), 3.12 (s, 3H) , 1.58(m, 1H), 1.11 (d, 6H). HR-MS(ESI): m/z Calcd. For $C_{21}H_{16}BrFN_5O_2$ 468.0477; found468.0478, [M-H]$^-$.

Compound Ⅳ-12：

Compound 8：

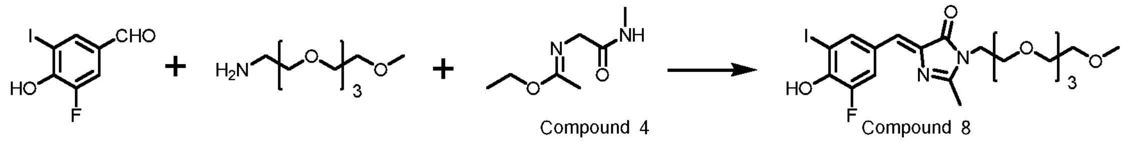

Compound 4

Compound 8

This compound was obtained by following the general procedure for Compound 5, ( 0.812 g, 91%)。$^1$H NMR (400 MHz, CD3OD) δ10.52 (s, 1H), 7.76 (d, J=8.5 Hz, 2H), 6.95 (s, 1H), 3.79 (t, J=5.2 Hz, 2H), 3.68-3.56(m, 8H), 3.55-3.48(m, 2H), 3.35 (s, 3H), 2.39(s, 3H). MS(ESI): m/z Calcd. For $C_{20}H_{25}FIN_2O_6$ 535.1; found 535.1, [M-H]$^-$.

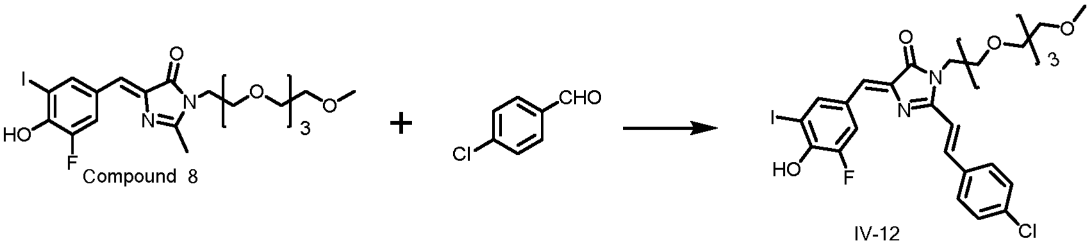

Compound 8

IV-12

This compound was obtained by following the general procedure for Compound 1, ( 0.156 g, 18%)。$^1$H NMR (400 MHz, $CD_3OD$) δ10.52 (s, 1H), 7.98-7.86 (m, 3H), 7.76 (d, J=8.5 Hz, 2H), 7.31 (t, *J* = 8.9 Hz, 2H), 7.18 (d, *J* = 15.9 Hz, 1H), 6.95 (s, 1H), 3.79 (t, J=5.2 Hz, 2H), 3.68-3.56(m, 8H), 3.55-3.48(m, 2H), 3.35 (s, 3H), 2.39(s, 3H). HR-MS(ESI): m/z Calcd. For $C_{27}H_{28}ClFIN_2O_6$ 657.0670; found657.0671, [M-H]$^-$.

Compound Ⅳ-13：

Compound 9：

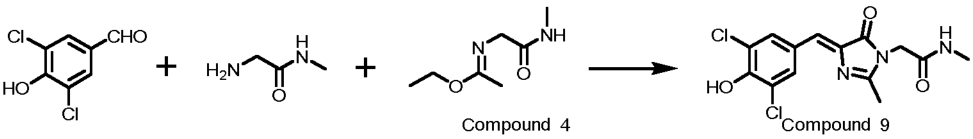

Compound 4

Compound 9

This compound was obtained by following the general procedure for Compound 5, ( 0.732 g, 93%)。$^1$H NMR (400 MHz, $CD_3OD$) δ10.52 (s, 1H), 7.76 (d, J=8.5 Hz, 2H), 6.95 (s, 1H), 4.12 (s, 3H), 3.62(s, 3H), 2.39(s, 3H). MS(ESI): m/z Calcd. For $C_{14}H_{12}Cl_2N_3O_3$ 340.0; found 340.0, [M-H]$^-$.

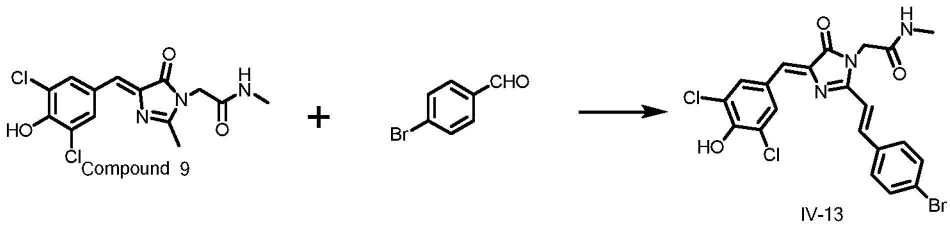

Compound 9

IV-13

This compound was obtained by following the general procedure for Compound 1, ( 0.156 g, 18%) 。 $^1$H NMR (400 MHz, $CD_3OD$) δ10.52 (s, 1H), 8.07 - 8.01 (m, 2H), 7.95 (d, J = 15.7 Hz, 1H),7.76 (d, J=8.5 Hz, 2H), 7.01 (d, J = 15.7 Hz, 1H),6.95 (s, 1H), 6.87 - 6.83 (m, 2H), 4.12 (s, 3H), 3.62(s, 3H), 2.39(s, 3H).HR-MS(ESI): m/z Calcd. For $C_{21}H_{15}BrCl_2N_3O_3$ 505.9679; found505.9680, $[M-H]^-$.

Compound Ⅳ-14 :

Compound 10 :

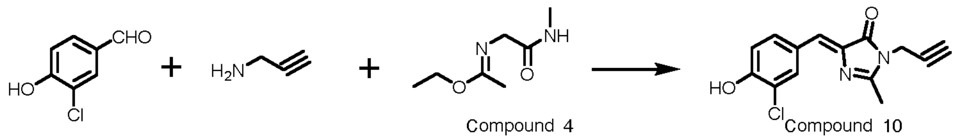
Compound 4
Compound 10

This compound was obtained by following the general procedure for Compound 5, ( 0.732 g, 93%) 。 $^1$H NMR (400 MHz, DMSO-$d_6$) δ10.52 (s, 1H), 8.19 (m, 1H), 7.76 (m, 1H), 6.99 (t, J=8.8 Hz, 1H), 6.89 (s, 1H), 3.89 (s, 3H), 2.34(s, 3H). MS(ESI): m/z Calcd. For $C_{14}H_{10}ClN_2O_2$ 273.0; found 273.0, $[M-H]^-$.

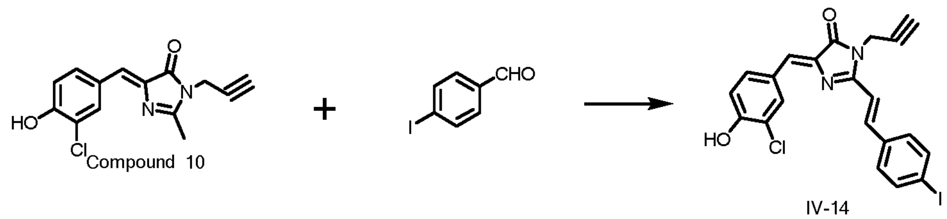
Compound 10
IV-14

This compound was obtained by following the general procedure for Compound 1, ( 0.156 g, 18%) 。 $^1$H NMR (400 MHz, DMSO-$d_6$) δ10.52 (s, 1H), 8.19 (m, 1H), 8.07 - 8.01 (m, 2H), 7.95 (d, J = 15.7 Hz, 1H), 7.76 (m, 1H), 7.01 (d, J = 15.7 Hz, 1H), 6.99 (t, J=8.8 Hz, 1H), 6.89 (s, 1H), 6.87 - 6.83 (m, 2H), 3.89 (s, 3H), 2.34(s, 3H). HR-MS(ESI): m/z Calcd. For $C_{21}H_{13}ClIN_2O_2$ 486.9716; found 486.9715, $[M-H]^-$.

Compound Ⅳ-15 :

Compound 11 :

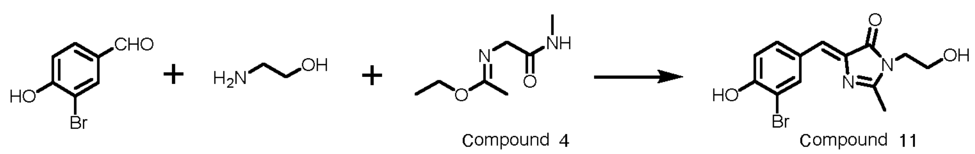
Compound 4
Compound 11

This compound was obtained by following the general procedure for Compound 5, ( 0.732 g, 93%) 。 $^1$H NMR (400 MHz, CD3OD) δ10.52 (s, 1H), 8.19 (m, 1H), 7.76 (m, 1H), 6.99 (t, J=8.8 Hz, 1H), 6.89 (s, 1H), 3.29 (t, J=4.4 Hz, 2H), 2.78(t, J=4.4 Hz, 2H), 2.34(s, 3H). MS(ESI): m/z Calcd. For $C_{13}H_{12}BrN_2O_3$ 323.0; found 323.0, $[M-H]^-$.

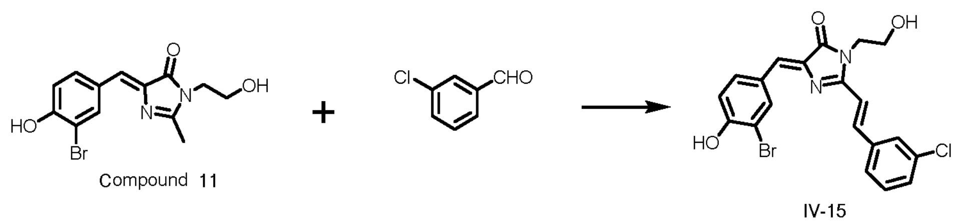
Compound 11
IV-15

This compound was obtained by following the general procedure for Compound 1, ( 0.156 g, 18%) 。 $^1$H NMR(400 MHz, CD3OD) δ10.52 (s, 1H), 8.19 (m, 1H), 7.95 (d, J = 15.7 Hz, 1H), 7.76 (m, 1H), 7.49(m, 1H), 7.40-7.22(m, 3H), 7.01

(d, J = 15.7 Hz, 1H), 6.99 (t, J=8.8 Hz, 1H), 6.89 (s, 1H), 3.29 (t, J=4.4 Hz, 2H), 2.78(t, J=4.4 Hz, 2H), 2.34(s, 3H). HR-MS(ESI): m/z Calcd. For $C_{20}H_{15}BrClN_2O_3$ 444.9960; found 444.6991, $[M-H]^-$.

Compound Ⅳ-16：

Compound 12：

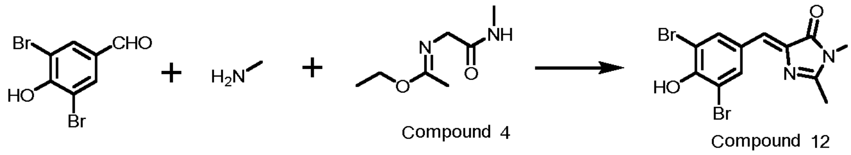

Compound 4

Compound 12

This compound was obtained by following the general procedure for Compound 5, ( 0.732g, 93%) $_o$ $^1H$ NMR (400 MHz, $CD_3OD$) δ10.52 (s, 1H), 7.76 (d, J=8.5 Hz, 2H), 6.95 (s, 1H), 2.42 (s, 3H), 2.39(s, 3H). MS(ESI): m/z Calcd. For $C_{12}H_9Br_2N_2O_2$ 370.9; found 370.9, $[M-H]^-$.

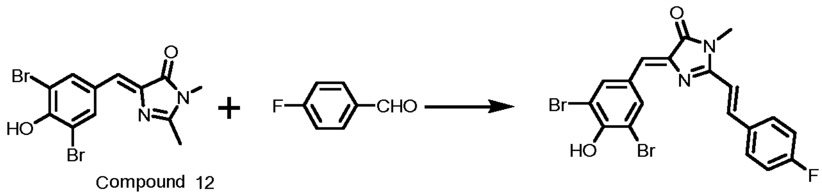

Compound 12

IV-16

This compound was obtained by following the general procedure for Compound 1, ( 0.156 g, 18%) $_o$ $^1H$ NMR (400 MHz, $CD_3OD$) δ10.52 (s, 1H), 8.07 - 8.01 (m, 1H), 7.76 (d, J=8.5 Hz, 2H), 7.97 (d, J = 15.7 Hz, 1H), 7.01 (d, J = 15.7 Hz, 1H), 6.87 - 6.83 (d,J=8.2 Hz, 2H), 6.95 (d,J=8.2 Hz, 2H)), 2.39(s, 3H). HR-MS(ESI): m/z Calcd. For $C_{19}H_{12}Br_2FN_2O_3$ 476.9255; found 476.9256, $[M-H]^-$.

Compound Ⅳ-17：

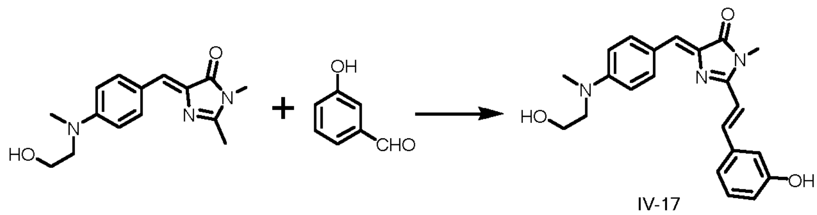

IV-17

This compound was obtained by following the general procedure for Compound 1, ( 0.286 g, 28%) $_o$ $^1H$-NMR(400 MHz,DMSO-$d_6$): δ 8.15 (d, J = 8.6 Hz, 2H), 7.82 (d, J = 15.8 Hz, 1H), 7.31 - 7.22 (m, 2H), 7.21 - 7.17 (m, 1H), 7.10 (d, J = 15.8 Hz, 1H), 6.94 (s, 1H), 6.88 - 6.73 (m, 3H), 3.59 (t, J = 5.8 Hz, 2H), 3.51 (t, J = 6.0 Hz, 2H), 3.26 (s, 3H), 3.05 (s, 3H). HR-MS(ESI): m/z Calcd. For $C_{22}H_{24}N_3O_3$ 378.1818; found 378.1819, $[M+H]^+$.

Compound Ⅳ-18 :

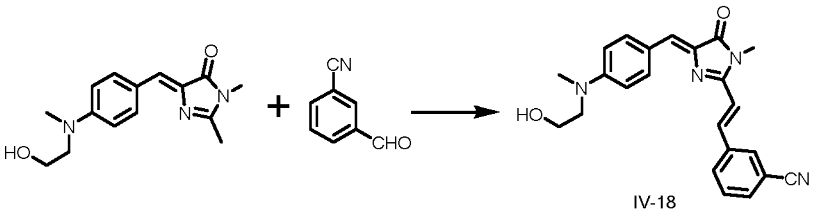

IV-18

This compound was obtained by following the general procedure for Compound 1, ( 0.486 g, 60%)。$^1$H-NMR(400 MHz, DMSO-$d_6$): δ 8.43 (d, J = 1.8 Hz, 1H), 8.16 (t, J = 8.5 Hz, 3H), 7.93 (d, J = 15.9 Hz, 1H), 7.85 (dt, J = 7.8, 1.4 Hz, 1H), 7.66 (t, J = 7.8 Hz, 1H), 7.40 (d, J = 15.9 Hz, 1H), 6.98 (s, 1H), 6.86-6.71 (m, 2H), 3.60 (t, J = 5.9 Hz, 2H), 3.52 (t, J = 5.9 Hz, 2H), 3.28 (s, 3H), 3.06 (s, 3H). HR-MS(ESI): m/z Calcd. For $C_{23}H_{23}N_4O_2$ 387.1821; found 387.1822, $[M+H]^+$.

Compound Ⅳ-19 :

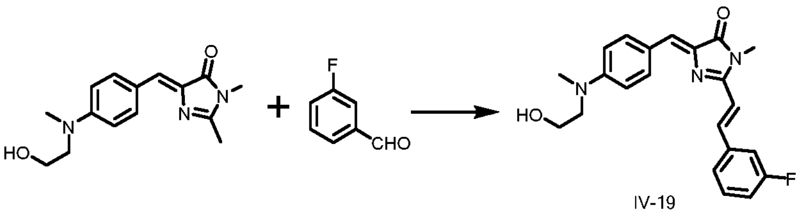

IV-19

This compound was obtained by following the general procedure for Compound 1, ( 0.286 g, 38%)。$^1$H-NMR(400 MHz, DMSO-$d_6$): δ 8.17 (d, J = 8.6 Hz, 2H), 7.91 (d, J = 15.8 Hz, 1H), 7.79 (dt, J = 10.6, 2.1 Hz, 1H), 7.66 (d, J = 7.8 Hz, 1H), 7.50 (td, J = 8.0, 6.2 Hz, 1H), 7.34-7.20 (m, 2H), 6.97 (s, 1H), 6.84-6.73 (m, 2H), 3.59 (t, J = 5.9 Hz, 2H), 3.52 (t, J = 5.9 Hz, 2H), 3.27 (s, 3H), 3.06 (s, 3H). MS(ESI): m/z Calcd. For $C_{22}H_{23}N_3O_3F$ 380.1774; found 380.1775, $[M+H]^+$.

Compound Ⅳ-20 :

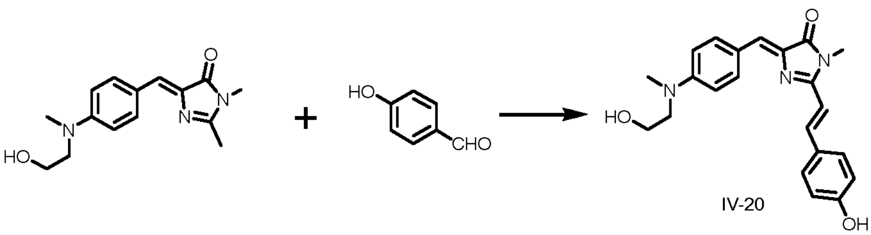

IV-20

This compound was obtained by following the general procedure for Compound 1, ( 0.222 g, 30%)。$^1$H NMR (400 MHz, DMSO-$d_6$): 8.14 (d, J= 8.5 Hz, 2H), 7.84 (d, *J*= 15.7 Hz, 1H), 7.68 (d, *J* = 8.4 Hz, 2H), 6.97 (d, *J* = 15.8 Hz, 1H), 6.88 (s, 1H), 6.84 (d, *J* = 8.5 Hz, 2H), 6.79 (d, *J* = 8.8 Hz, 2H), 3.59 (q, *J* = 5.4 Hz, 2H), 3.51 (t, *J* = 5.9 Hz, 2H), 3.24 (s, 3H), 3.05 (s, 3H). MS(ESI): m/z Calcd. For $C_{22}H_{24}N_3O_3$ 378.1818; found 378.1819, $[M+H]^+$.

Compound Ⅳ-21 :

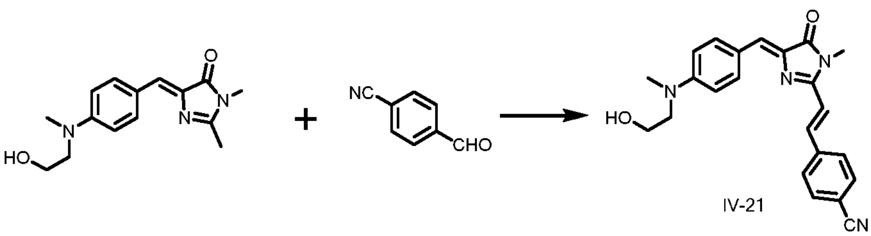

IV-21

This compound was obtained by following the general procedure for Compound 1, ( 0.352 g, 56%)$_{o}$ $^{1}$H-NMR(400 MHz,DMSO-$d_6$): δ 8.18 (d, J = 8.5 Hz, 2H), 8.08-8.02 (m, 2H), 7.98-7.90 (m, 3H), 7.40 (d, J = 15.9 Hz, 1H), 7.00 (s, 1H), 6.86-6.72 (m, 2H), 3.60 (t, J = 5.9 Hz, 2H), 3.52 (t, J = 5.6 Hz, 2H), 3.28 (s, 3H), 3.06 (s, 3H). MS(ESI): m/z Calcd. For $C_{23}H_{23}N_4O_2$ 387.1821; found 387.1820, [M+H]$^+$.

Compound Ⅳ-22 :

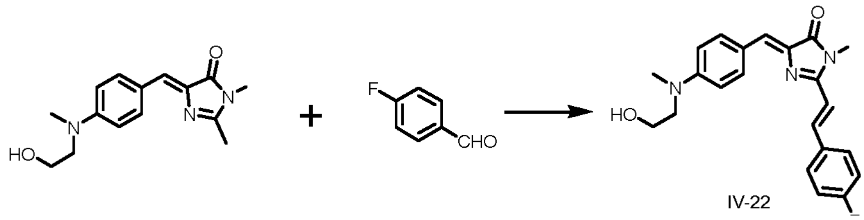

IV-22

This compound was obtained by following the general procedure for Compound 1, ( 0.252 g, 32%)$_{o}$ $^{1}$H-NMR(400 MHz, DMSO-$d_6$): δ 8.16 (d, *J* = 8.5 Hz, 2H), 7.93 (t, *J* = 4.4 Hz, 2H), 7.90 (d, *J* = 6.1 Hz, 1H), 7.31 (t, *J* = 8.9 Hz, 2H), 7.18 (d, *J* = 15.9 Hz, 1H), 6.95 (s, 1H), 6.83-6.72 (m, 2H), 3.59 (t, *J* = 5.9 Hz, 2H), 3.51 (t, *J* = 5.9 Hz, 2H), 3.27 (s, 3H), 3.05 (s, 3H). MS(ESI): m/z Calcd. For $C_{22}H_{23}FN_3O_2$ 380.1774; found 380.1775, [M+H]$^+$.

Compound Ⅳ-23 :

Compound 13 :

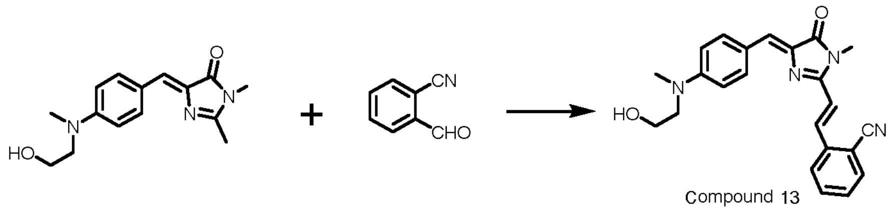

Compound 13

This compound was obtained by following the general procedure for Compound 1, ( 0.252 g, 32%)$_{o}$ $^{1}$H-NMR(400 MHz, DMSO-$d_6$): δ 8.43 (d, J = 1.8 Hz, 1H), 8.16 (t, J = 8.5 Hz, 3H), 7.93 (d, J = 15.9 Hz, 1H), 7.85 (dt, J = 7.8, 1.4 Hz, 1H), 7.66 (t, J = 7.8 Hz, 1H), 7.40 (d, J = 15.9 Hz, 1H), 6.98 (s, 1H), 6.86-6.71 (m, 2H), 3.60 (t, J = 5.9 Hz, 2H), 3.52 (t, J = 5.9 Hz, 2H), 3.28 (s, 3H), 3.06 (s, 3H). MS(ESI): m/z Calcd. For $C_{23}H_{23}N_4O_2$ 387.2; found 387.2, [M+H]$^+$.

Compound 14 :

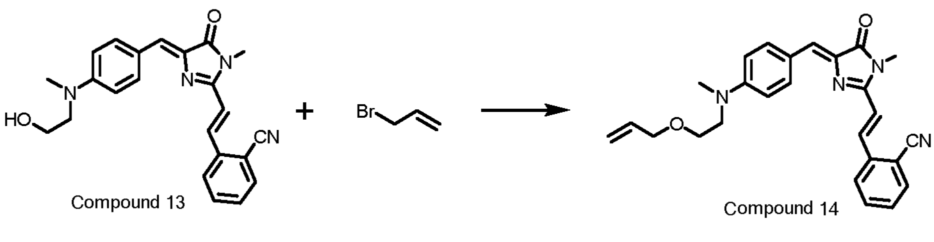

Compound 13

Compound 14

2 ml Allyl bromide was added to the mixture of compound 14 (0.774 g, 2.0 mmol), $K_2CO_3$ (0.276g, 2.0 mmol) in acetonitrile (100 ml) with constant stirring. This reaction mixture was heated to reflux. The progress of reaction was monitored on silica gel TLC. After completion of reaction, the reaction mixture was filtered then the solvent was removed under reduce pressure to give the crude product, then purified by silica gel column chromatography to afford a target compound 18 (0.673 g, 79%). $^{1}$H-NMR(400 MHz, DMSO-$d_6$): δ 8.43 (d, J= 1.8 Hz, 1H), 8.16 (t, J = 8.5 Hz, 3H), 7.93 (d, J = 15.9 Hz, 1H), 7.85 (dt, J = 7.8, 1.4 Hz, 1H), 7.66 (t, J = 7.8 Hz, 1H), 7.40 (d, J = 15.9 Hz, 1H), 6.98 (s, 1H), 6.86-6.71 (m, 2H), 3.81 (s,2H), 3.60 (t, J = 5.9 Hz, 2H), 3.52 (t, J = 5.9 Hz, 2H), 3.41 (s, 3H), 3.06 (s, 3H). MS(ESI): m/z Calcd. For $C_{26}H_{27}N_4O_2$ 427.2; found 427.2, [M+H]$^+$.

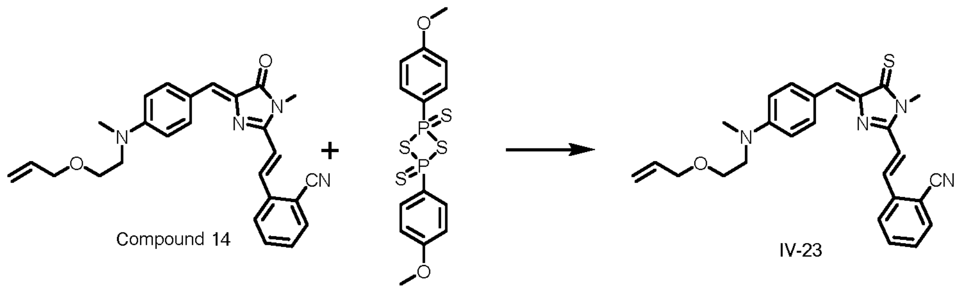

Compound 14

IV-23

This compound was obtained by following the general procedure for Compound 7, ( 0.152 g, 72%)。$^1$H-NMR(400 MHz, DMSO-$d_6$): δ 8.43 (d, J = 1.8 Hz, 1H), 8.16 (t, J = 8.5 Hz, 3H), 7.93 (d, J = 15.9 Hz, 1H), 7.85 (dt, J = 7.8, 1.4 Hz, 1H), 7.66 (t, J = 7.8 Hz, 1H), 7.40 (d, J = 15.9 Hz, 1H), 6.98 (s, 1H), 6.86-6.71 (m, 2H), 3.81 (s,2H), 3.60 (t, J = 5.9 Hz, 2H), 3.52 (t, J = 5.9 Hz, 2H), 3.41 (s, 3H), 3.06 (s, 3H). MS(ESI): m/z Calcd. For $C_{26}H_{27}N_4OS$ 443.1906; found 443.1905, $[M+H]^+$.

Compound Ⅳ-24：

Compound 15：

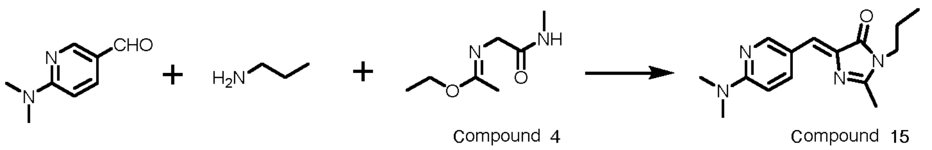

Compound 4

Compound 15

This compound was obtained by following the general procedure for Compound 5, ( 0.692 g, 82%)。$^1$H NMR (400 MHz, $CD_3OD$) δ =8.02 (d, J= 2.4 Hz, 1H), 7.44 (dd, J= 8.7 Hz, J= 2.4 Hz, 1H), 7.09 (s, 1H), 6.51 (d, J= 8.7 Hz, 1H), 3.56 (t, J HH = 7.6 Hz, 2H), 3.08 (s, 6H), 1.66 (m, 2H), 2.38 (s, 3H), 0.95 (t, J = 7.6 Hz, 3H). MS(ESI): m/z Calcd. For $C_{15}H_{21}N_4O$ 273.2; found 273.2, $[M+H]^+$.

Compound 16：

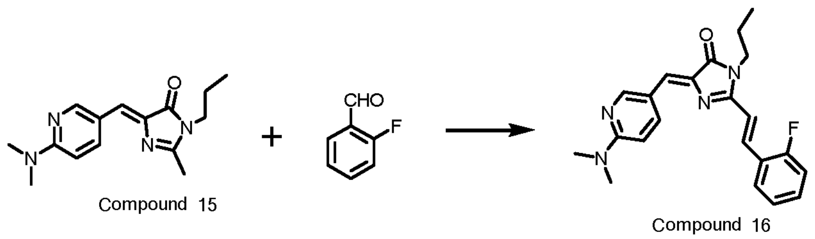

Compound 15

Compound 16

This compound was obtained by following the general procedure for Compound 1, ( 0.452 g, 34%)。$^1$H NMR (400 MHz, $CD_3OD$) δ =8.02 (d, J= 2.4 Hz, 1H), 7.95 (m, 2H), 7.68-7.50 (m, 1H), 7.44 (dd, J= 8.7 Hz, J= 2.4 Hz, 1H), 7.34-7.06 (m, 2H), 7.09 (s, 1H), 7.00 (d, J = 15.7 Hz, 1H), 6.51 (d, J= 8.7 Hz, 1H), 3.56 (t, J HH = 7.6 Hz, 2H), 3.08 (s, 6H), 1.66 (m, 2H), 2.38 (s, 3H), 0.95 (t, J = 7.6 Hz, 3H). MS(ESI): m/z Calcd. For $C_{22}H_{24}FN_4O$ 379.2; found 379.2, $[M+H]^+$.

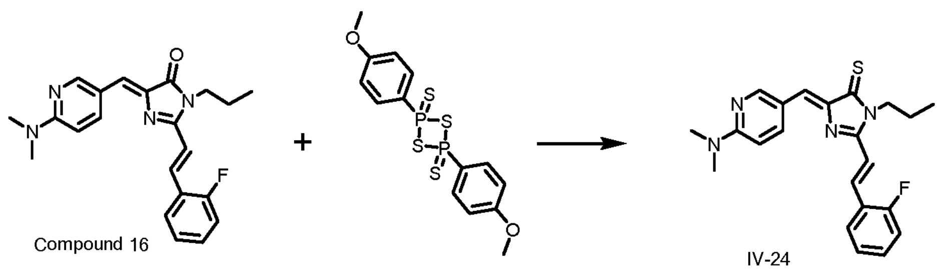

Compound 16

IV-24

This compound was obtained by following the general procedure for Compound 7, ( 0.152 g, 72%) 。$^{1}H$ NMR (400 MHz, $CD_3OD$) δ =8.02 (d, J= 2.4 Hz, 1H), 7.95 (m, 2H), 7.68-7.50 (m, 1H), 7.44 (dd, J= 8.7 Hz, J= 2.4 Hz, 1H), 7.34-7.06 (m, 2H), 7.09 (s, 1H), 7.00 (d, J = 15.7 Hz, 1H), 6.51 (d, J= 8.7 Hz, 1H), 3.56 (t, J HH = 7.6 Hz, 2H), 3.08 (s, 6H), 1.66 (m, 2H), 2.38 (s, 3H), 0.95 (t, J = 7.6 Hz, 3H). HR-MS(ESI): m/z Calcd. For $C_{22}H_{24}FN_4S$ 395.1706; found 395.1705, $[M+H]^+$.

Compound IV-25:

Compound 17 :

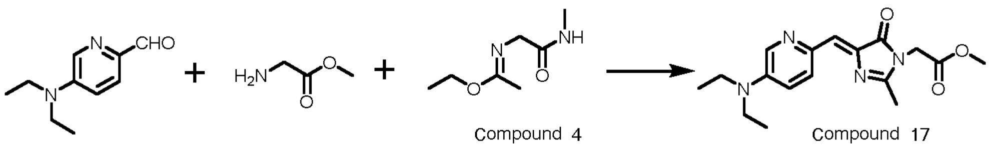

Compound 4

Compound 17

This compound was obtained by following the general procedure for Compound 5, ( 0.692 g, 82%) 。$^{1}H$ NMR (400 MHz, $CD_3OD$) δ =8.02 (d, J= 2.4 Hz, 1H), 7.44 (dd, J= 8.7 Hz, J= 2.4 Hz, 1H), 7.09 (s, 1H), 6.51 (d, J= 8.7 Hz, 1H), 4.21(s, 2H), 3.86 (s, 2H), 3.00 (q, J=4.8 Hz, 4H),2.45 (s, 3H), 1.22 (t, J = 4.8 Hz, 6H). MS(ESI): m/z Calcd. For $C_{17}H_{23}N_4O_3$ 331.2; found 331.2, $[M+H]^+$.

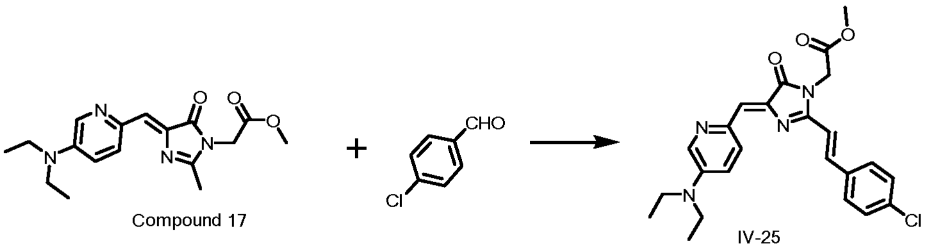

Compound 17

IV-25

This compound was obtained by following the general procedure for Compound 1, ( 0.452 g, 34%) 。$^{1}H$ NMR (400 MHz, $CD_3OD$) δ =8.02 (d, J= 2.4 Hz, 1H), 7.44 (dd, J= 8.7 Hz, J= 2.4 Hz, 1H), 7.31 (t, J = 8.9 Hz, 2H), 7.18 (d, J = 15.9 Hz, 1H), 7.09 (s, 1H), 6.95 (s, 1H), 6.83-6.72 (m, 2H), 6.51 (d, J= 8.7 Hz, 1H), 4.21(s, 2H), 3.86 (s, 2H), 3.00 (q, J=4.8 Hz, 4H),2.45 (s, 3H), 1.22 (t, J = 4.8 Hz, 6H). MS(ESI): m/z Calcd. For $C_{24}H_{26}ClN_4O_3$ 453.1693; found 453.1694, $[M+H]^+$.

Compound IV-26:

Compound 18 :

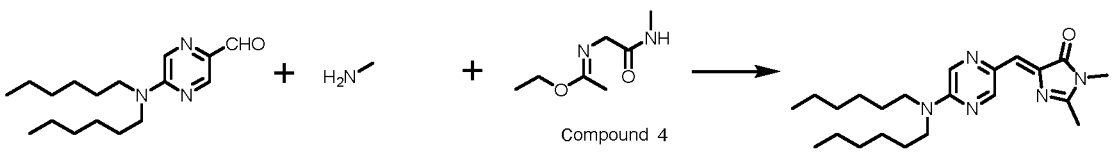

Compound 4

Compound 18

This compound was obtained by following the general procedure for Compound 5, ( 0.892 g, 80%) 。$^{1}H$ NMR (400 MHz, $CD_3OD$) δ8.41 (d, J = 1.5 Hz, 1 H), 7.97 (d, J = 1.5 Hz, 1 H), 7.31(s, 1H), 5.86 (s, 1 H), 3.46 (t, J = 6.6 Hz, 4 H), 3.15(s, 3H), 2.32(s, 3H), 1.61 (m, 4 H),1.32 (m, 12 H), 0.89 (t, 6 H). MS(ESI): m/z Calcd. For $C_{22}H_{36}N_5O$ 386.3; found 386.3, $[M+H]^+$.

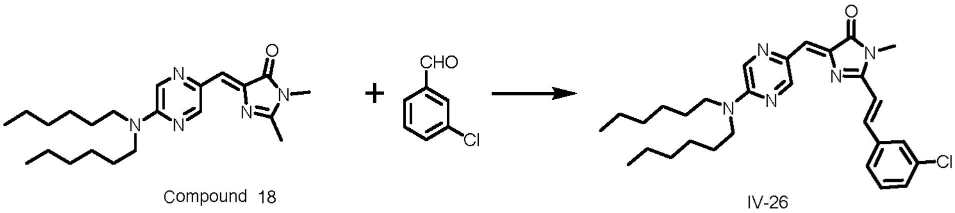

Compound 18

IV-26

This compound was obtained by following the general procedure for Compound 1, ( 0.452 g, 34%) 。 $^1$H NMR (400 MHz, $CD_3OD$) δ8.41 (d, J = 1.5 Hz, 1 H), 7.97 (d, J = 1.5 Hz, 1 H), 7.85 (d, J = 15.7 Hz, 1H), 7.49(m, 1H), 7.40-7.22(m, 3H), 7.31(s, 1H), 7.01 (d, J = 15.7 Hz, 1H), 5.86 (s, 1 H), 3.46 (t, J = 6.6 Hz, 4 H), 3.15(s, 3H), 2.32(s, 3H), 1.61 (m, 4 H),1.32 (m, 12 H), 0.89 (t, 6 H). MS(ESI): m/z Calcd. For $C_{29}H_{39}ClN_5O$ 508.2843; found 508.2842, $[M+H]^+$.

## Compound Ⅳ-27:

### Compound 19 :

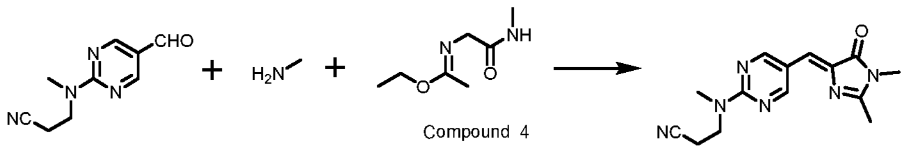

Compound 4

Compound 19

This compound was obtained by following the general procedure for Compound 5, ( 0.812 g, 81%) 。 $^1$H NMR (400 MHz, $CDCl_3$) δ 7.93(s, 2H), 7.31 (s, 1H), 4.24(t, J=6.8 Hz, 2H), 3.44 (s, 3H), 2.82(s, 2H), 2.43(s, 3H). MS(ESI): m/z Calcd. For $C_{14}H_{17}N_6O$ 285.1; found 285.1, $[M+H]^+$.

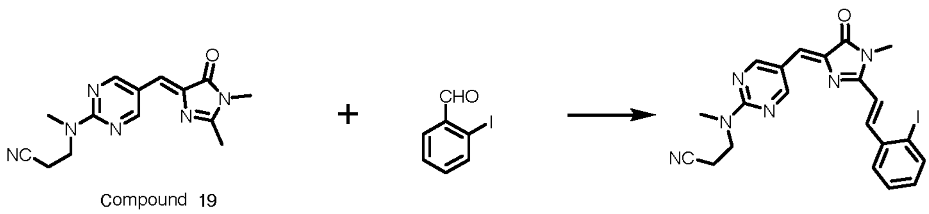

Compound 19

IV-27

This compound was obtained by following the general procedure for Compound 1, ( 0.312 g, 26%) 。 $^1$H NMR (400 MHz, $CDCl_3$) δ =8.02 (d, J = 15.7 Hz, 1H),7.93(m, 3H), 7.68-7.50 (m, 1H),7.31 (s, 1H), 7.24-7.06 (m, 2H), 7.01 (d, J = 15.7 Hz, 1H), 4.24(t, J=6.8 Hz, 2H), 3.44 (s, 3H), 2.82(s, 2H), 2.43(s, 3H). MS(ESI): m/z Calcd. For $C_{21}H_{20}IN_6O$ 499.0743; found 499.0742, $[M+H]^+$.

## Compound Ⅳ-28:

### Compound 20 :

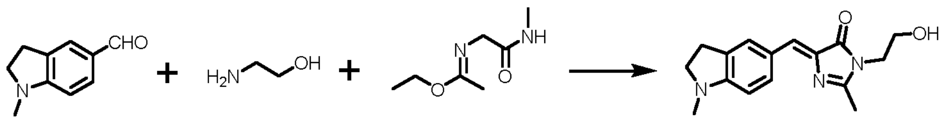

Compound 4

Compound 20

This compound was obtained by following the general procedure for Compound 5, ( 0.572 g, 80%) 。 1H NMR (400 MHz, $CDCl_3$) δ 7.60 (s, 2H), 7.16(s, 1H), 6.44 (d, J=8.26 Hz, 1H), 3.60 (t, J=8.46 Hz, 2H), 3.06 (dd, J=18.05, 9.48 Hz, 2H), 4.01 (t, J=8.4 Hz, 2H), 2.93 (t, J=8.4 Hz, 2H), 2.39(s, 3H). MS(ESI): m/z Calcd. For $C_{16}H_{20}N_3O_2$ 286.2; found

286.2, [M+H]+.

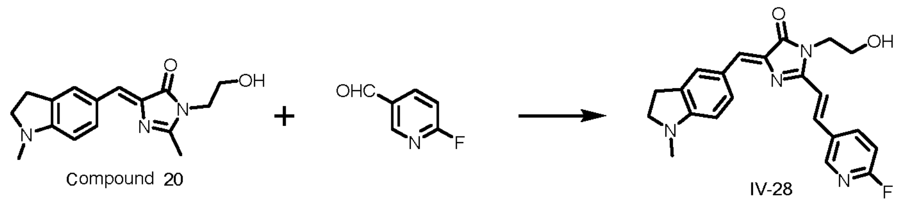

This compound was obtained by following the general procedure for Compound 1, ( 0.333 g, 26%) 。 $^1H$ NMR (400 MHz, $CDCl_3$) δ 8.75 (d, J=1.5 Hz, 1H), 8.32 (dt, J=2.1, 8.2 Hz, 1H), 7.95 (d, J = 16.0 Hz, 1H), 7.60 (s, 2H), 7.16(s, 1H), 7.05 (dd, J= 2.5, 8.4 Hz, 1H), 6.95 (d, J = 16.0 Hz, 1H), 6.44 (d, J=8.26 Hz, 1H), 3.60 (t, J=8.46 Hz, 2H), 3.06 (dd, J=18.05, 9.48 Hz, 2H), 4.01 (t, J=8.4 Hz, 2H), 2.93 (t, J=8.4 Hz, 2H). MS(ESI): m/z Calcd. For $C_{22}H_{22}FN_4O_2$ 393.1727; found 393.1726, [M+H]+.

Compound IV-29:

Compound 21 :

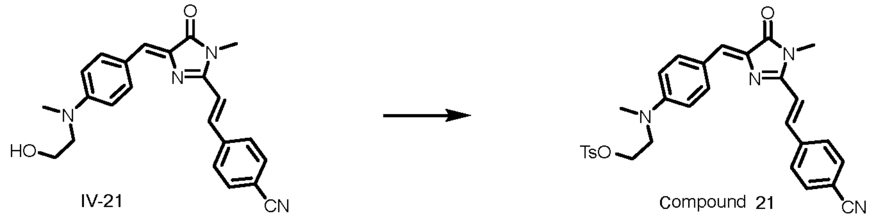

To a stirred solution of compound IV-21 (0.792 g, 2.0 mmol), p-toluenesulfonyl chloride (0.476 g, 2.5 mmol) in 100 mL dry DCM, TEA (0.303 g, 3.0 mmol) was added at rt under Ar atmosphere. The progress of reaction was monitored on silica gel TLC. After completion of reaction, the reaction was poured into 200 mL water, and extracted with DCM three times. The organic layer was dried over anhydrous $MgSO_4$, filtered, and concentrated under reduced pressure to give the crude product, then purified by silica gel column chromatography to afford a target compound 27 (0.822 g, 76%).$^1H$-NMR(400 MHz, DMSO -$d_6$): δ 8.18 (d, J = 8.5 Hz, 2H), 8.08-8.02 (m, 2H), 7.98-7.90 (m, 3H), 7.40 (d, J = 15.9 Hz, 1H), 7.18 (d, *J*=8.2 Hz, 2H), 7.00 (s, 1H), 6.86-6.72 (m, 2H), 6.47 (d, *J*=8.2 Hz, 2H), 4.06 (t, *J*=6.1 Hz, 2H), 3.49 (t, *J*=6.1 Hz, 2H),3.28 (s, 3H), 2.77 (s, 3H), 2.31 (s, 3H). MS(ESI): m/z Calcd. For $C_{30}H_{29}N_4O_4S$ 541.2; found 541.2, [M+H]+.

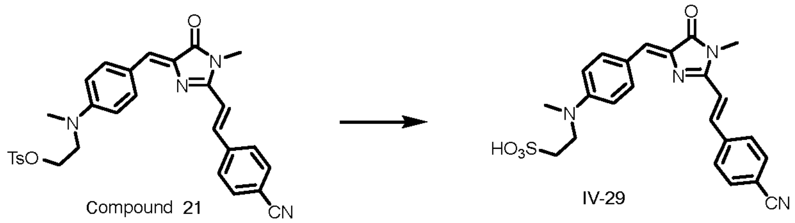

To a stirred solution of compound 20 (0.541 g, 1.0 mmol) in 20 mL dry DMF, sodium sulfite (0.630 g, 5.0 mmol) was added, the mixture solution was heated to 50 °C and stirred for 24 hrs under Ar atmosphere. The progress of reaction was monitored on silica gel TLC. After completion of reaction, the solvent was removed under reduced pressure to give the crude product, then purified by silica gel column chromatography to afford a target compound 27 (0.301 g, 60%). $^1H$-NMR(400 MHz,DMSO-$d_6$): δ 8.18 (d, J = 8.5 Hz, 2H), 8.08-8.02 (m, 2H), 7.98-7.90 (m, 3H), 7.40 (d, J = 15.9 Hz, 1H), 7.00 (s, 1H), 6.86-6.72 (m, 2H), 3.85 (m, 4H), 3.60 (t, J = 5.9 Hz, 2H), 3.52 (t, J = 5.6 Hz, 2H), 3.28 (s, 3H), 3.16 (m, 4H), 2.77 (s, 3H). MS(ESI): m/z Calcd. For $C_{23}H_{21}N_4O_4S$ 499.1289; found 499.1288, [M-H]-.

Compound Ⅳ-30:

Compound 22 :

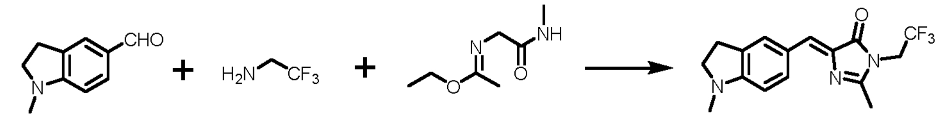

Compound 4 Compound 22

This compound was obtained by following the general procedure for Compound 5, ( 0.572 g, 80%) 。$^1$H NMR (400 MHz, $CDCl_3$) δ 7.60 (s, 2H), 7.04 (s, 1H), 6.44 (d, J=8.26 Hz, 1H), 3.60 (t, J=8.46 Hz, 2H), 3.06 (dd, J=18.05, 9.48 Hz, 2H), 4.57 (q, J= 9.25, 2H), 2.93 (s, 3H), 2.42 (s, 3H). MS(ESI): m/z Calcd. For $C_{16}H_{17}F_3N_3O$ 324.1; found 324.1, $[M+H]^+$.

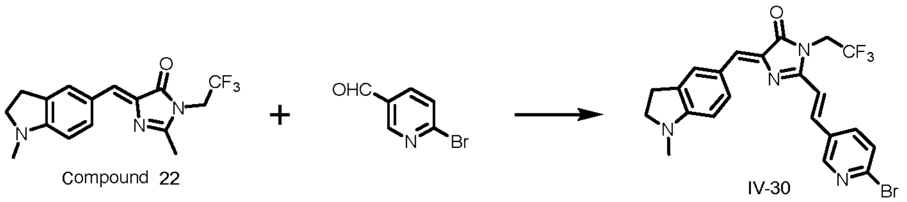

Compound 22 IV-30

This compound was obtained by following the general procedure for Compound 1, ( 0.433 g, 21%) 。$^1$H NMR (400 MHz, $CDCl_3$) δ 8.75 (d, J=1.5 Hz, 1H), 8.32 (dt, J=2.1, 8.2 Hz, 1H), 7.95 (d, J = 16.0 Hz, 1H), 7.60 (s, 2H), 7.11 (dd, J= 2.5, 8.4 Hz, 1H), 7.04 (s, 1H), 6.95 (d, J = 16.0 Hz, 1H), 6.44 (d, J=8.26 Hz, 1H), 3.60 (t, J=8.46 Hz, 2H), 3.06 (dd, J=18.05, 9.48 Hz, 2H), 4.57 (q, J= 9.25, 2H), 2.93 (s, 3H). MS(ESI): m/z Calcd. For $C_{22}H_{19}BrF_3N_4O$ 491.0694; found 491.0693, $[M+H]^+$.

Compound Ⅳ-31:

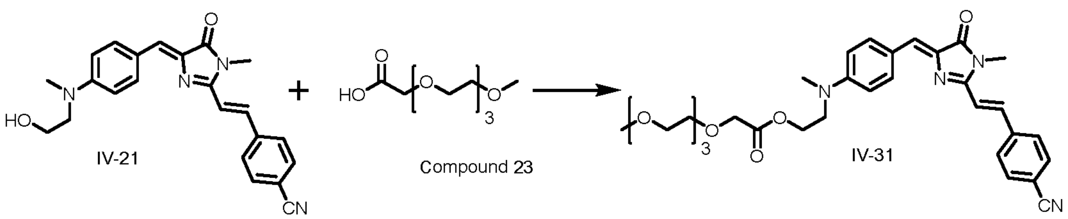

IV-21 Compound 23 IV-31

To a stirred solution of compound IV-21 (0.386 g, 1.0 mmol), compound 31 (0.265 g, 1.2 mmol), EDCI (0.382 g, 2.0 mmol) in 30 mL dry DMF, DMAP (0.183 g, 1.5 mmol) was added. The mixture solution was stirred at rt under Ar atmosphere. The progress of reaction was monitored on silica gel TLC. After completion of reaction, the solvent was removed under reduced pressure to give the crude product, then purified by silica gel column chromatography to afford a target compound IV-29 (0.490 g, 83%). $^1$H-NMR(400 MHz,DMSO-$d_6$): δ 8.18 (d, J = 8.5 Hz, 2H), 8.08-8.02 (m, 2H), 7.98-7.90 (m, 3H), 7.40 (d, J = 15.9 Hz, 1H), 7.00 (s, 1H), 6.86-6.72 (m, 2H), 4.17 (s, 2 H), 3.75 (s, 3 H), 3.6-3.7 (m, 10 H), 3.57 (m, 2H), 3.52 (t, J = 5.6 Hz, 2H), 3.38 (s, 3 H),3.28 (s, 3H), 3.06 (s, 3H). MS(ESI): m/z Calcd. For $C_{32}H_{39}N_4O_7S$ 591.2819; found 591.2820, $[M+H]^+$.

Compound Ⅳ-32:

Compound 31 :

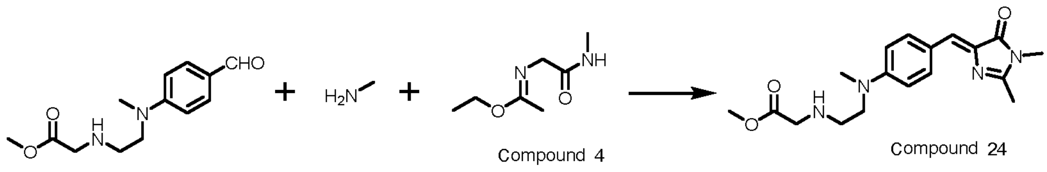

Compound 4 Compound 24

This compound was obtained by following the general procedure for Compound 5, ( 0.612 g, 87%) 。$^1$H NMR (400 MHz, $CDCl_3$) δ 8.15 (d, J=9.0 Hz, 2 H), 8.14 (d, J=9.0 Hz, 2 H), 7.21 (s, 1H), 4.23(s, 2 H), 4.11 (s, 3 H), 3.38 (t, J = 6.4 Hz, 2 H), 3.01(s, 3H), 2.92 (t, J = 6.4 Hz, 2 H), 2.41 (s, 3H). MS(ESI): m/z Calcd. For $C_{18}H_{25}N_4O_3$ 345.2; found 345.2, $[M+H]^+$.

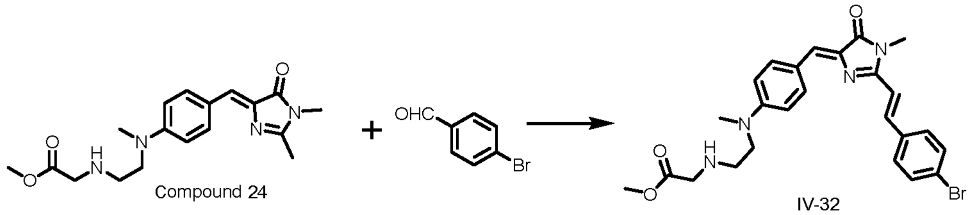

Compound 24 IV-32

This compound was obtained by following the general procedure for Compound 1, ( 0.422 g, 36%) 。$^1$H NMR (400 MHz, $CDCl_3$) δ 8.15 (d, J=9.0 Hz, 2 H), 8.14 (d, J=9.0 Hz, 2 H), 7.95 (d, J = 16.0 Hz, 1H), 7.82(d, J=8.4 Hz, 2H), 7.32 (d, J=8.4 Hz, 2H), 7.21 (s, 1H), 7.01 (d, J = 16.0 Hz, 1H), 4.23(s, 2 H), 4.11 (s, 3 H), 3.38 (t, J = 6.4 Hz, 2 H), 3.01(s, 3H), 2.92 (t, J = 6.4 Hz, 2 H). MS (ESI): m/z Calcd. For $C_{25}H_{28}BrN_4O_3$ 511.1345; found 511.1344, $[M+H]^+$.

Compound Ⅳ-33:

Compound 25 :

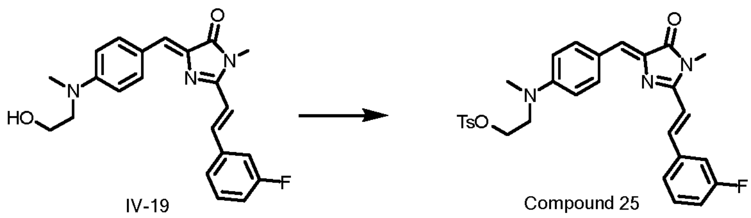

IV-19 Compound 25

This compound was obtained by following the general procedure for Compound 27, ( 0.912 g, 89%) 。$^1$H-NMR(400 MHz, DMSO-$d_6$): δ 8.17 (d, J = 8.6 Hz, 2H), 7.91 (d, J = 15.8 Hz, 1H), 7.79 (dt, J = 10.6, 2.1 Hz, 1H), 7.66 (d, J = 7.8 Hz, 1H), 7.50 (td, J = 8.0, 6.2 Hz, 1H), 7.34-7.20 (m, 2H), 7.18 (d, J=8.2 Hz, 2H), 6.97 (s, 1H), 6.84-6.73 (m, 2H), 6.47 (d, J=8.2 Hz, 2H), 4.06 (t, 1=6.1 Hz, 2H), 3.49 (t, 1=6.1 Hz, 2H), 3.27 (s, 3H), 3.06 (s, 3H), 2.77 (s, 3H), 2.31 (s, 3H). MS(ESI): m/z Calcd. For $C_{29}H_{29}FN_3O_4S$ 534.2; found 534.2, $[M+H]^+$.

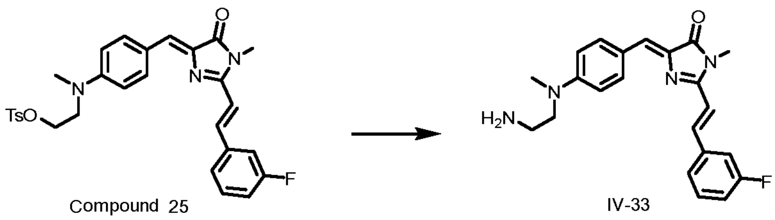

Compound 25 IV-33

To a stirred solution of compound 25 (0.534 g, 1.0 mmol) in 35 mL dry DMF, $NaN_3$ (0.195 g, 3.0 mmol) was added carefully. The mixture solution heated to 50 °C over night under Ar atmosphereThe solution was cooled down to rt and poured into 100 ml water and extracted with DCM for three times. The organic layer was dried over anhydrous $MgSO_4$, filtered, and concentrated under reduced pressure to give the crude product, which was used for next step without further purification.

To a stirred solution of crude production and $Ph_3P$ (0.524 g, 2.0 mmol) in 30 mL THF , 2 mL water was added. The mixture solution was stirred at rt under Ar atmosphere over night. After completion of reaction, the solvent was removed under reduced pressure to give the crude product, then purified by silica gel column chromatography to afford a target compound (0.301 g, 79%) . $^1$H-NMR(400 MHz, DMSO-$d_6$): δ 8.17 (d, J = 8.6 Hz, 2H), 7.91 (d, J = 15.8 Hz, 1H), 7.79 (dt, J = 10.6, 2.1 Hz, 1H), 7.66 (d, J = 7.8 Hz, 1H), 7.50 (td, J = 8.0, 6.2 Hz, 1H), 7.34-7.20 (m, 2H), 6.97 (s, 1H), 6.84-6.73 (m, 2H), 3.38 (t, J = 6.4 Hz, 2 H), 2.92 (t, J = 6.4 Hz, 2 H), 3.27 (s, 3H), 3.06 (s, 3H). MS(ESI): m/z Calcd. For $C_{22}H_{24}FN_4O$ 379.1934; found 379.1935, $[M+H]^+$.

## Compound Ⅳ-34:

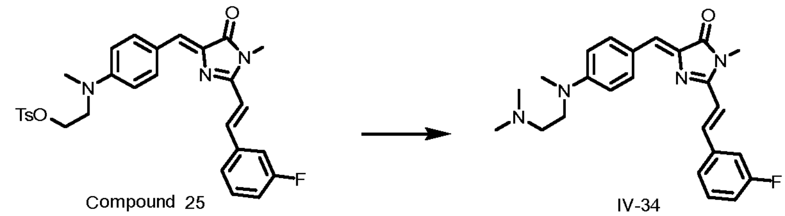

Compound 25 IV-34

To a stirred solution of compound 25 (0.534 g, 1.0 mmol) in 50 mL ethanol, Dimethylamine aqueous solution, the complete mixture was heated to reflux under Ar atmosphere. The progress of reaction was monitored on silica gel TLC. After completion of reaction, the reaction cooled down to rt and the solvent was removed under reduced pressure to give the crude product, then purified by silica gel column chromatography to afford a target compound (0.276 g, 68%). $^1$H-NMR(400 MHz, DMSO-$d_6$): δ 8.17 (d, J = 8.6 Hz, 2H), 7.91 (d, J = 15.8 Hz, 1H), 7.79 (dt, J = 10.6, 2.1 Hz, 1H), 7.66 (d, J = 7.8 Hz, 1H), 7.50 (td, J = 8.0, 6.2 Hz, 1H), 7.34-7.20 (m, 2H), 6.97 (s, 1H), 6.84-6.73 (m, 2H), 3.47 (t, J = 7.6 Hz, 2H), 2.96 (s, 3H), 2.49 (t, J = 7.6 Hz, 2H), 2.31 (s, 6H). MS(ESI): m/z Calcd. For $C_{24}H_{28}FN_4O$ 407.2247; found 407.2246, $[M+H]^+$.

## Compound Ⅳ-35:

### Compound 26 :

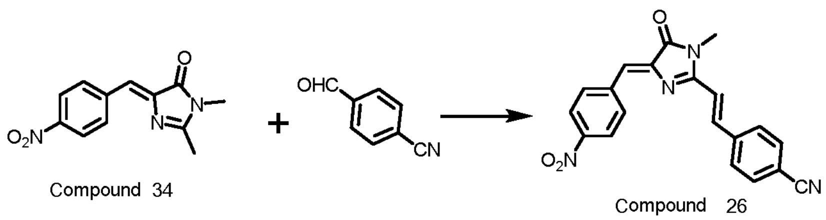

Compound 34 Compound 26

This compound was obtained by following the general procedure for Compound 1, ( 0.732 g, 39%) .$^1$H NMR (400 MHz, $CDCl_3$) δ 8.19 (d, J = 8.5 Hz, 2H), 8.07 (m, 4H), 8.04 (s, 1H), 7.94 (d, J = 8.3 Hz, 2H), 7.44 (d, J = 15.9 Hz, 1H), 7.03 (s, 1H), 3.29 (s, 3H). MS(ESI): m/z Calcd. For $C_{20}H_{15}N_4O_3$ 359.1; found 359.1, $[M+H]^+$.

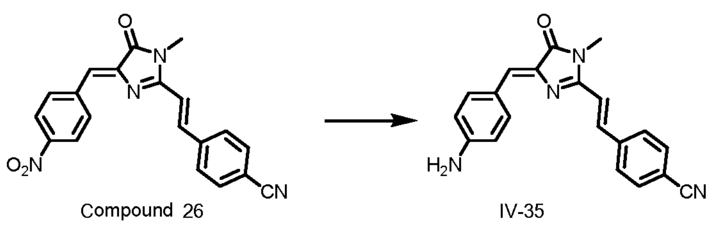

Compound 26 IV-35

To a stirred solution of compound 26 (0.718 g, 2.0 mmol) in 100 mL ethyl acetate, Anhydrous stannous chloride (0.758 g, 4.0 mmol) was added. The complete mixture was heated to reflux under Ar atmosphere. The progress of reaction was monitored on silica gel TLC. After completion of reaction, the reaction was poured into 150 mL water, and extracted with ethyl acetate for three times. The organic layer was dried over anhydrous $MgSO_4$, filtered, and concentrated under reduced pressure to give the crude product, then purified by silica gel column chromatography to afford a target compound (0.586 g, 89%).$^1$H NMR (400 MHz, $CDCl_3$) δ 8.19 (d, J = 8.5 Hz, 2H), 7.97 (m, 4H), 8.04 (s, 1H), 7.94 (d, J = 8.3 Hz, 2H), 7.44 (d, J = 15.9 Hz, 1H), 7.03 (s, 1H), 3.29 (s, 3H). MS(ESI): m/z Calcd. For $C_{20}H_{17}N_4O$ 329.1402; found 329.1403, $[M+H]^+$.

Compound Ⅳ-36:

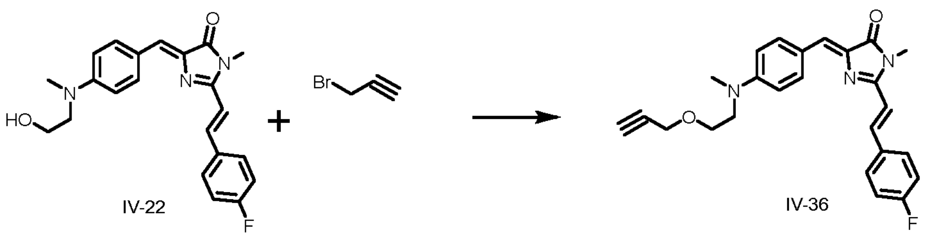
IV-22
IV-36

This compound was obtained by following the general procedure for Compound 18, ( 0.322g, 79%) 。$^1$H-NMR(400 MHz, DMSO-$d_6$): δ 8.16 (d, *J* = 8.5 Hz, 2H), 7.93 (t, *J* = 4.4 Hz, 2H), 7.90 (d, *J* = 6.1 Hz, 1H), 7.31 (t, *J* = 8.9 Hz, 2H), 7.18 (d, *J* = 15.9 Hz, 1H), 6.95 (s, 1H), 6.83-6.72 (m, 2H),4.21(s,2 H), 3.59 (t, *J* = 5.9 Hz, 2H), 3.51 (t, *J* = 5.9 Hz, 2H), 3.27 (s, 3H), 3.05 (s, 3H). MS(ESI): m/z Calcd. For $C_{25}H_{25}FN_3O_2$ 418.1931; found 418.1932, $[M+H]^+$.

Compound Ⅳ-37 :

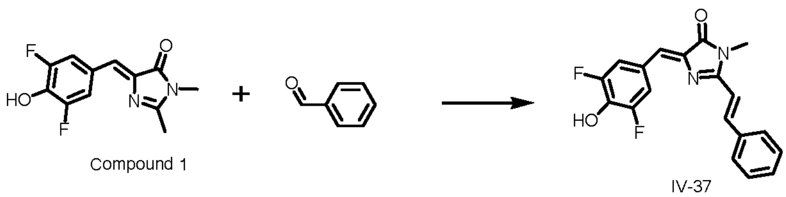
Compound 1
IV-37

This compound was obtained by following the general procedure for Compound IV-1, (0.275 g, 75%) .$^1$H NMR (400 MHz, DMSO-$d_6$) δ 11.00 (s, 1H), 8.08-8.04 (m, 2H), 8.02 (d, *J* = 15.9 Hz, 1H), 7.92-7.87 (m, 2H), 7.49-7.45 (m, 2H), 7.26 (d, *J* = 15.9 Hz, 1H), 6.98 (s, 1H), 3.29 (s, 3H). MS(ESI): m/z Calcd. For $C_{19}H_{13}F_2N_3O_2$ 339.0951; found 339.0950, $[M-H]^-$.

Compound Ⅳ-38 :

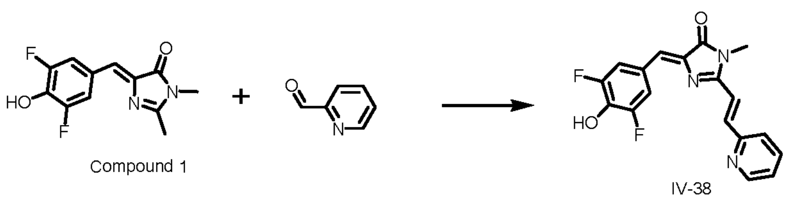
Compound 1
IV-38

This compound was obtained by following the general procedure for Compound IV-1, (0.327 g, 55%) .$^1$H NMR (400 MHz, DMSO-$d_6$) δ 9.70 (s, 1H), 8.05 (d, J = 8.9 Hz, 2H), 7.98 - 7.89 (m, 1H), 7.30 (t, J = 6.2 Hz, 1H), 7.26 (d, J = 8.9 Hz, 1H), 7.15 (d, J = 15.8 Hz, 1H), 6.97 (s, 1H), 6.87 (d, J = 7.5 Hz, 1H), 3.27 (s, 3H). MS(ESI): m/z Calcd. For $C_{18}H_{12}F_2N_3O_2$ 340.0903; found 340.090, $[M-H]^-$.

Compound Ⅳ-39 :

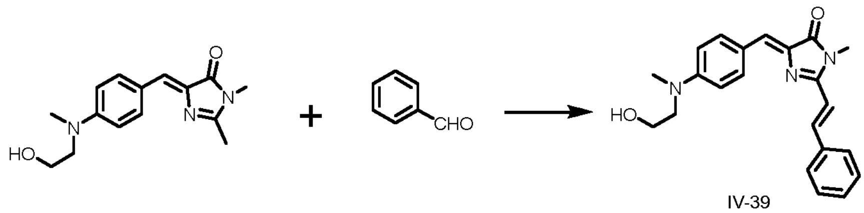

IV-39

This compound was obtained by following the general procedure for Compound IV-1, (0.342 g, 46%) 。$^1$H-NMR(400 MHz,DMSO-$d_6$): δ=8.21(d,2H,J=8.8Hz),8.00(d,1 H,J=16 Hz),7.85(d,2 H,J=8.0 Hz), 7.50-7.43 (m, 2H), 7.42 (d, *J* = 2.6 Hz, 1H),7.24(s,1 H),7.01(s,1 H),6.92(d,2 H,J=8.8 Hz),3.85 (t,2H,J=5.6 Hz),3.60(t,2 H,J=5.6 Hz),3.10(s,3 H). MS(ESI): m/z Calcd. For $C_{22}H_{24}N_3O_2$ 362.1869; found 362.1868, [M+H]$^+$.

Compound Ⅳ-40 :

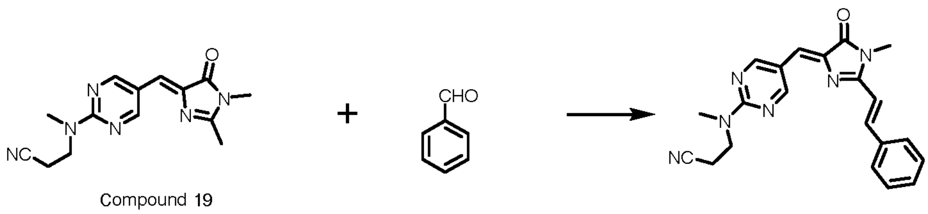

Compound 19

IV-40

This compound was obtained by following the general procedure for Compound IV-1, (0.412 g, 46%) 。$^1$H-NMR(400 MHz,DMSO-$d_6$): δ=8.72 (s, 2H), 8.00(d,1 H,J=16 Hz), 7.50-7.43 (m, 2H), 7.42 (d, J = 2.6 Hz, 1H),7.24(s,1 H),7.01(s,1 H),6.92(d,2 H,J=8.8 Hz),3.75 (t, J= 6.8Hz, 2H), 3.60(t,2 H,J=6.8 Hz),3.05(s,3 H). MS(ESI): m/z Calcd. For $C_{21}H_{21}IN_6O$ 373.1777; found 373.1778, [M+H]$^+$.

It should be understood that the amounts, reaction conditions, etc. in the various embodiments of this specification are approximate unless otherwise noted, and may be changed slightly to obtain similar results depending on the circumstances. Except as specifically defined, all professional and scientific terms used herein have the same meaning as understood by those skilled in the art. All literature referred to herein is introduced into this application as a reference. This specification describes a preferred embodiment for exemplary purposes, and a person skilled in the art may use methods and materials similar to those described herein to implement the invention to obtain the same or similar results, and various changes or modifications to the invention remain within the limits of the claims appended to this application.

## Claims

1. A nucleic acid aptamer molecule comprising the following nucleotide sequences (a), (b) or (c):

   (a): a nucleotide sequence $N_1$AGAUUGUAAACAN$_{14}$-N$_{15}$-N$_{16}$GACACUN$_{23}$, wherein $N_1$, $N_{14}$, $N_{15}$, $N_{16}$ and $N_{23}$ represent nucleotide fragments greater than or equal to 1 in length, at least one base pair in $N_1$ and $N_{23}$ nucleotide sequences forms a complementary pair, and at least one base pair in $N_{14}$ and $N_{16}$ nucleotide sequences forms a complementary pair;
   (b): a nucleotide sequence with an identity of at least 70%, 72%, 77%, 83%, 88%, 94% or 100% to the nucleotide sequence (a); and
   (c): a nucleic acid aptamer molecule derived from the nucleotide sequence (a) at a position other than $N_1$, $N_{14}$, $N_{15}$, $N_{16}$ and $N_{23}$ in the nucleotide sequence (a), with substitution, missing and/or addition of one or several nucleotides, and having an aptamer function.

2. The nucleic acid aptamer molecule according to claim 1, wherein the nucleotide sequence (c) is nucleic acid aptamer molecules obtained with substitution, missing and/or addition of 5, 4, 3, 2 or 1 nucleotide at a position other than $N_1$, $N_{14}$, $N_{15}$, $N_{16}$ and $N_{23}$ in the nucleotide sequence (a).

**3.** The nucleic acid aptamer molecule according to claim 1, wherein: when $N_1$ and $N_{23}$ in the nucleotide sequence (a) form a complementary pair(s), the direction of $N_1$ nucleotide sequence is 5'-3', and the direction of $N_{23}$ nucleotide sequence is 3'-5'; when $N_{14}$ and $N_{16}$ form a complementary pair(s), the direction of $N_{14}$ nucleotide sequence is 5'-3', and the direction of $N_{16}$ nucleotide sequence is 3'-5'.

**4.** The nucleic acid aptamer molecule according to claim 3, wherein: when at least one fragment of $N_1$ and $N_{23}$ is greater than or equal to 5 nucleotide bases in length, at least two pairs of nucleotide bases in $N_1$ and $N_{23}$ nucleotide sequences form complementary pairs; when at least one fragment of $N_{14}$ and $N_{16}$ is greater than or equal to 5 nucleotide bases in length, at least two pairs of bases in $N_{14}$ and $N_{16}$ nucleotide sequences form complementary pairs.

**5.** The nucleic acid aptamer molecule according to claim 1, wherein the nucleotide substitution in the nucleotide sequence (a) is selected from one of the following groups: A4U, A4G, A4C, USA, U5G, U5C, G7C, G7A, G7U, U8C, A10U, A10G, A10C, A11U, A11G, A11C, C12G, C12A, C12U, A13U, A13G, A13C, G17A, C19A, C19U, A20C, A4C/USA, A4C/USC, A4C/A11G, A4C/C12A, A4C/A13C, U5A/A11G, U5A/C12A, U5A/A13C, U5G/A13C, U5C/G7U, U5C/A11G, U5C/C12G, U5C/C12A, U5C/C12U, U5C/A13C, G7U/A13C, A11G/A13C, C12G/A13C, C12A/A13C, C12U/A13C, A4C/U5C/A13C, U5C/G7U/A13C, U5C/C12G/A13C, U5C/C12U/A13C, U5C/A11G/A13C, U5C/C12A/A13C, A4C/U5C/A11G/A13C, A4C/U5C/C12A/A13C, A4C/U5C/G7U/A11G/A13C, A4C/U5C/G7U/C12A/A13C, A4C/U5A/A11G/C12A/A13C, A4C/U5C/A11G/C12A/A13C$_o$

**6.** The nucleic acid aptamer molecule according to claim 1, wherein the nucleotide sequences at $N_1$ and $N_{23}$ in the nucleotide sequence (a) are F30 or tRNA scaffold RNA sequences.

**7.** The nucleic acid aptamer molecule according to claim 1, wherein the aptamer molecules are RNA molecules or base-modified RNA molecules.

**8.** The nucleic acid aptamer molecule according to claim 1, wherein the aptamer molecules are DNA-RNA hybrid molecules or base-modified DNA-RNA molecules.

**9.** The nucleic acid aptamer molecule according to claim 1, wherein $N_{14}$-$N_{15}$-$N_{16}$ therein contains a nucleotide sequence capable of identifying target molecules.

**10.** The nucleic acid aptamer molecule according to claim 9, wherein the target molecules include but are not limited to: proteins, nucleic acid, lipid molecules, carbohydrates, hormones, cytokines, chemokines, metabolite and metal ions.

**11.** The nucleic acid aptamer molecule according to claim 9, wherein the $N_{14}$-$N_{15}$-$N_{16}$ is a nucleotide sequence capable of identifying S-ademetionine and adenosine molecules.

**12.** The nucleic acid aptamer molecule according to claim 1, wherein the aptamer function refers to that the nucleic acid aptamer can enhance fluorescence intensity of fluorophore molecules under light excitation at suitable wavelength by at least two times, by at least 5 to 10 times, by at least 20 to 50 times, by at least 100 to 200 times or by at least 200 times.

**13.** The nucleic acid aptamer molecule according to claim 1, wherein: the nucleic acid aptamer molecule may further include concatemers that can bind to multiple fluorophore molecules, the concatemers are connected by a spacer sequence(s) of suitable length, and the number may be 2, 3, 4, 5, 6, 7, 8 or more; the nucleotide of the concatemer(s) is selected from sequences SEQ ID No: 7, 8, 9, 10, 11, 12, 13, 14, 15, 16, 17, 18, 19 and 20.

**14.** The nucleic acid aptamer molecule according to claim 1, wherein the nucleic acid aptamer molecule has a sequence selected from SEQ ID No: 1, 2, 3, 4, 5, 6, 7, 8, 9, 10, 11, 12, 13, 14, 15, 16, 17, 18, 19, 20, 22, 24, 25, 26, 27, 28, 29, 30 or 31.

**15.** A complex of nucleic acid aptamer molecules and fluorophore molecules, wherein the nucleic acid aptamer molecule(s) is the nucleic acid aptamer molecule(s) according to claim 1, and the fluorophore molecule(s) has a structure shown in Formula (I) below:

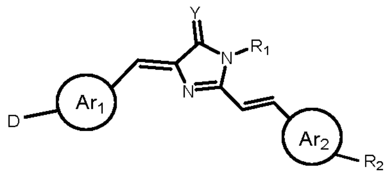 (I)

wherein: $Ar_1$, and $Ar_2$ are independently hexahydric aryl group or hexahydric heteroaryl group; D- is HO- or $N(X_1)(X_2)$-, and $X_1$ and $X_2$ are independently selected from hydrogen, alkyl and modified alkyl; $X_1$ and $X_2$ are optionally interconnected and form an alicyclic heterocycle with N atom; when D- is $N(X_1)(X_2)$- and $Ar_1$ is phenyl group, $X_1$ and $X_2$ independently form a saturated or unsaturated alicyclic heterocycle with the benzene ring; when D- is HO- and $Ar_1$ is phenyl group, at least one hydrogen atom adjacent to HO- is substituted by halogen; Y is O or S; $R_1$ is hydrogen, alkyl or modified alkyl; $R_2$ is a hydrogen atom, a halogen atom, -OH, or -CN; and the alkyl is independently $C_1$-$C_{10}$ straight or branched alkyl;

the modified alkyl is a group obtained by replacing any carbon atom of the alkyl with one or more groups selected from halogen atoms, -OH, -CO-, -O-, -CN, $-SO_3H$, primary amino group, secondary amino group, and tertiary amino group, and the modified alkyl has 1 to 10 carbon atoms, wherein the carbon-carbon single bond is optionally and independently replaced by a carbon-carbon double bond or a carbon-carbon triple bond; the replacement of carbon atoms refers to that carbon atoms are replaced with corresponding group or that carbon atoms, together with hydrogen atoms thereon, are replaced with corresponding group.

**16.** The complex according to claim 15, wherein the fluorophore molecule is selected from compounds below:

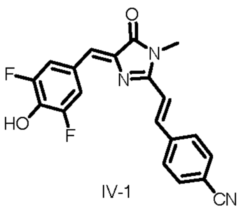 IV-1 ,
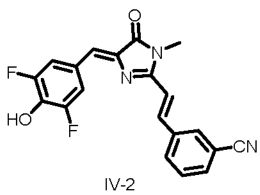 IV-2 ,
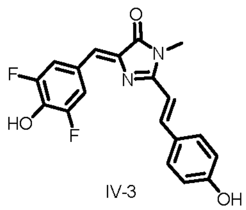 IV-3 ,
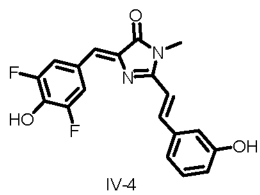 IV-4 ,

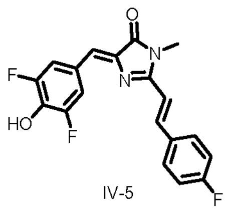 IV-5 ,
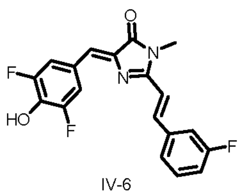 IV-6 ,
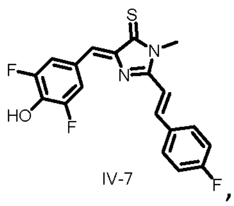 IV-7 ,
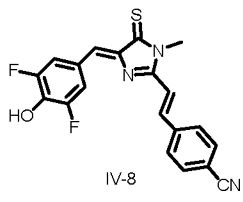 IV-8 ,

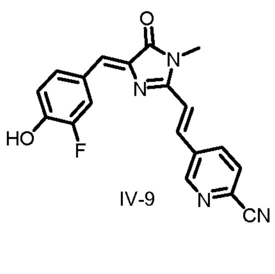 IV-9 ,
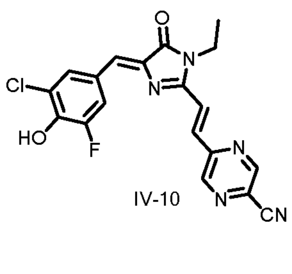 IV-10 ,
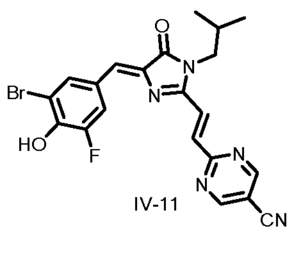 IV-11 ,
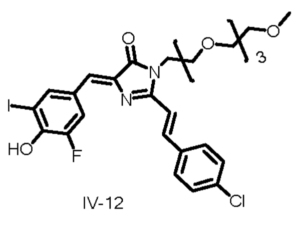 IV-12 ,

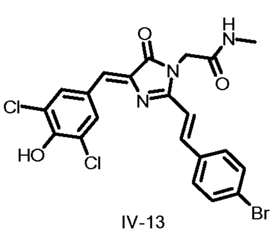 IV-13 ,
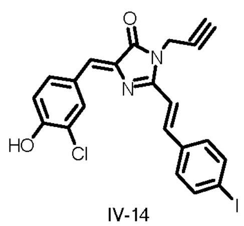 IV-14 ,
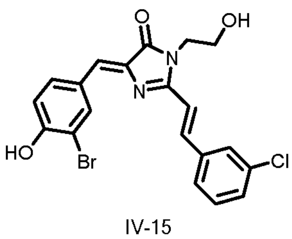 IV-15 ,
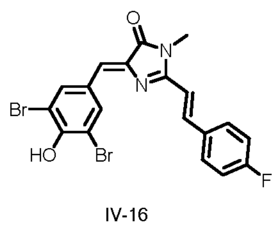 IV-16 ,

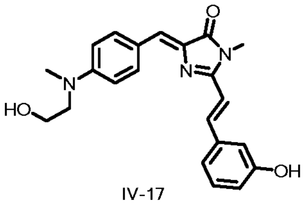
IV-17 ,

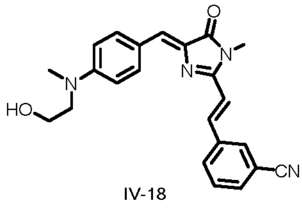
IV-18 ,

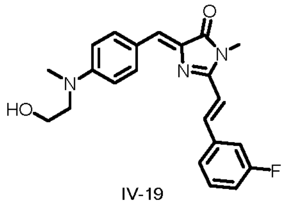
IV-19 ,

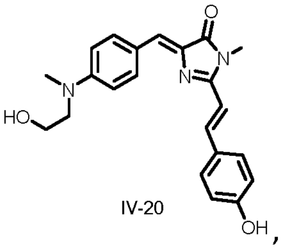
IV-20 ,

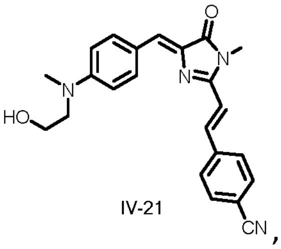
IV-21 ,

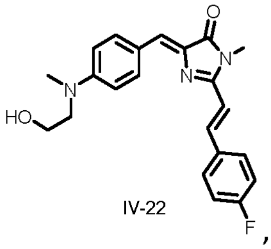
IV-22 ,

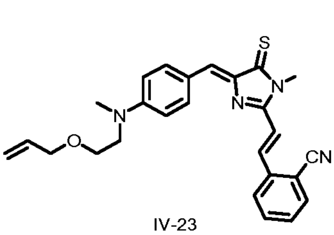
IV-23 ,

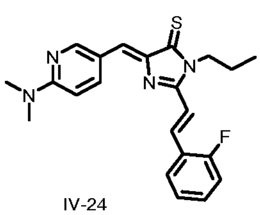
IV-24 ,

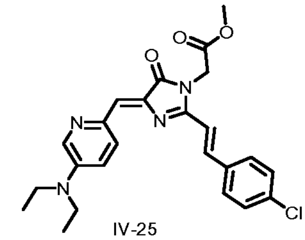
IV-25 ,

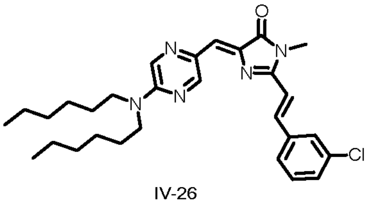
IV-26 ,

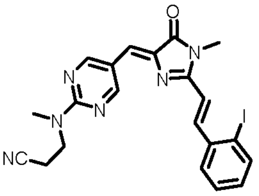
IV-27 ,

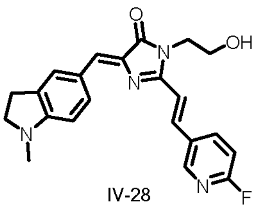
IV-28 ,

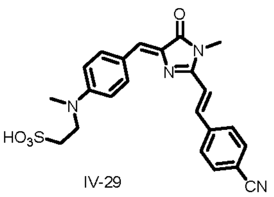
IV-29 ,

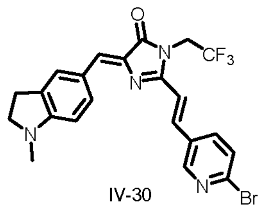
IV-30 ,

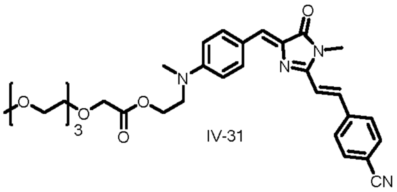
IV-31 ,

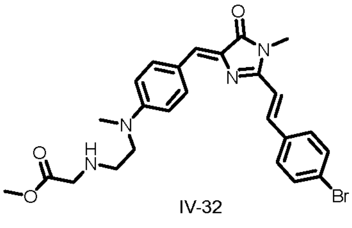
IV-32 ,

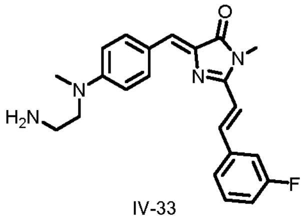
IV-33 ,

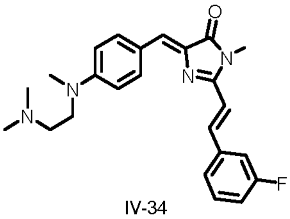
IV-34 ,

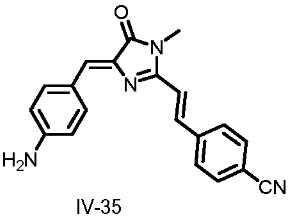
IV-35 ,

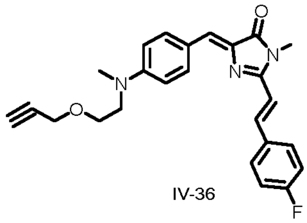
IV-36 ,

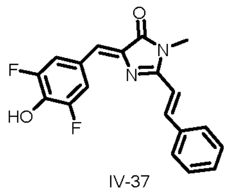
IV-37 ,

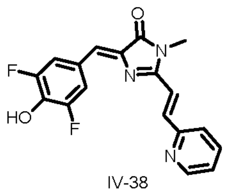
IV-38 ,
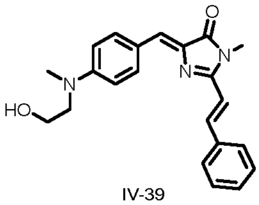
IV-39 ,
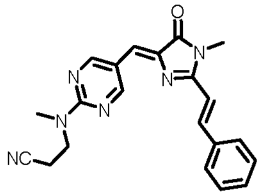
IV-40 .

**17.** The complex according to claim 15, wherein the aptamer molecules in the complex contain nucleotide sequence SEQ ID No: 1, 2, 3, 4, 5, 6, 7, 8, 9, 10, 11, 12, 13, 14, 15, 16, 17, 18, 19, 20, 22, 24, 25, 26, 27, 28, 29, 30 or 31.

**18.** A kit containing at least one of: the nucleic acid aptamer molecule according to claim 1, the complex according to claim 15, expression vectors or host cells, wherein:

the expression vectors contain DNA molecules which transcribes the nucleic acid aptamer molecule according to claim 1; and
the host cells contain the expression vectors.

**19.** Use of the complex according to claim 15 for in vivo or in vitro detection or labeling of target nucleic acid molecules, detection or labeling of intracellular or extracellular target molecules, imaging of genome DNA, detection of interaction between RNA and protein, detection of genome DNA, as well as RNA extraction and purification.

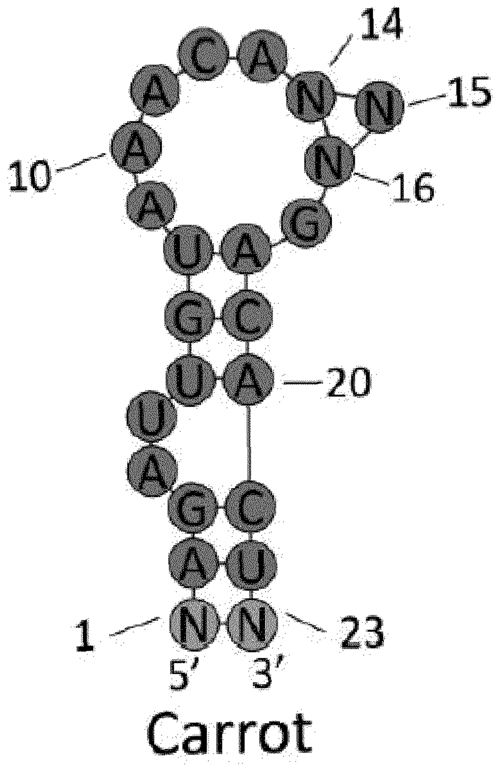
14
15
10
16
20
1
23
5'
3'
Carrot

Fig.1

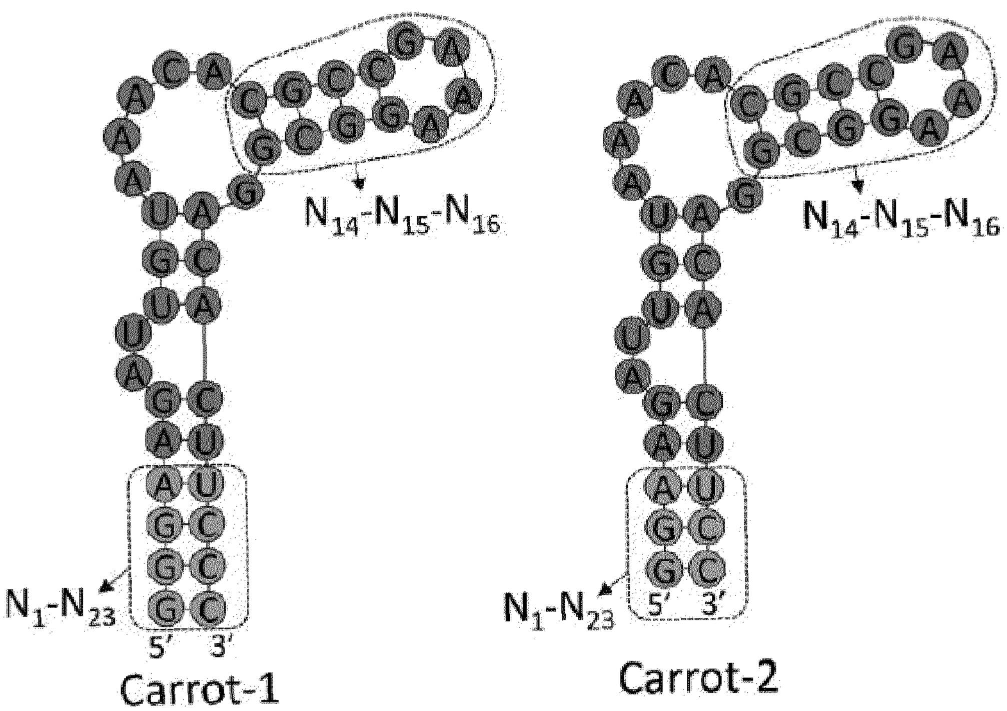
$N_{14}$-$N_{15}$-$N_{16}$
$N_1$-$N_{23}$
5'
3'
Carrot-1
$N_{14}$-$N_{15}$-$N_{16}$
$N_1$-$N_{23}$
5'
3'
Carrot-2

Fig.2

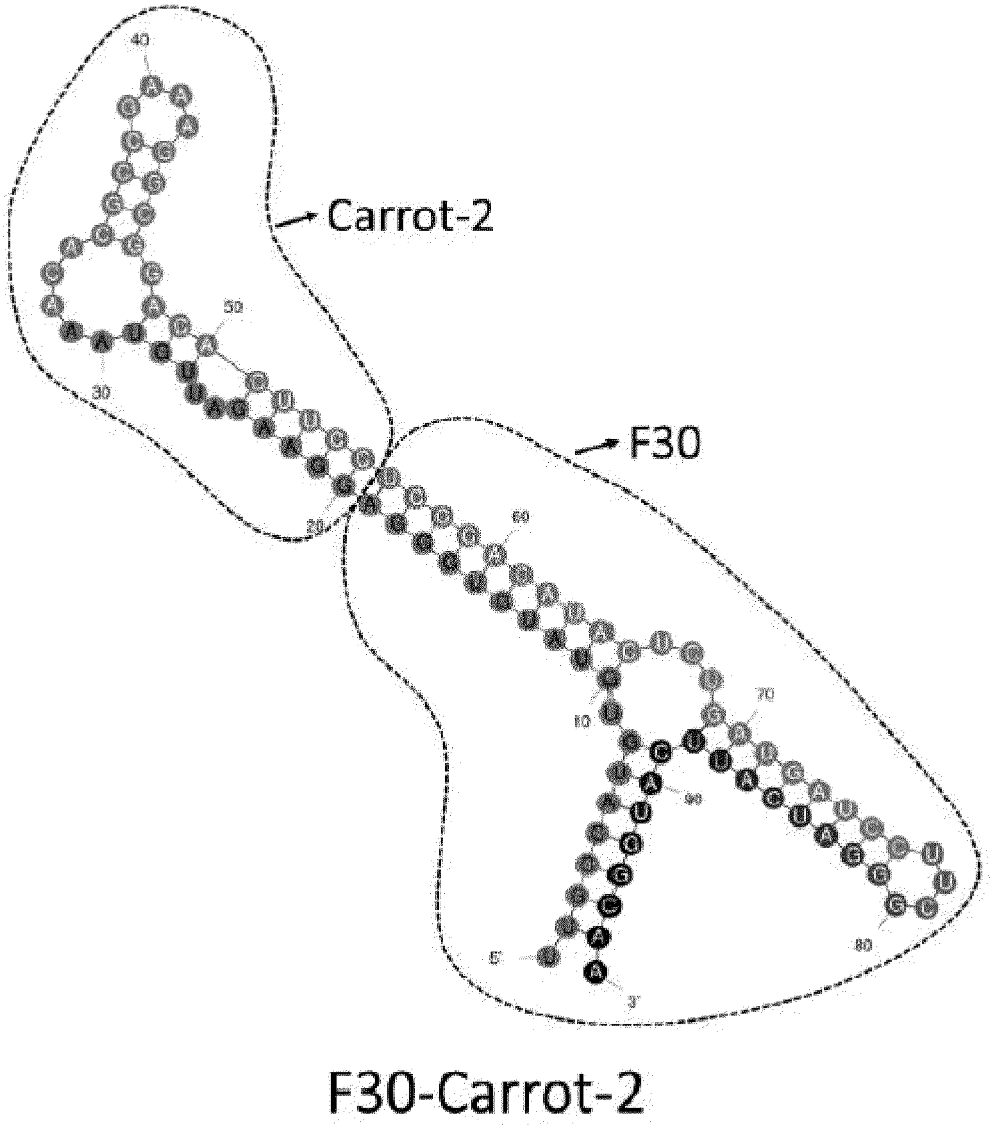


Fig. 3

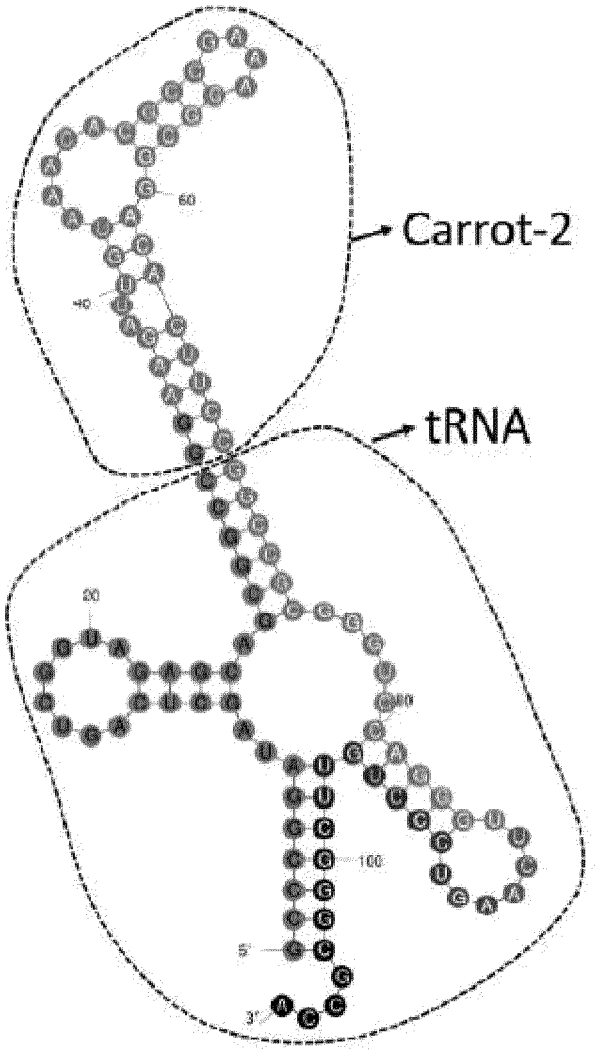


tRNA-Carrot-2

Fig. 4

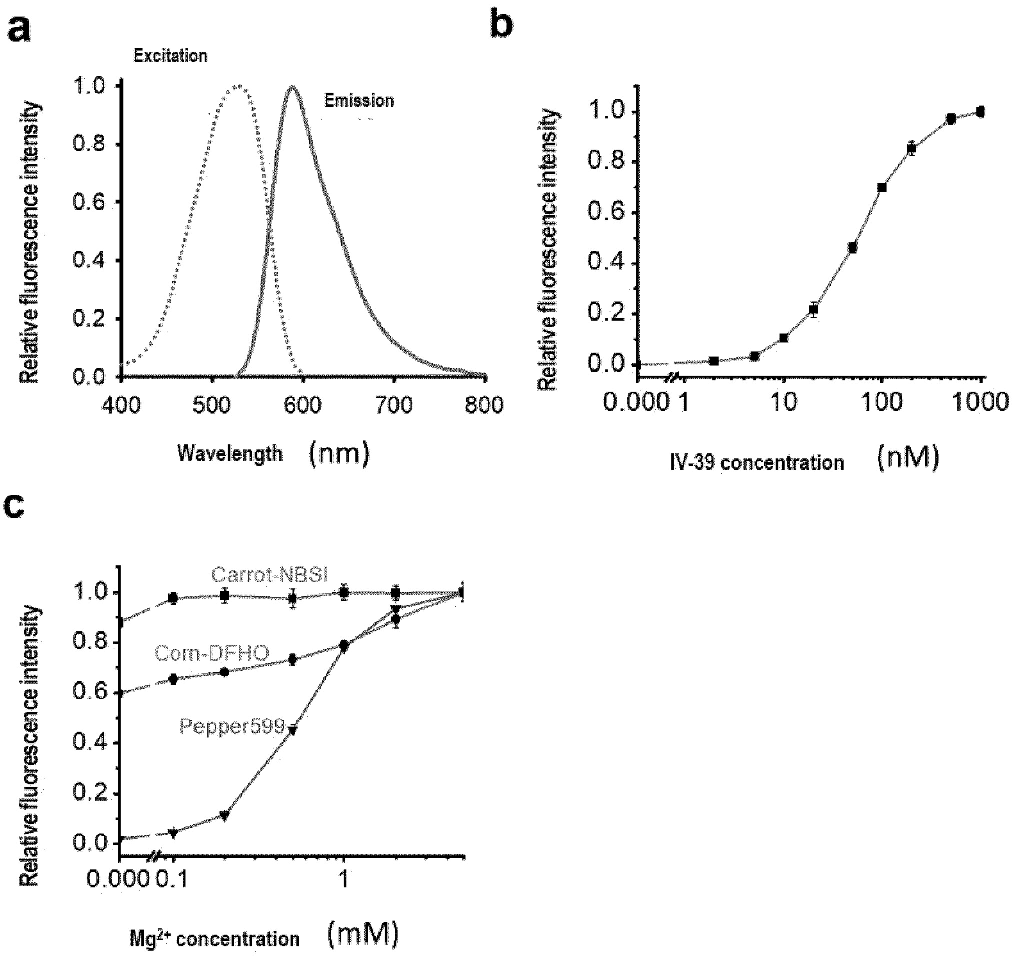
a
Excitation
Emission
Relative fluorescence intensity
1.0
0.8
0.6
0.4
0.2
0.0
400
500
600
700
800
Wavelength (nm)
b
Relative fluorescence intensity
1.0
0.8
0.6
0.4
0.2
0.0
0.000 1
10
100
1000
IV-39 concentration (nM)
c
Carrot-NBSI
Corn-DFHO
Pepper599
Relative fluorescence intensity
1.0
0.8
0.6
0.4
0.2
0.0
0.000 0.1
1
Mg2+ concentration (mM)

Fig. 5

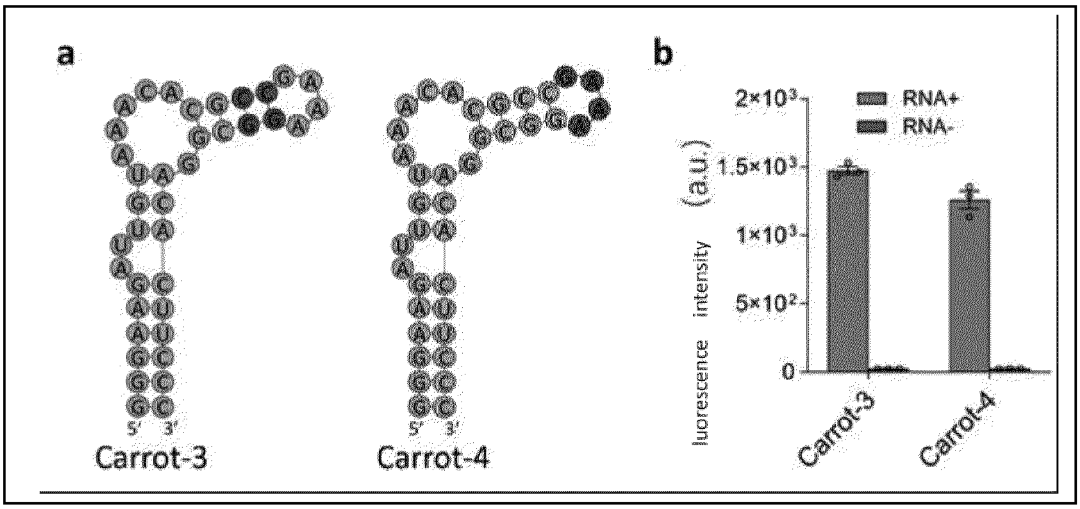
a
Carrot-3
Carrot-4
5' 3'
b
2×10³
1.5×10³
1×10³
5×10²
0
RNA+
RNA-
luorescence intensity (a.u.)
Carrot-3
Carrot-4

Fig. 6

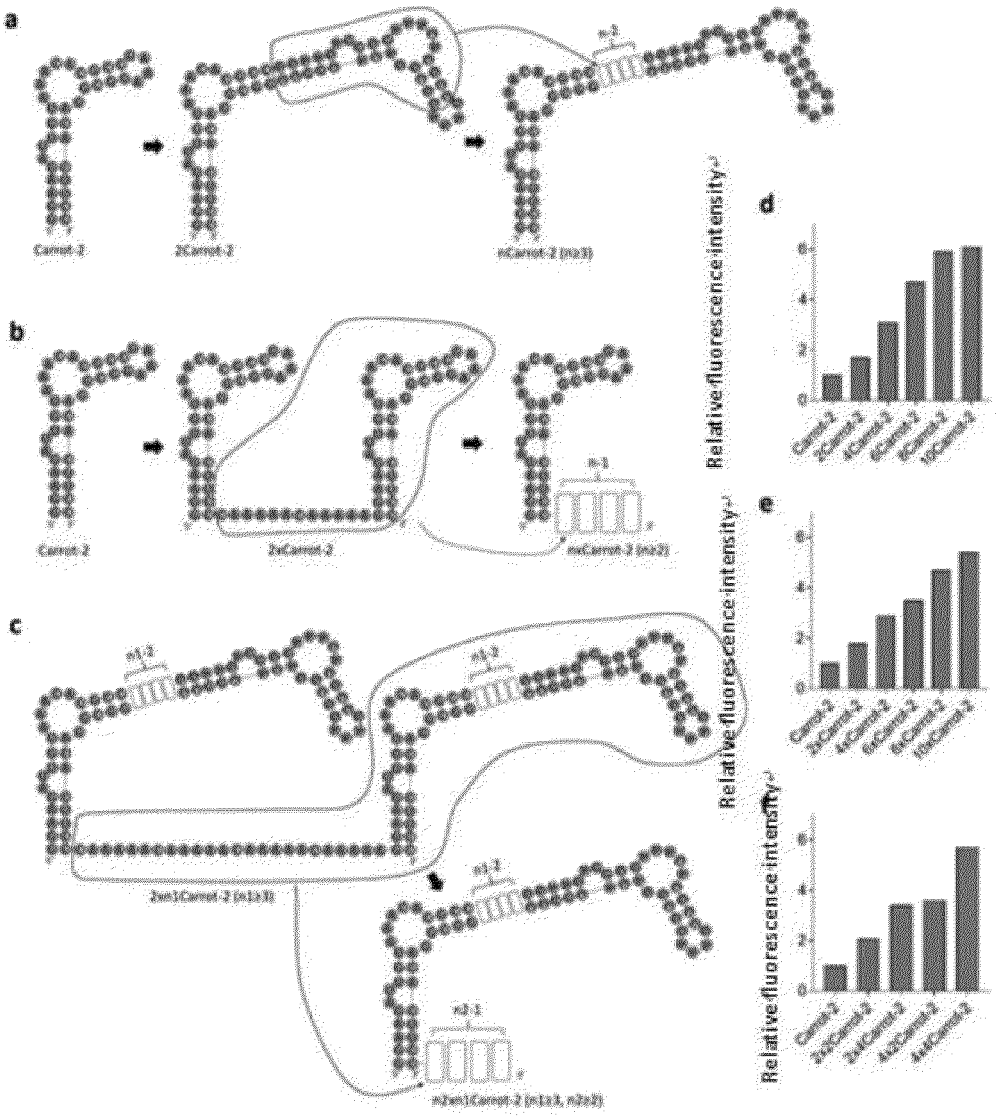
a
b
c
d
e
f
Carrot-2
2Carrot-2
nCarrot-2 (n≥3)
2xCarrot-2
nxCarrot-2 (n≥2)
2xn1Carrot-2 (n1≥3)
n2xn1Carrot-2 (n1≥3, n2≥2)
Relative fluorescence intensity


Fig. 7

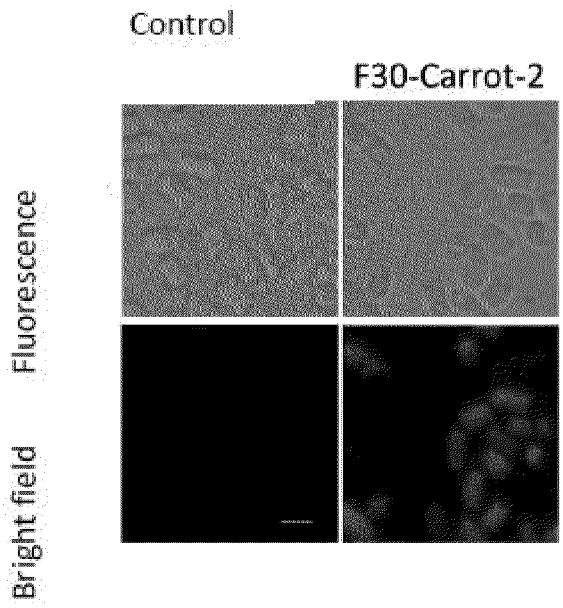
Control
F30-Carrot-2
Fluorescence
Bright field

Fig. 8

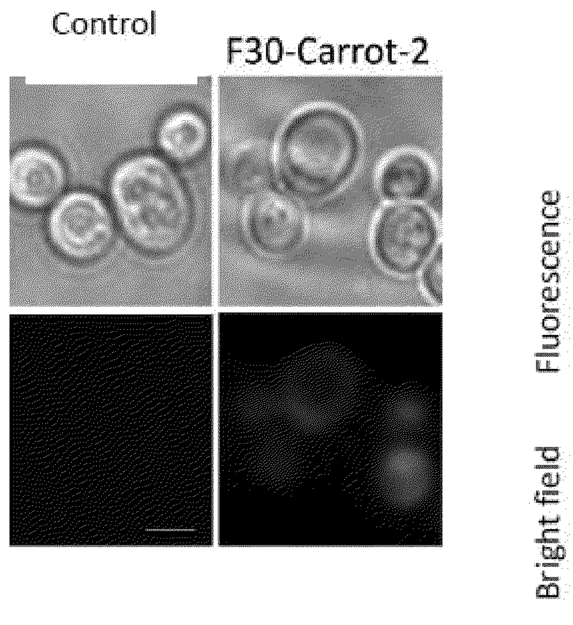
Control
F30-Carrot-2
Fluorescence
Bright field

Fig. 9

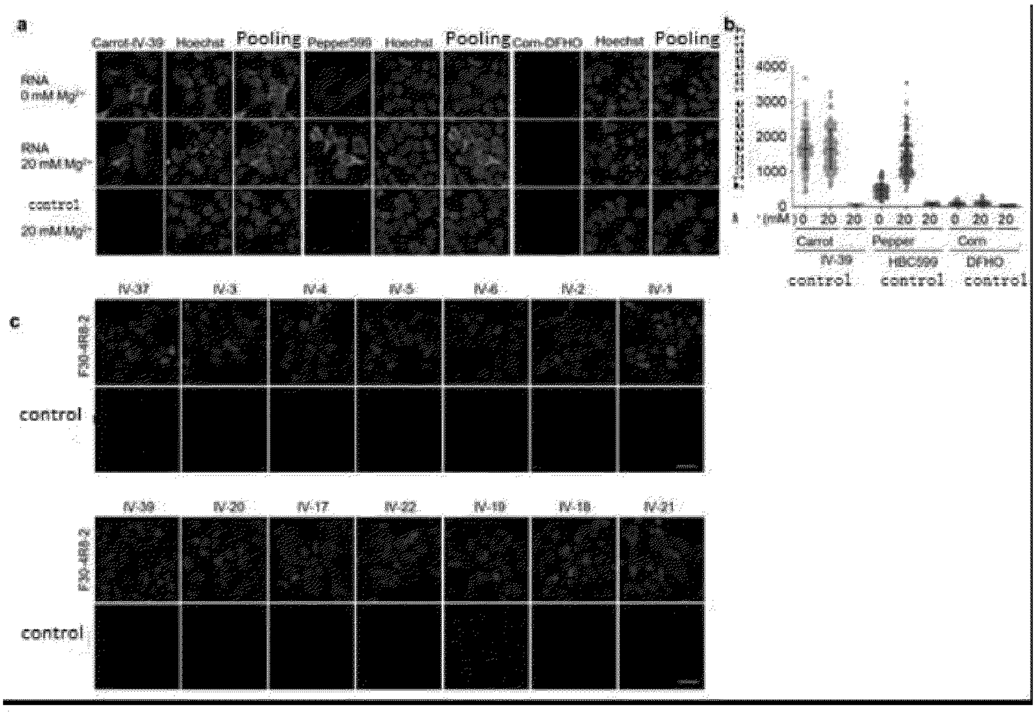
a
Carrot-IV-39
Hoechst
Pooling
Pepper599
Hoechst
Pooling
Corn-DFHO
Hoechst
Pooling
RNA
0 mM Mg2+
RNA
20 mM Mg2+
control
20 mM Mg2+
b
Fluorescence intensity
4000
3000
2000
1000
0
(mM) 0 20 20 0 20 20 0 20 20
Carrot
IV-39
control
Pepper
HBC599
control
Corn
DFHO
control
c
IV-37
IV-3
IV-4
IV-5
IV-6
IV-2
IV-1
F30-4R8-2
control
IV-39
IV-20
IV-17
IV-22
IV-19
IV-18
IV-21
F30-4R8-2
control

Fig. 10

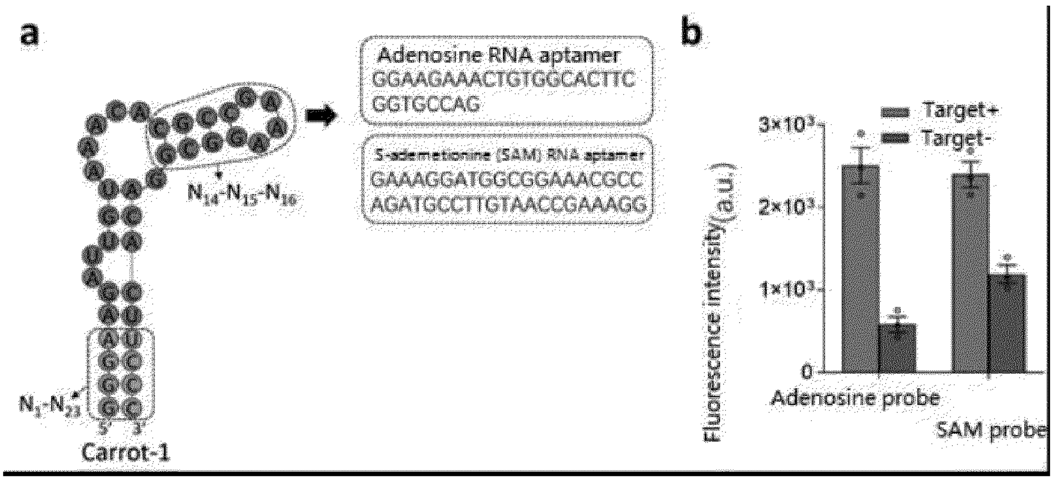
a
Adenosine RNA aptamer
GGAAGAAACTGTGGCACTTC
GGTGCCAG
S-ademetionine (SAM) RNA aptamer
GAAAGGATGGCGGAAACGCC
AGATGCCTTGTAACCGAAAGG
N14-N15-N16
N1-N23
5'
3'
Carrot-1
b
Target+
Target-
3×10³
2×10³
1×10³
0
Fluorescence intensity(a.u.)
Adenosine probe
SAM probe

Fig. 11

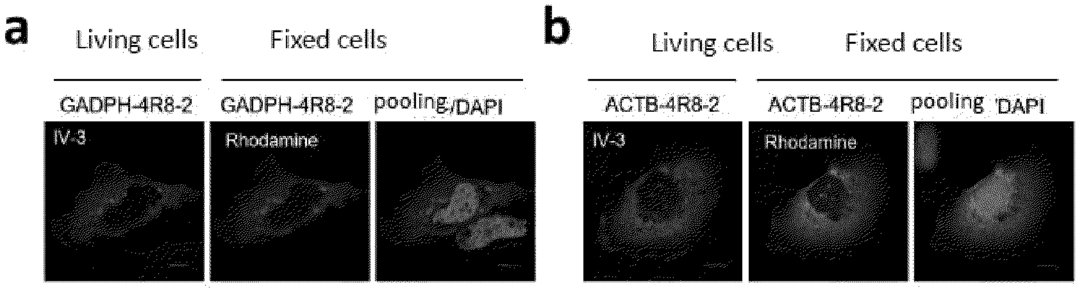
a
Living cells
Fixed cells
GADPH-4R8-2
GADPH-4R8-2
pooling/DAPI
IV-3
Rhodamine
b
Living cells
Fixed cells
ACTB-4R8-2
ACTB-4R8-2
pooling DAPI
IV-3
Rhodamine

Fig. 12

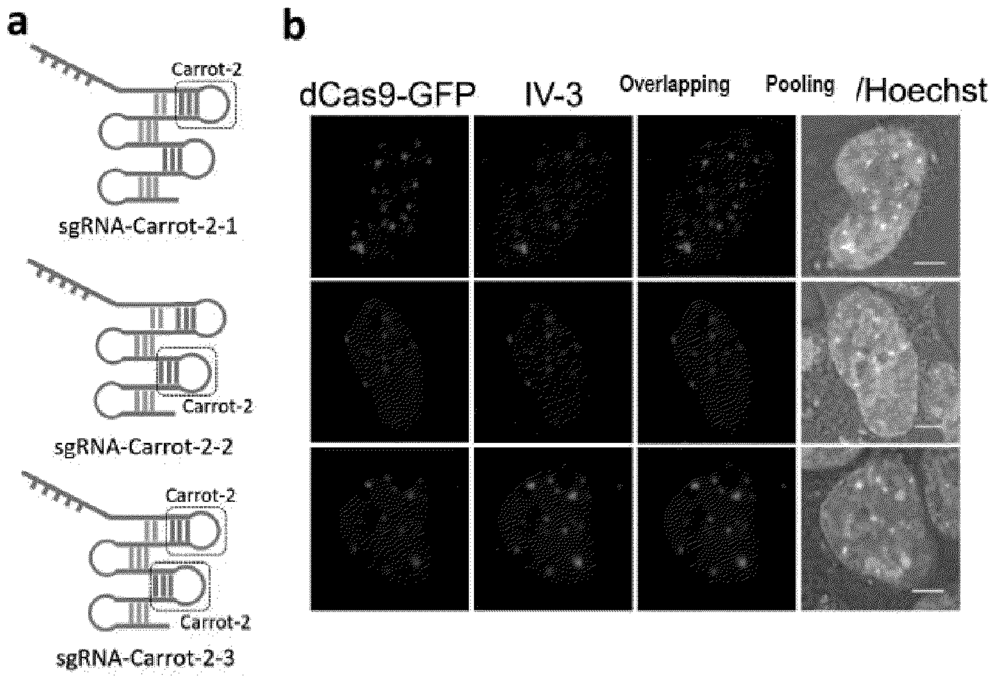
a
Carrot-2
sgRNA-Carrot-2-1
Carrot-2
sgRNA-Carrot-2-2
Carrot-2
Carrot-2
sgRNA-Carrot-2-3
b
dCas9-GFP
IV-3
Overlapping
Pooling
/Hoechst

Fig. 13

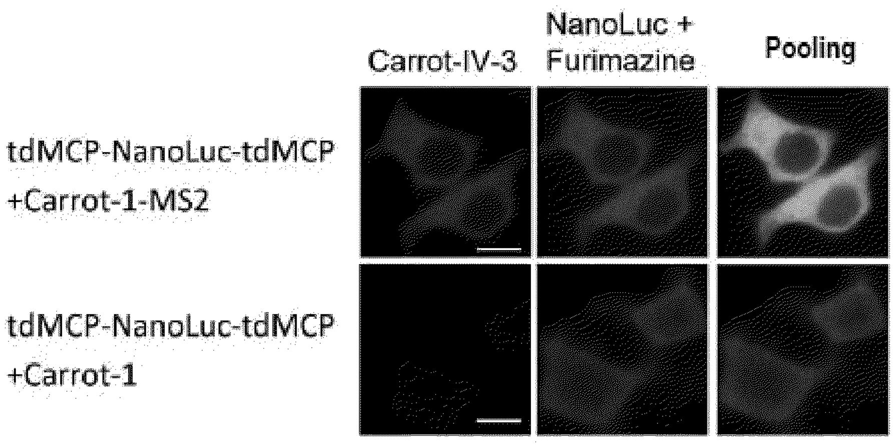
Carrot-IV-3
NanoLuc + Furimazine
Pooling
tdMCP-NanoLuc-tdMCP +Carrot-1-MS2
tdMCP-NanoLuc-tdMCP +Carrot-1

Fig. 14

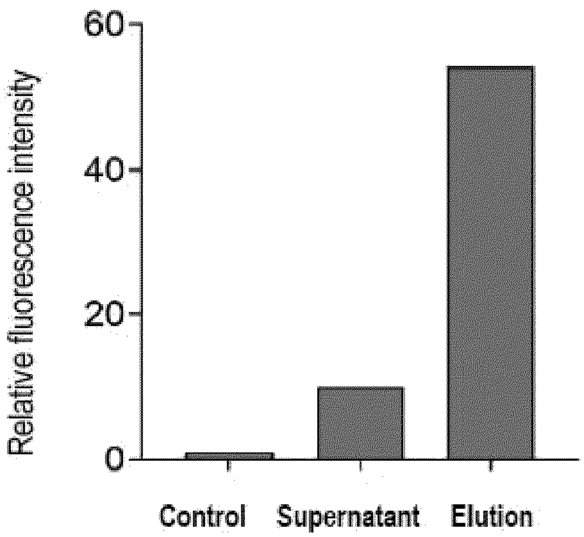
60
40
20
0
Relative fluorescence intensity
Control
Supernatant
Elution

Fig. 15

# INTERNATIONAL SEARCH REPORT

International application No.
**PCT/CN2021/083579**

**A. CLASSIFICATION OF SUBJECT MATTER**

C12N 15/115(2010.01)i; C12Q 1/04(2006.01)i; C12Q 1/68(2018.01)i; C12N 15/10(2006.01)i

According to International Patent Classification (IPC) or to both national classification and IPC

**B. FIELDS SEARCHED**

Minimum documentation searched (classification system followed by classification symbols)

C12N; C12Q

Documentation searched other than minimum documentation to the extent that such documents are included in the fields searched

Electronic data base consulted during the international search (name of data base and, where practicable, search terms used)

Database: CNABS, VEN(DWPI+SIP0ABS), CNTXT, EPTXT, USTXT, WOTXT, CNKI, PubMed, Elsevier Science, ISI-WEB OF SCIENCE, Genbank, EMBL, 中国专利生物序列检索系统, CHINESE PATENT BIOLOGICAL SEQUENCE RETRIEVAL SYSTEM; Search Terms: 核酸, 适配体, 检测, 定量, 荧光, 复合物, DNA, RNA, aptamer, quant+, detect+, fluorescen+, complex, SEQ ID NO.1-20, 22, 24-31序列比对, SEQ ID NO.1-20, 22, 24-31 sequence alignment

**C. DOCUMENTS CONSIDERED TO BE RELEVANT**

| Category* | Citation of document, with indication, where appropriate, of the relevant passages | Relevant to claim No. |
|---|---|---|
| A | CN 103205431 A (HUNAN UNIVERSITY) 17 July 2013 (2013-07-17)<br>see entire document | 1-19 |
| A | CN 104726548 A (SHENZHEN INSTITUTES OF ADVANCED TECHNOLOGY, CHINESE ACADEMY OF SCIENCES) 24 June 2015 (2015-06-24)<br>see entire document | 1-19 |
| A | CN 109682973 A (INSTITUTE OF CHEMISTRY, CHINESE ACADEMY OF SCIENCES) 26 April 2019 (2019-04-26)<br>see entire document | 1-19 |
| A | US 2009004667 A1 (SOMALOGIC INC.) 01 January 2009 (2009-01-01)<br>see entire document | 1-19 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 June 2021** | **08 July 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/083579**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.
**PCT/CN2021/083579**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **19**
because they relate to subject matter not required to be searched by this Authority, namely:

[1] The subject matter of claim 19 relates to a method of treatment of a human or animal body. A search on claim 19 is thus made on the basis of anticipating the subject matter of claim 19 as "a complex of claim 15 for preparing a product for the detection or labeling of target nucleic acid molecules in vitro or in vivo, the detection or labeling of extracellular or intracellular target molecules, imaging genomic DNA, detecting RNA-protein interactions, detecting genomic DNA and products for the refinement and purification of RNA."

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/083579**

| Patent document cited in search report | Publication date (day/month/year) | Patent family member(s) | Publication date (day/month/year) |
|---|---|---|---|
| CN 103205431 A | 17 July 2013 | CN 103205431 B | 29 April 2015 |
| CN 104726548 A | 24 June 2015 | None | |
| CN 109682973 A | 26 April 2019 | None | |
| US 2009004667 A1 | 01 January 2009 | US 2018155725 A1 | 07 June 2018 |
| | | US 7947447 B2 | 24 May 2011 |
| | | US 2017137819 A1 | 18 May 2017 |
| | | US 2015197753 A1 | 16 July 2015 |
| | | US 2019330634 A1 | 31 October 2019 |
| | | US 2011245479 A1 | 06 October 2011 |
| | | US 10316321 B2 | 11 June 2019 |
| | | US 2019264208 A1 | 29 August 2019 |
| | | US 8975388 B2 | 10 March 2015 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **RAJ et al.** *Nature Methods,* 2018, vol. 5, 877-879
- **CHEN et al.** *Nucleic Acids Res,* 2010, vol. 38, e148
- **YOU et al.** *Ann. Rev. Biophys,* 2015, vol. 44, 187-206
- **BERTRAND et al.** *Molecular Cell,* 1998, vol. 2, 437-445
- **DAIGLE et al.** *Nature Methods,* 2007, vol. 4, 633-636
- **NELLES.** *Cell,* 2016, vol. 165, 488-496
- **DOLGOSHEINA et al.** *ACS chemical biology,* 2014, vol. 9, 2412-2420
- **SUNBUL et al.** *Angewandte Chemie,* 2013, vol. 52, 13401-13404
- **PAIGE et al.** *Science,* 2011, vol. 333, 642-646
- **SONG et al.** *Nature Chemical Biology,* 2017, vol. 13, 1187-1194
- **FILONOV et al.** *Journal of the American Chemical Society,* 2014, vol. 136, 16299-16308
- **STRACK et al.** *Nature methods,* 2013, vol. 10, 1219-1224
- **CHEN et al.** *Nature Biotechnology,* 2019, vol. 37, 1287-1293
- **NEEDLEMAN ; WUNSCH.** *J.Mol.Biol.,* 1970, vol. 48, 443-453
- EMBOSS: The European Molecular Biology Open Software Suite, Rice etc. *Trends in Genetics,* 2000, vol. 16, 276-277
- **JANE ROSKAMS ; SAMBROOK.J ; D. W. RUSSELL ; PEITANG HUANG et al.** Molecular Cloning-A Laboratory Manual. Science Press, August 2002
- **SHAO et al.** *Nucleic acids research,* 2016, vol. 44, e86